(19) Europäisches Patentamt
European Patent Office
Office européen des brevets

(11) Publication number : **0 299 974 B1**

(12) # EUROPEAN PATENT SPECIFICATION

(45) Date of publication of patent specification : **22.01.92 Bulletin 92/04**

(21) Application number : **87902990.8**

(22) Date of filing : **03.04.87**

(86) International application number : **PCT/US87/00699**

(87) International publication number : **WO 87/06127 22.10.87 Gazette 87/23**

(51) Int. Cl.$^5$ : **A61K 31/655,** C07D 333/22, C07D 333/28, C07D 307/52, C07D 207/32, C07D 409/12, C07D 405/12, C07D 401/12, C07D 407/12

(54) **ANTHELMINTIC ACYLHYDRAZONES, METHOD OF USE AND COMPOSITIONS.**

(30) Priority : **07.04.86 US 849035**

(43) Date of publication of application : **25.01.89 Bulletin 89/04**

(45) Publication of the grant of the patent : **22.01.92 Bulletin 92/04**

(84) Designated Contracting States : **AT BE CH DE FR GB IT LI LU NL SE**

(56) References cited :
**EP-A- 0 172 031**
**EP-A- 0 183 398**
**GB-A- 1 581 675**
**US-A- 2 639 287**
**US-A- 3 733 319**
**US-A- 4 064 262**

(73) Proprietor : **THE UPJOHN COMPANY**
**301 Henrietta Street**
**Kalamazoo, Michigan 49001 (US)**

(72) Inventor : **RECTOR, Douglas, L.**
**6075 Litchfield Lane**
**Kalamazoo, MI 49009 (US)**
Inventor : **CONDER, George, A.**
**6835 East "F" Avenue**
**Richland, MI 49083 (US)**
Inventor : **FOLZ, Sylvester, D.**
**6209 Enola Drive**
**Kalamazoo, MI 49004 (US)**

(74) Representative : **Perry, Robert Edward et al**
**GILL JENNINGS & EVERY 53-64 Chancery Lane**
**London WC2A 1HN (GB)**

EP 0 299 974 B1

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

### FIELD OF THE INVENTION

This invention relates to compounds for use in killing and controlling worms in animals, and to novel such compounds.

### BACKGROUND OF THE INVENTION

The diseases or groups of diseases described generally as helminthiasis are due to infection of the animal with parasitic worms known as helminths. Helminthiasis and helminthosis are prevalent and may lead to serious economic problems in valuable domestic warm-blooded animals such as sheep, swine, cattle, goats, dogs, cats, horses, poultry and man. Among the helminths, the groups of worms known as nematodes, trematodes and cestodes cause widespread and often serious infections in various species of animals including man. The most common genera of such nematodes, trematodes and cestodes are Dictyocaulus, Haemonchus, Trichostrongylus, Ostertagia, Nematodirus, Cooperia, Bunostomum, Oesophagostomum, Chabertia, Strongyloides, Trichuris, Fasciola, Dicrocoelium, Enterobius, Ascaris, Toxascaris, Toxocara, Ascaridia, Capillaria, Heterakis, Ancylostoma, Uncinaria, Dirofilaria, Onchocerca, Taenia, Moniezia, Dipyldium, Metastrongylus, Triodontophorus, Macracanthorhynchus, Hyostrongylus and Strongylus. Some of these genera attack primarily the intestinal tract, while others inhabit the stomach, lungs, liver and subcutaneous tissues. The parasitic infections causing helminthiasis and helminthosis lead to anemia, malnutrition, weakness, weight-loss, unthriftiness, severe damage to the gastrointestinal trace wall and, if left to run their course, death.

WO-A-8605982 (which has been published after the present priority date) discloses quinolyl acylhydrazones and their anthelmintic use.

### SUMMARY OF THE INVENTION

One aspect of this invention lies in the use of acylhydrazones of formula I for the manufacture of a medicament for use in killing parasitic worms in humans and valuable warm-blooded domestic animals. Another aspect lies in novel acylhydrazones of formula I (as defined in claim 6). Known compounds of formula I have not previously been associated with anthelmintic activity.

### DETAILED DESCRIPTION OF THE INVENTION

The acylhydrazones as used according to this invention, including hydrates or pharmaceutically acceptable salts thereof, are represented by Formula I wherein X is (a) hydrogen; with the proviso that when X is hydrogen and the compound is a 2-thienyl acylhydrazone, and $R_1$ is 5-methyl and $R_2$ is hydrogen, $R_3$ is other than methyl; (b) $C_1$-$C_{10}$ alkyl; with the proviso that when X is ethyl and the compound is a 2-thienyl acylhydrazone, and $R_1$ is 5-Cl and $R_2$ is hydrogen, $R_3$ is other than methyl; (c) $C_2$-$C_6$ alkenyl, preferably $C_2$-$C_4$ alkenyl; (d) $C_2$-$C_6$ alkynyl; (e) cyclo($C_3$-$C_{10}$)alkyl optionally substituted with one, 2 or 3 $C_1$-$C_4$ alkyl, $C_2$-$C_4$ alkenyl, $C_1$-$C_4$ alkoxy, trifluoromethyl, or halo; with the proviso that when X is cyclohexyl or chrysanthemyl and the compound is a 2-thienyl acylhydrazone, and $R_1$ is 5-Cl and $R_2$ is hydrogen, $R_3$ is other than methyl; (f) pyrrolidinyl; (g) piperidinyl; (h) 1-methylpyrrolidinyl; (i) 1-methylpiperidinyl; (j) $C_2$-$C_6$ alkoxyalkyl; (k) cyclo($C_3$-$C_{10}$)alkyl($C_1$-$C_4$)alkyl; (l) phenyl($C_1$-$C_4$)alkyl optionally substituted with one, 2 or 3 $C_1$-$C_4$ alkyl, $C_1$-$C_4$ alkoxy, halo, or trifluoromethyl; (m) cyano($C_1$-$C_3$)alkyl; (n) naphthyl($C_1$-$C_3$)alkyl optionally substituted with one or two $C_1$-$C_4$ alkyl, $C_1$-$C_4$ alkoxy, halo, or trifluoromethyl; (o) $C_1$-$C_6$ alkoxy, with the proviso that when X is methoxy and the compound is a 2-thienyl acylhydrazone, and $R_1$ is 5-methyl and $R_2$ is hydrogen, $R_3$ is other than methyl; with the proviso that when X is ethoxy or t-butoxy and the compound is a 2-thienyl acylhydrazone, and $R_1$ is 5-Cl and $R_2$ is hydrogen, $R_3$ is other than methyl; with the proviso that when X is ethoxy and the compound is a 2-thienyl acylhydrazone, and $R_1$ is 4-methyl and $R_2$ is hydrogen, $R_3$ is other than methyl; with the proviso that when X is ethoxy and the compound is a 2-thienyl acylhydrazone, and $R_1$ and $R_2$ are hydrogen, $R_2$ is other than phenyl or 4-ethoxyphenyl; (p) diphenylmethoxy; (q) cyclo($C_3$-$C_6$)alkyloxy optionally substituted with one or two $C_1$-$C_3$ alkyl; (r) phenoxy optionally substituted with one, 2 or 3 $C_1$-$C_4$ alkyl, $C_1$-$C_4$ alkoxy, halo, or trifluoromethyl; with the proviso that when X is phenoxy and the compound is a 2-thienyl acylhydrazone, and $R_1$ is 5-Cl and $R_2$ is hydrogen, $R_3$ is other than methyl; (s) benzyloxy optionally substituted with one, 2 or 3 $C_1$-$C_4$ alkyl, $C_1$-$C_4$ alkoxy, halo, or trifluoromethyl; with the proviso that when X is benzyloxy and the compound is a 2-thienyl acylhydrazone, and $R_1$ is 4-methyl or 5-methyl and $R_2$ is hydrogen, $R_3$ is other than methyl; (t) heteroaromatic optionally substituted with one, 2 or 3 $C_1$-$C_4$ alkyl, $C_1$-$C_3$ alkoxy, halo, $C_1$-$C_3$ alkylthio, or trifluoromethyl; (u) phenyl optionally substituted with one, 2 or 3 $C_1$-$C_4$ alkyl, $C_1$-$C_3$ alkoxy, halo, trifluoromethyl, $C_2$-$C_6$ dialkylamino, $C_1$-$C_3$ alkylthio, nitro, or phenoxy optionally substituted with one, 2 or 3 $C_1$-$C_4$ alkyl, $C_1$-$C_3$ alkoxy, halo, or trifluoromethyl; with the proviso that when X is 4-chlorophenyl and the compound is a 2-thienyl acylhydrazone, and $R_1$ and $R_2$ are hydrogen, $R_3$ is other than hydrogen; with the proviso that when X is 3-chlorophenyl, 2-methylphenyl or 4-t-butylphenyl and the compound is a 2-thienyl acylhydrazone, and $R_1$ and $R_2$ are hydrogen, $R_3$ is other than methyl; with the further proviso that when X is 2-nitrophenyl or 2-ethoxyphenyl and the compound is a 2-furanyl acylhydrazone, and $R_1$ and $R_2$ are hydrogen, $R_3$ is other than methyl; with the further proviso that when X is phenyl and the compound is a 1-methyl-1H-pyrrol-2-yl acylhydrazone, $R_1$ and $R_2$ are hydrogen, $R_3$ is other than hydrogen; with the proviso that when X is 2-chlorophenyl or 4-chlorophenyl and the compound is a 2-thienyl acylhydrazone, and $R_2$ and $R_3$ are hydrogen, $R_1$ is other than 5-methyl; with the further proviso that when X is 2-nitrophenyl and the compound is a 2-thienyl acylhydrazone, and $R_1$ and $R_2$ are hydrogen, $R_3$ is other than ethyl; with the further proviso that when X is 2-methylphenyl and the compound is a 1H-pyrrol-2-yl acylhydrazone, $R_1$ and $R_2$ are hydrogen, $R_3$ is other than hydrogen; (v) phenyl optionally substituted with the divalent $C_1$-$C_2$ alkylenedioxy; (w) naphthyl optionally substituted with one or 2 $C_1$-$C_4$ alkyl, $C_1$-$C_3$ alkoxy, halo, trifluoromethyl, $C_2$-$C_6$ dialkylamino, $C_1$-$C_3$ alkylthio, nitro; (x) bridged polycyclic hydrocarbon substituents of six to 10 nuclear carbons, optionally substituted with one, 2 or 3 ($C_1$-$C_3$) alkyl groups; (y) perhalo($C_1$-$C_7$)alkyl; (z) N-morpholinyl($C_1$-$C_4$)alkyl; (aa) N-piperidinyl($C_1$-$C_4$)alkyl; (bb) N-pyrrolidinyl($C_1$-$C_4$)alkyl;

wherein Y is an oxygen atom; a sulfur atom; or a N-Z group;

wherein Z is hydrogen; $C_1$-$C_4$ alkyl; phenyl optionally substituted with one, 2 or 3 $C_1$-$C_4$ alkyl, $C_1$-$C_3$ alkoxy, halo, trifluoromethyl, $C_2$-$C_6$ dialkylamino, $C_1$-$C_3$ alkylthio, nitro, or phenoxy optionally substituted with one, 2 or 3 $C_1$-$C_4$ alkyl, $C_1$-$C_3$ alkoxy, halo, or trifluoromethyl; or phenyl($C_1$-$C_4$)alkyl optionally substituted with one, 2 or 3 $C_1$-$C_4$ alkyl, $C_1$-$C_4$ alkoxy, halo, or trifluoromethyl;

wherein $R_1$ and $R_2$, being the same or different, are hydrogen; hydroxy; $C_1$-$C_4$ alkyl, preferably $C_1$-$C_3$ alkyl; $C_1$-$C_3$ alkoxy; $C_1$-$C_3$ alkylthio; halo or trifluoromethyl; with the proviso that $R_1$ and $R_2$ are not both halo; and

wherein $R_3$ is hydrogen; $C_1$-$C_6$ alkyl; cyclo($C_3$-$C_6$)alkyl optionally substituted with one, 2 or 3 $C_1$-$C_3$ alkyl, preferably cyclo(C3-$C_5$)alkyl optionally substituted; phenyl optionally substituted with one, 2 or 3 $C_1$-$C_4$ alkyl, halo, trifluoromethyl, or $C_1$-$C_3$ alkoxy; phenyl($C_1$-$C_3$)alkyl optionally substituted with one, 2 or 3 $C_1$-$C_4$ alkyl, halo, trifluoromethyl, or $C_1$-$C_3$ alkoxy; 1,3-dioxacyclohexan-5-yl; thienylvinyl; furanylvinyl; or phenylvinyl.

C__- C__ means the carbon content of various hydrocarbon-containing moieties is indicated by a prefix designating the minimum and maximum number of carbon atoms in the moiety. Thus ($C_1$-$C_3$) alkyl refers to alkyl of one to 3 carbon atoms, inclusive or methyl, ethyl, propyl, and isopropyl.

Halogen atom (halo) refers to a bromo, chloro, iodo or fluoro atom.

Heteroaromatic refers to an aromatic heterocycle of 5 to 10 members, containing one or two heteroatoms selected from the group consisting of oxygen, nitrogen or sulfur and includes quinoline, pyrrole, indole, benzofuran, benzothiophene, quinazoline, quinoxaline, imidazole, pyrazole, oxazole, isoxazole, thiazole, isothiazole, pyridazine, pyrimidine, pyrazine, benzimidazole, benzothiazole, benzoxazole, pyridine, thiophene or furan, as well as the N-oxides, hydrates and pharmaceutically acceptable salts thereof.

Pharmaceutically acceptable refers to those properties and/or substances which are acceptable to the patient from a pharmacologically-toxicological point of view and to the manufacturing pharmaceutical chemist from a physical-chemical point of view regarding composition, formulation, stability, patient acceptance and bioavailability.

Examples of $C_1$-$C_4$ alkyl are methyl, ethyl, propyl, butyl and isomeric forms thereof. Examples of $C_1$-$C_3$ alkoxy are methoxy, ethoxy, propoxy and isomeric forms thereof. Examples of phenoxy substituted with one, 2 or 3 $C_1$-$C_4$ alkyl are (o -, m-, or p-)tolyl, (o-, m-, or p-) ethylphenyl, p-tert-butylphenyl,2,5-dimethylphenyl, 2,6-dimethylphenyl, 2,4-dimethylphenyl, (2,3,4-, 2,3,5-, 2,3,6-, 2,4,6- or 2,4,5-)trimethylphenyl.

Examples of $C_2$-$C_6$ dialkylamino are dimethylamino, diethylamino, methylethylamino, dipropylamino and ethylpropylamino.

Examples of phenyl($C_1$-$C_3$)alkyl are benzyl, phenylethyl and phenylpropyl. Examples of phenyl($C_1$-$C_3$)alkyl substituted with one, 2 or 3 $C_1$-$C_4$ alkoxy, halo or trifluoromethyl include 4-chlorobenzyl, 2-chlorophenylethyl, p-tolylethyl, 2-methylbenzyl, 4-methoxybenzyl. Examples of $C_1$-$C_3$ alkylthio include methylthio, ethylthio, and n-propylthio.

Examples of substituted cyclo($C_3$-$C_{10}$)alkyl are chrysanthemyl, 1-methylcyclopropyl and 2-methylcyclopropyl. Examples of cyclo($C_3$-$C_{10}$)alkyl($C_1$-$C_4$)alkyl are 2-cyclohexylethyl and cyclohexylmethyl. An example of substituted cyclo($C_3$-$C_6$)alkyloxy is menthyl.

Examples of naphthyl($C_1$-$C_3$)alkyl include 2-naphthylmethyl and 1-naphthylethyl. Examples of substituted naphthyl($C_1$-$C_3$)alkyl is (3,8-dichloro-1-naphthyl)methyl; (4-chloro-1-naphthyl)methyl; and (4-methoxy-1-naphthyl)methyl. Examples of substituted naphthyl include 3,6-dichloro-1-naphthyl; 3,5-dichloro-2-naphthyl; 6-methyl-2-naphthyl; and 4,6-dichloro-1-haphthyl.

Examples of bridged polycyclic hydrocarbon substituents of six to 10 nuclear carbons, optionally substituted with one, 2 or 3 ($C_1$-$C_3$) alkyl groups include exo or endo-2-norbonyl, bicyclo[2,2,2]oct-1-yl, and 1-adamantyl.

Examples of perhalo ($C_1$-$C_7$) alkyl include trifluoromethyl, n-heptafluoropropyl and n-undecafluoropentyl.

Preferred acylhydrazones of Formula I are 2-thienyl acylhydrazones (IA) or 2-furanyl acylhydrazones (IB).

Preferred $R_1$ and $R_2$ include hydrogen, methyl, or a chloro atom.

Preferred $R_3$ includes hydrogen, methyl or ethyl.

Preferred X include hydrogen; $C_1$-$C_4$ alkyl; cyclohexylethyl; phenyl optionally substituted with one, 2 or 3 $C_1$-$C_4$ alkyl, $C_1$-$C_2$ alkoxy, trifluoromethyl and chloro; $C_1$-$C_4$ alkoxy; phenoxy optionally substituted with one, 2 or 3 $C_1$-$C_2$ alkyl, $C_1$-$C_2$ alkoxy, trifluoromethyl and chloro; cyclo($C_3$-$C_6$)alkyl; pyridinyl; thienyl; furanyl; benzyloxy optionally substituted with one or 2 $C_1$-$C_2$ alkoxy; di($C_1$-$C_2$)alkoxyphenylmethyl; N-morpholinylethyl; or 1-menthyl0.

Preferred compounds of this invention are the compounds of Table A represented by compound nos: 1, 6, 11, 18, 22, 25, 28, 30, 31, 32, 35,45, 48, 49, 54, 55, 57, 58, 59, 60, 61, 62, 64, 66, 68, 69, 70, 71, 72, 73, 74, 75, 81, 84, 85, 86, 87, 88, 89, 91, 94, 95, 97, 100, 108, 114, 115, 116, 118, 119, 120, 124, 126, 130, 131, 133, 137, 138, 144, 146, 148, 150, 154, 178, 186, 192, 201, 202, 203, 208, 209, 211, 224, 240, 241, 264, 266, 267, 268, 270, 271, 272, 274, 276, 279, 283, 285, 288, 289, 290, 291, 292, 293, 294, 296, 300, 301, 303, 305, 307, 311, 312, 313, 315, 317, 318, 320, 323, 324, 325, 327, 328, 329, 331, 343, 345, 346, 361, 363, 365, 366, 367, 370, 384, 386, 390, 391, 392, 394, 395, 397, 400, 402, 404, 406, 410, 411, 413, 422, 423, 424, 426, 429, 430, 432, 434, 435, 436, 437, 438, 439, 440, 441, 443, 445, 446, 450, 457, 483, 487, 494, 504, 506, 568, 561, 575, 585, 590, 591, 600, 604 and 605.

One embodiment of this invention includes, of course, the anthelmintic use and anthelmintic compositions of compounds of Formula I, IA, IB, or IC hydrates thereof or pharmaceutically acceptable salts thereof.

Another embodiment of this invention are the novel compounds, hydrates thereof or pharmaceutically acceptable salts thereof according to Formula I, IA, IB, and IC.

Another embodiment of this invention are the novel compounds of Formula I, the hydrates thereof or pharmaceutically acceptable salts thereof where X is (e) cyclo($C_3$-$C_{10}$)alkyl optionally substituted with one, 2 or 3 $C_1$-$C_4$ alkyl, $C_2$-$C_4$ alkenyl, $C_1$-$C_4$ alkoxy, trifluoromethyl, or halo; with the proviso that when X is cyclohexyl and the compound is a 2-thienyl acylhydrazone, and $R_1$ is 5-Cl and $R_2$ is hydrogen, $R_3$ is other than methyl; (j) $C_2$-$C_6$ alkoxyalkyl; (k) cyclo($C_3$-$C_{10}$)alkyl($C_1$-$C_4$)alkyl; (q) cyclo($C_3$-$C_6$)alkyloxy optionally substituted with one or two $C_1$-$C_3$ alkyl; (s) benzyloxy optionally substituted with one, 2 or 3 $C_1$-$C_4$ alkyl, $C_1$-$C_4$ alkoxy, halo, or trifluoromethyl; with the proviso that when X is benzyloxy and the compound is a 2-thienyl acylhydrazone, and $R_1$ is 4-methyl or 5-methyl and $R_2$ is hydrogen, $R_3$ is other than methyl; or (u) phenyl substituted with one, 2 or 3 $C_1$-$C_3$ alkoxy, phenoxy or trifluoromethyl; with the proviso that when X is 2-ethoxyphenyl and the compound is a 2-furanyl acylhydrazone, and $R_1$ and $R_2$ are hydrogen, $R_3$ is other than methyl.

Still another embodiment of this invention are the novel compounds of Table A as defined in the claims and represented by compound nos:

11, 22, 28, 30, 31, 32, 35,45, 48, 49, 54, 55, 57, 58, 59, 60, 61, 62, 64, 66, 68, 69, 70, 71, 72, 73, 74, 75, 81, 84, 85, 86, 87, 88, 89, 91, 94, 95, 97, 100, 108, 114, 116, 118, 119, 120, 124, 126, 130, 131, 133, 137, 138, 144, 146, 148, 150, 154, 178, 186, 192, 201, 202, 203, 208, 209, 211, 224, 240, 241, 264, 266, 267, 268, 270, 272, 274, 276, 279, 283, 285, 288, 289, 290, 291, 292, 293, 294, 296, 300, 301, 303, 305, 307, 311, 312, 313, 315, 317, 318, 320, 323, 325, 327, 328, 329, 343, 346, 361, 363, 365, 366, 384, 386, 390, 391, 392, 394, 395, 400, 402, 404, 406, 411, 413, 422, 423, 426, 429, 430, 432, 434, 435, 436, 437, 438, 439, 440, 441, 443, 445, 446, 450, 457, 483, 487, 494, 506, 568, 569, 575, 585, 590, 591, 600, 604 and 605,

Among the acylhydrazones of Formula I are the compounds of TAble A represented by compound nos: 1, 6, 7, 8, 12, 17, 18, 20, 24, 25, 26, 56, 115, 230, 265, 271, 275, 284, 324, 331, 332, 333, 345, 359, 367, 368, 369, 370, 385, 388, 396, 397, 398, 405, 409, 410, 421, 424, 425 and 504 are known.

The acylhydrazones of this invention (Formula I) are readily prepared by reacting the appropriate ketone/aldehyde (II) with the acylhydrazide/carbazate (III) (Chart A, Scheme A) or by heating the heteroaromatic ketone/aldehyde (II) with the appropriate hydrazine (IV) to form the hydrazone intermediate (V) which is then acylated with the halide or anhydride (VI) to form the acylhydrazone (I) (Chart A, Scheme B).

The reaction of Scheme A is carried out in the presence of a suitable solvent, for example, water, alcohols, ethers, halogenated hydrocarbons, hydrocarbons and include methanol, ethanol, isopropanol, propanol, hexane, tetrahydrofuran, dioxane, methylene chloride, and preferably ethanol. A catalyst such as glacial acetic acid, hydrochloric acid, sulfuric acid or p-toluenesulfonic acid can be utilized to enhance the yield/rate of the reaction, particularly when $R_3$ is alkyl of 3 or more atoms, arylalkyl or aryl.

The acylation reaction of Scheme B is carried out in the presence of a suitable base such as an organic tertiary amine, or inorganic base, for example, triethylamine or sodium carbonate or preferably, pyridine. The

base may also be the solvent.

The starting compounds are known or can be readily prepared by known methods. R. L. Frank and C. Weatherbee, J. Am. Chem. Soc., 70, 3482-3 (1948); N. B. Mahishi, et al., J. Indian Chem. Soc., 42, 67-74 (1965) and M. Ogata and H. Kano, Chem, Pharm, Bull (Tokyo), 11, 32 (1963).

The following detailed examples/procedures describe how to prepare various acylhydrazones of the invention and are to be construed as merely illustrative, and not limitations of the preceding disclosure in any way whatsoever. Those skilled in the art will promptly recognize appropriate variations from the procedures both as to reactants as well as to reaction conditions and techniques.

Procedure 1 Preparation of benzoic acid (2-thienylmethylene)-hydrazide, Compound 1.

A mixture of 6.81 gm (0.05 mole) of benzoic acid hydrazide, 5.6 gm (0.05 mole) of 2-thiophenecarboxaldehyde and 100 ml of absolute ethanol is refluxed 8 hr. Sufficient dioxane is added at boiling to furnish a solution. The hot solution is filtered. The filtrate is cooled and then chilled. The product is collected and dried to yield 7.68 gm (67%) of the title compound having a melting point of 203.3°C.

| | |
|---|---|
| Analysis Calcd: | C, 62.61; H, 4.35. |
| Found: | C, 62.34; H, 4.36. |

Procedure 2 Preparation of 4-methylbenzoic acid (2-thienylmethylene)hydrazide, Compound 3.

A mixture of 3.36 gm (0.03 mole) of 2-thiophenecarboxaldehyde, 4.51 gm (0.03 mole) of 4-methylbenzoic acid hydrazide, ten drops of concentrated hydrochloric acid and 100 ml of absolute ethanol is refluxed 6 hr. Sufficient dioxane is added to the boiling mixture to furnish as solution. The hot solution is filtered. The filtrate is cooled to room temperature, then chilled. The product is collected, washed with Skellysolve B and dried to yield 5.01 gm (69%) of the title compound having a melting point of 219.1°C.

| | |
|---|---|
| Analysis Calcd: | C, 63.93; H, 4.92; N, 11.45. |
| Found: | C, 63.77; H, 5.00; N, 11.59. |

Procedure 3 Preparation of cyanoacetic acid (2-thienylmethylene)-hydrazide. Compound 12.

A mixture of 2.97 gm (0.03 mole) of cyanoacetylhydrazide, 3.36 gm (0.03 mole) of 2-thiophenecarboxaldehyde and 200 ml of absolute ethanol is refluxed 6 hr. Sufficient dimethylformamide is added to give a solution. The hot solution is filtered. The filtrate is cooled and then chilled. The product is collected, washed with Skellysolve B and dried to yield 3.23 gm (56%) of the title compound having a melting point of 158.0°C.

| | |
|---|---|
| Analysis Calcd: | C, 49.74; H, 3.63. |
| Found: | C, 50.00; H, 3.70. |

Procedure 4 Preparation of 3-nitrobenzoic acid [1-(2-thienyl)-ethylidene]hydrazide. Compound 14.

A mixture of 3.62 gm (0.02 mole) of 3-nitrobenzoic acid hydrazide, 2.24 gm (0.02 mole) of 2-acetylthiophene, 1 ml of glacial acetic acid and 100 ml of absolute ethanol is refluxed 10 hr. A sufficient amount of dimethylformamide is added to the boiling mixture to furnish a solution. The solution is filtered. The filtrate is diluted with water to the cloud point and cooled, then chilled. The product is collected, washed with ether and dried to yield 4.3 gm (75%) of the title compound having a melting point of 223.1°C.

| | |
|---|---|
| Analysis Calcd: | C, 53.98; H, 3.81; N, 14.53. |
| Found: | C, 54.27; H, 3,96; N, 14.49. |

Procedure 5 Preparation of ethyl (2-thienylmethylene)carbazate Compound 18.

A mixture of 3.36 gm (0.03 mole) of 2-thiophenecarboxaldehyde, 3.12 gm (0.03 mole) of ethyl carbazate and 100 ml of absolute ethanol is refluxed 8 hr. The hot solution is filtered. The filtrate is diluted with water, cooled to room temperature and then chilled. The product is collected, washed with water and dried to yield 4.32 gm (73%) of the title compound having a melting point of 102.0°C.

| | |
|---|---|
| Analysis Calcd: | C, 48.48; H, 5.05; N, 14.14. |
| Found: | C, 48.35; H, 5.15; N, 14.09. |

Procedure 6 Preparation of benzyl [1-(5-methyl-2-furanyl)ethyl-idene]-carbazate. Compound 36.

To 9.14 gm (0.055 mole) of benzylcarbazate dissolved in 100 ml warm water is added 6.82 gm (0.055 mole) of 2-acetyl-5-methylfuran in 30 ml of ethanol. The mixture is refluxed 16 hr. The solid which separates is collected. The product is recrystallized from absolute ethanol to yield 9.81 gm (66%) of the title compound having a melting point of 110.3°C.

| | |
|---|---|
| Analysis Calcd: | C, 66.18; H, 5.88; N, 10.29. |
| Found: | C, 66,21; H, 5.87; N, 10.44. |

Procedure 7 Preparation of 1-menthyl[1-(2-thienyl)ethylidene]-carbazate. Compound 38.

A mixture of 6.31 gm (0.05 mole) of 2-acetylthiophene, 10.72 gm (0.05 mole) of 1-menthylcarbazate, 100 ml of ethanol and 5 drops of concentrated hydrochloric acid is refluxed 20 hr. The hot solution is filtered. The filtrate is cooled to room temperature and then chilled. The product is collected, washed with Skellysolve B and dried to yield 12.11 gm (75%) of the title compound having a melting point of 168.0°C.

| | |
|---|---|
| Analysis Calcd: | C, 63,35; H, 8,08; N, 8.70; S, 9.94. |
| Found: | C, 63,46; H, 8,14; N, 8.83; S, 10.02. |

Procedure 8 Preparation of 4-dimethylaminobenzoic acid [1-(2-thienyl)ethylidene]hydrazide. Compound 49.

A mixture of 6.31 gm (0,05 mole) of 2-acetylthiophene, 8.99 gm (0,05 mole) of 4-dimethylaminobenzoic acid hydrazide, 5 drops of glacial acetic acid and 100 ml of ethanol is refluxed 20 hr. The mixture is cooled and then chilled. The product is collected, washed with Skellysolve B and dried to yield 12.28 gm (85%) of the title compound having a melting point of 175.3°C.

| | |
|---|---|
| Analysis Calcd: | C, 62.72; H, 5.92; N, 14.63 |
| Found: | C, 62,30; H, 6.25; N, 15.15. |

Procedure 9 Preparation of formic acid [(3-methyl-2-thienyl)-methylene]hydrazide. Compound 57.

A mixture of 6.31 gm (0.05 mole) of 3-methylthiophene-2-carboxaldehyde, 3.00 gm (0.05 mole) of formic acid hydrazide and 100 ml of ethanol is refluxed 10 hr. The reaction mixture is treated with Darco decolorizing carbon and filtered. The filtrate is cooled to room temperature and then chilled. The product is collected to yield 2.34 gm (28%) of the title compound having a melting point of 125.2°C.

| | |
|---|---|
| Analysis Calcd: | C, 50.00; H, 4.76; N, 16.67; S, 16.10. |
| Found: | C, 49.93; H, 4.91; N, 16.10; S, 16.26. |

Procedure 10 Preparation of 3-chlorobenzoic acid [(3-methyl-2-thienyl)methylene]hydrazide. Compound 71.

A mixture of 6.31 gm (0.05 mole) of 3-methylthiophene-2-carboxaldehyde, 8.53 gm (0.05 mole) of 3-chlorobenzhydrazide and 100 ml of ethanol is refluxed 10 hr. The mixture is cooled and chilled. The tan product is collected, then crystallized from isopropyl alcohol (decolorized with Darco) to yield 3,99 gm (59%) of the title compound having a melting point of 208.8°C.

| | |
|---|---|
| Analysis Calcd: | C, 56.01; H, 3.95; N, 10.05; Cl, 12.75; S. 11.91, |
| Found: | C, 55,89; H, 4.07; N, 9.91; Cl, 12.95; S, 11.77. |

Procedure 11 Preparation of 4-dimethylaminobenzoic acid [(3-methyl-2-thienyl)methylene]hydrazide. Compound 77.

A mixture of 6.31 mg (0.05 mole) of 3-methylthiophene-2-carboxaldehyde, 8.99 gm (0.05 mole) of 4-dimethylaminobenzoic acid hydrazide and 100 ml of ethanol is refluxed 6 hr. The solvent is removed. The crude product is suspended in 90 ml of boiling isopropyl alcohol and sufficient dimethylformamide added to give a solution. The solution is treated with Darco decolorizing carbon and filtered. The filtrate is diluted with water to the cloud point and chilled. The product is collected, washed with water and dried to yield 10.5 gm (73%) of the title compound having a melting point of 231.5°C.

| | |
|---|---|
| Analysis Calcd: | C, 62,72; H, 5.92; N, 14.63; S, 11.15. |
| Found: | C, 62,48; H, 5.94; N, 14.50; S. 11.19. |

Procedure 12 Preparation of 4-nitrobenzoic acid [(5-methyl-2-thienyl)methylene]hydrazide. Compound 112.

A mixture of 6.31 gm (0.05 mole) of 5-methylthiophene-2-carboxaldehyde, 9.06 gm (0.05 mole) of 4-nitrobenzoic acid hydrazide and 100 ml of ethanol is refluxed 12 hr. The solvent is evaporated. The crude product is suspended in boiling isopropyl alcohol. Sufficient dimethylformamide is added to furnish a solution. The solution is filtered and the filtrate diluted with water to the cloud point and chilled. The product is collected, washed with water and dried to yield 14.45 gm (100%) of the title compound having a melting point of 224.8°C.

| | |
|---|---|
| Analysis Calcd: | C, 53.98; H, 3.81; N, 14.53; S, 11.07. |
| Found: | C, 53.82; H, 3.86; N, 14.31; S, 11.14. |

Procedure 13 Preparation of 2-chlorobenzoic acid [1-(5-chloro-2-thienyl)ethylidene]hydrazide. Compound 170.

A mixture of 8.03 gm (0.05 mole) of 2-acetyl-5-chlorothiophene, 8.53 gm (0.05 mole) of 2-chlorobenzhydrazide, 100 ml of ethanol and 5 drops of concentrated hydrochloric acid is refluxed 20 hr. The mixture is cooled to room temperature and then chilled. The product is collected, washed with water and dried to yield 8.53 gm (55%) of the title compound having a melting point of 229.9°C (decomposition).

| | |
|---|---|
| Analysis Calcd: | C, 49.84; H, 3.19; Cl, 22.68; N, 8.95; S, 10.72. |
| Found: | C, 50.06; H, 3.31; Cl, 23.01; N, 8.86; S, 10.42. |

Procedure 14 Preparation of 2,4,5-trichlorophenoxyacetic acid [1-(2-thienyl)ethylidene]hydrazide, Compound 261.

A mixture of 4.04 gm (0.015 mole) of 2,4,5-trichlorophenoxy-acetic acid, 1.89 gm (0,015 mole) of 2-acetylthiophene, 100 ml of dioxane and 1 ml of glacial acetic acid is refluxed 4 hr. Sufficient dimethylformamide is added to furnish a solution. The hot solution is filtered and filtrate diluted with water to the cloud point. The mixture is cooled and then chilled. The product is collected, washed with methanol and dried to yield 4.11 gm (73%) of the title compound having a melting point of 221.4°C.

| | |
|---|---|
| Analysis Calcd: | C, 44.50; H, 2.91; N, 7.42 |
| Found: | C, 44.72; H, 3.11; N, 7.51 |

Procedure 15 Preparation of β-morpholinopropanoic acid (2-thienyl-methylene)hydrazide. Compound 263.

A mixture of 3.46 gm (0.02 mole) of β-morpholinopropanoic acid hydrazide, 2.24 gm (0.02 mole) of 2-thiophenecarboxaldehyde and 100 ml of ethanol is refluxed 16 hr. The solvent is evaporated. The residue is dissolved in boiling ethyl acetate. The solution is treated with Darco decolorizing carbon and filtered. The filtrate is diluted with Skellysolve B, cooled, then chilled. The product is collected, washed with cold ether and dried to yield 3.35 (63%) of the title compound having a melting point of 160.7°C.

| | |
|---|---|
| Analysis Calcd: | C, 53.93; H, 6.37; N, 15.73; S, 11.99 |
| Found: | C, 53.49; H, 6.75; N, 16.13; S, 11.79 |

Procedure 16 Preparation of methyl(2-furanylmethylene)carbazate Compound 265.

A mixture of 2.70 gm (0.03 mole) of methyl carbazate, 2.88 gm (0.03 mole) of 2-furaldehyde and 50 ml of methanol is refluxed 8 hr. The hot solution is filtered. The filtrate is diluted with water, cooled and chilled. The product is collected, washed with water and dried to yield 3.07 gm (61%) of the title compound having a melting point of 124.5°C with decomposition.

| | |
|---|---|
| Analysis Calcd: | C, 50.00; H, 4.76; N, 16.67 |
| Found: | C, 49.90; H, 4.79; N, 16.51 |

Procedure 17 Preparation of 4-methylbenzoic acid [(5-methyl-2-furanyl)-methylene]hydrazide. Compound 317.

To a mixture of 5.50 gm (0.05 mole) of 5-methyl-2-furaldehyde in 100 ml of ethanol is added 7.51 gm (0.05 mole) of 4-methylbenzoic acid hydrazide. The mixture is refluxed 6 hr and chilled. The product is collected, washed with cold ethanol and dried to yield 11.18 gm (92%) of the title compound having a melting point of 187. 5°C.

| | |
|---|---|
| Analysis Calcd: | C, 69,41; H, 5.82; N, 11.56 |

Found: C, 69.11; H, 5.82; N, 11.49

Procedure 18 Preparation of cyclohexylacetic acid [1-(2-furanyl)-ethylidene]hydrazide. Compound 322.

A mixture of 5.50 gm (0.05 mole) of 2-acetylfuran in 100 ml of ethanol is added 7.81 gm (0.05 mole) of cyclohexylacetic acid hydrazide and 10 drops of glacial acetic acid. The mixture is refluxed 18 hr and diluted with water to the cloud point. The mixture is cooled and then chilled. The product is collected, washed with water, and dried to yield 9.41 gm (76%) of the title compound having a melting point of 132.1°C.
Analysis Calcd: C, 67.72; H, 8.12; N, 11.27
Found: C, 67.40; H, 8.16; N, 11.05

Procedure 19 Preparation of 2,2-dimethyl-3-(2-methylpropenyl)-cyclopropanecarboxylic acid (2-furanylmethylene)-hydrazide. Compound 195.

A mixture of 28.83 gm (0.3 mole) of 2-furaldehyde, 54.6 gm (0.3 mole) of 2,2-dimethyl-3-(2-methylpropenyl)cyclopropanecarboxylic acid hydrazide and 400 ml of absolute ethanol is refluxed 5 hr. The solvent is removed in vacuo to give an oil which crystallizes on standing in a freezer for 16 hours. The product is slurried with Skellysolve B and filtered to yield 23.0 gm (29%) of the title compound having a melting point of 133.0°C.
Analysis Calcd: C, 69.21; H, 7.74; N, 10.76
Found: C, 68.86; H, 7.84; N, 10.76

Procedure 20 Preparation of butyric acid [1-(1H-pyrrol-2-yl)ethylidene]hydrazide. Compound 196.

A solution of 8.73 gm (0.080 mole) of 2-acetylpyrrole, 8,17 gm (0,08 mole) of n-butyric acid hydrazide, 0.30 gm of p-toluenesulfonic acid and 200 mol of tetrahydrofuran is refluxed for 8 hours. The reaction is chilled. The insoluble material is filtered off and discarded. The filtrate is diluted with water and chilled. The product separates and is collected and dried to yield 9.01 gm (58%) of the title compound having a melting point of 115.0°C.
Analysis Calcd: C, 62.15; H, 7,82; N, 21.74
Found: C, 61.75; H, 7.53; N, 21,52

The compounds prepared according to Procedures 1-20 are tabulated in Table A along with other illustrative compounds of the invention prepared following the general procedure indicated (Procedures 1-20) and making non-critical variations, except starting with the appropriate ketone/aldehyde (II) and acylhydrazide/carbazate (III).

The acylhydrazones of this invention (Formula I) are effective against parasitic worms, particularly those of valuable domestic warm-blooded animals and more particularly helminth parasites in ovines (sheep) and bovines (cattle).

Observations in sheep experimentally infected with Haemonchus contortus in accordance with Procedure 1, generally confirm anthelmintic activity at 100 mg/kg of body weight upon oral and/or parenteral administration as set forth in Table I. Acylhydrazones which are toxic at 100 mg/kg are expected to exhibit anthelmintic activity at a lower non-toxic dose. Further observations in sheep naturally infected with various helminths also confirmed broad-spectrum anthelmintic activity of acylhydrazones of this invention. See Procedure 2 and the results as set forth in Tables IIA-G.

Procedure No. 1

In individual experiments all sheep are treated identically, however non-critical variations occur between experiments. All of the sheep used in this procedure are treated twice with levamisole hydrochloride orally at 8 mg/kg or once each with ivermectin parenterally at 200 μg/kg and levamisole hydrochloride orally at 8 mg/kg. The second treatment in each case is administered 4-7 days after the first treatment. Two weeks after the second treatment all sheep are inoculated per os with ~3,500 to ~7,500 infective larvae of H. contortus. Rectal fecal samples are taken from each sheep 26-41 days post-inoculation (PI), and these samples are examined for eggs of H. contortus using the McMaster counting chamber technique. All sheep harboring good infections of H. contortus are randomly allocated to a treatment group; those which do not exhibit suitable infections are dropped from the study. One-three days later on days 27-42 PI each sheep remaining in the study (excluding the nontreated controls) is treated with a test compound (orally or parenterally at 100 mg/kg unless indicated otherwise) or a standard (levamisole hydrochloride orally at 8 mg/kg) or is used as an untreated control. All sheep received food and water ad lib. throughout the experiment.

Prior to administration, all solid compounds are finely ground using a mortar and pestle. Oral compounds

are suspended in 20-30 ml of sterile vehicle #98 (each ml contains: carboxymethylcellulose - 10 mg, polysorbate 80-4 mg, propylparaben - 0.42 mg) using a sonicator and administered along with a tap water wash via a stomach tube. The parenteral compounds are similarly suspended in 20-30 ml of the sterile vehicle and given by intraperitoneal injection using a 13 gauge, 2 inch needle and a 50 ml syringe. All test compounds are given to a single sheep/route of administration. Two or more sheep are treated with levamisole hydrochloride and five are used as nontreated controls. All animals are monitored for signs of toxicity following treatment.

The sheep are sacrificed 7-12 days after treatment (days 35-49 PI), and the abomasum is ligated and removed from each sheep. Each abomasum is longitudinally sectioned and rinsed into an 80 mesh sieve. Sieve contents are collected in individual containers and fixed in formol-alcohol. Later each sample is transferred to a 1000 or 2000 ml beaker and the volume brought to 400-1000 ml with tap water. The total number of worms in a 40-100 ml aliquot (10%) is determined. When no worms are found in the 10% aliquot, the entire sample is examined. Total worm number/sheep and percentage clearance for each treatment are calculated. Percentage clearance for a particular test compound in a given trial is determined according to the following formula:

Percentage Clearance = [(Mean number of worms recovered from nontreated control sheep - Number of worms recovered from treated sheep)/Mean number of worms recovered from nontreated control sheep] x 100.

Sheep which die within 24 hr following treatment are not examined for worms (Toxic), while any that die between 24 hr post-treatment and necropsy are examined in an identical manner as that described above. The results of various trials are combined and reported in Table I as percentage clearance.

Procedure No. 2

Parasitized sheep are randomly assigned to groups of five animals based on parasitic burden, sex, and farm origin. Sheep are double ear-tagged, weighed, housed in a barn in community pens, fed hay supplemented with 1/2 lb corn/head/day. Water is given ad lib. Animals are allowed to acclimate for one-two weeks prior to treatment.

Each group of sheep receives a test compound either orally or parenterally at a dosage rate of 100 mg/kg. A group of sheep is treated with 8 mg/kg of levamisole hydrochloride and another group serves as an untreated control group. Orally administered compounds are suspended in 20-30 ml of sterile vehicle #98 (each ml contains: carboxymethylcellulose - 10 mg, polysorbate 80-4 mg, propylparaben - 0.42 mg) using a sonicator and administered along with a tap water wash via a stomach tube. For parenteral administration, compounds are similarly suspended in 20-30 ml of the sterile vehicle and given by intraperitoneal injection using a 20 gauge, 1 inch needle and a 50 ml syringe. Following treatment, all animals are observed for signs of toxicity.

The number and classification of helminth eggs per gram of feces (e.p.g.) are determined for each sheep during the acclimation period and in some cases at necropsy. Egg counts are made using the McMaster counting chamber technique and rectal fecal samples. Animals dying during the 24 hours immediately following dosing are not subjected to necropsy. Sheep that die 1-6 days posttreatment are posted and complete worm counts performed. All remaining animals are sacrificed on days 7-8 posttreatment. Each sheep is euthanised and bled out prior to opening the abdominal cavity. Ligatures are placed at the reticulo-omasal junction, the pyloric valve, and the ileocecal junction. The abomasum and small intestine are freed of fat and mesenteric attachments, longitudinally opened, and their contents placed in individual containers. The mucosal surface of each is washed with tap water, rubbed clean, and rinsed several times. Washings and ingesta for each organ are made up to 1 liter and a 10% aliquot in formalin is stored for later examination. The cecum, large intestine, and colon are freed of mesenteric attachments, each is longitudinally opened, and their contents washed, collected, and made up to 1 liter in 10% formalin. The entire sample is stored. All carcasses are incinerated.

Ten percent of the total contents collected from the abomasum and small intestine and the entire contents of the large intestine, cecum, and colon are examined under stereoscopic magnification (40X). All worms are identified to genus and in some instances species. Separate adult and larval counts are determined.

The mean percentage clearance against specific helminths in the test sheep is calculated by subtracting the mean number of helminths observed in the treated sheep at necropsy from the mean number observed in the nontreated controls at necropsy, dividing the remainder by the latter mean number and multiplying by 100. The mean percentage clearances against the various helminths identified in the test sheep are calculated. The results for acylhydrazones of Formula I are set forth in Tables IIA-G.

From an evaluation of the test results set forth in Tables I and IIA-G, it is clear that the acylhydrazones of this invention (Formula I) are broad-spectrum anthelmintic agents.

DETAILED DESCRIPTION (cont'd)

The acylhydrazones of Formula I can be used as the pure compounds or as mixtures of pure compounds

but for practical reasons the compounds are preferably formulated as anthelmintic compositions and administered as a single or multiple dose, alone or in combination with other anthelmintics (e.g. avermectins, benzimidazoles, levamisole, praziquantel, etc.). For example, aqueous or oil suspensions can be administered orally, or the compounds can be formulated with a solid carrier for feeding. Furthermore, an oil suspension can be converted into an aqueous emulsion by mixing with water and injecting the emulsion intramuscularly, subcutaneously or into the peritoneal cavity. In addition, the active compound(s) can be administered topically to the animal in a conventional pour-on formulation.

Pure compounds, mixtures of the active compounds, or combinations thereof with a solid carrier can be administered in the animal's food, or administered in the form of tablets, pills, boluses, wafers, pastes, and other conventional unit dosage forms, as well as sustained release dosage forms which deliver the active compound over an extended period of days, weeks or months. All of these various forms of the active compounds of this invention can be prepared using physiologically acceptable carriers and known methods of formulation and manufacture.

Representative solid carriers conveniently available and satisfactory for physiologically acceptable, unit dosage formulations include corn starch, powdered lactose, powdered sucrose, talc, stearic acid, magnesium stearate, finely divided bentonite, and the like. The active agent can be mixed with a carrier in varying proportions from, for example, about 0.001 percent by weight in animal feed to about 90 or 95 percent or more in a pill or capsule. In the latter form, one might use no more carrier than sufficient to bind the particles of active compound.

In general, the compounds can be formulated in stable powders or granules for mixing in an amount of feed for a single feeding or enough feed for one day and thus obtain therapeutic efficacy without complication. It is the prepared and stored feeds or feed premixes that require care. A recommended practice is to coat a granular formulation to protect and preserve the active ingredient. A prepared hog-feed containing about 0.2 percent of the active compound will provide a dosage of about 100 mg per kg body weight for each 100 1b pig in its daily ration.

A solid diluent carrier need not be a homogeneous entity, but mixtures of different diluent carriers can include small proportions of adjuvants such as water; alcohols; protein solutions and suspensions like skimmed milk; edible oils; solutions, e.g., syrups; and organic adjuvants such as propylene glycols, sorbitol, glycerol, diethyl carbonate, and the like.

The solid carrier formulations of the inventions are conveniently prepared in unit dosage forms, to facilitate administration to animals. Accordingly, several large boluses (about 20 g weight) amounting to about 54 g of active compound would be required for a single dosage to a 900 lb horse at a dosage rate of 50 mg/kg of body weight. Similarly, a 60 lb lamb at a dosage rate of 100 mg/kg of body weight would require a pill, capsule, or bolus containing about 2.7 g of active compound, A small dog, on the other hand, weighing about 20 lbs. would require a total dosage of about 225 mg at a dosage rate of 25 mg/kg of body weight. The solid, unit dosage forms can be conveniently prepared in various sizes and concentrations of active ingredient, to accomodate treatment of the various sizes of animals that are parasitized by worms.

Liquid formulations can also be used. Representative liquid formulations include aqueous (including isotonic saline) suspensions, oil solutions and suspensions, and oil in water emulsions. Aqueous suspensions are obtained by dispersing the active compound in water, preferably including a suitable surface-active dispersing agent such as cationic, anionic, or non-ionic surface-active agents. Representative suitable ones are polyoxyalkylene derivatives of fatty alcohols and of sorbitan esters, and glycerol and sorbitan esters of fatty acids. Various dispersing or suspending agents can be included and representative ones are synthetic and natural gums, tragacanth, acacia, alginate, dextran, gelatin, sodium carboxymethylcellulose, methylcellulose, sodium polyvinylpyrrolidone, and the like. The proportion of the active compound in the aqueous suspensions of the invention can vary from about 1 percent to about 20 percent or more.

Oil solutions are prepared by mixing the active compound and an oil, e.g. an edible oil such as cottonseed oil, peanut oil, coconut oil, modified soybean oil, and sesame oil. Usually, solubility in oil will be limited and oil suspensions can be prepared by mixing additional finely divided compound in the oil.

Oil in water emulsions are prepared by mixing and dispersing an oil solution or suspension of the active compound in water preferably aided by surface-active agents and dispersing or suspending agents as indicated above.

In general, the formulations of this invention are administered to animals so as to achieve therapeutic or prophylactic levels of the active compound. At present, it is known that a dose of 100 mg/kg of body weight in sheep of a acylhydrazone of this invention will effectively combat a wide variety of parasites. Much lower effective dosages of various compounds are contemplated, e.g., in the range of 1 to 75 mg/kg of body weight.

In other animals, and for other kinds of parasitic worms, definitive dosages can be proposed. Contemplated are dosage rates of about 1 mg to about 800 mg/kg of body weight. A preferred, contemplated range of dosage

rates is from about 5 mg to about 400 mg/kg of body weight. In this regard, it should be noted that the concentration of active compound in the formulation selected for administration is in many situations not critical. One can administer a larger quantity of a formulation having a relatively low concentration and achieve the same therapeutic or prophylactic dosage as a relatively small quantity of a relatively more concentrated formulation. More frequent small dosages will likewise give results comparable to one large dose. One can also administer a sustained release dosage system (protracted delivery formulation) so as to provide therapeutic and/or prophylactic dosage amounts over an extended period. Unit dosage forms in accordance with this invention can have anywhere from less than 1 mg to 500 g of active compound per unit.

Although the anthelmintic agents of this invention will find their primary use in the treatment and/or prevention of helminth parasitisms in valuable warm-blooded domesticated animals such as sheep, cattle, horses, dogs, swine, goats and poultry, they are also effective in treatment that occurs in other warm blooded animals including man. The optimum amount to be employed for best results will, of course, depend upon the particular compound employed, species of animal to be treated, the regimen treatment and the type and severity of helminth infection. Generally good results are obtained with compounds of Formula I by the oral or parenteral route of administration of about 1 to 300 mg/kg of animal bodyweight (such total dose being given at one time, in a protracted manner or in divided doses over a short period of time such as 1-4 days). The technique for administering these materials to animals are known to those skilled in the veterinary and medical fields.

It is contemplated that the acylhydrazones of Formula I can be used to treat various helminth diseases in humans, including those caused by Ascaris, Enterobius, Ancylostoma, Trichuris, Strongyloides, Fasciola, Taenia, and/or Onchocerca or other filaria at a dose of from 1 mg/kg to 300 mg/kg of body weight upon oral and/or parenteral administration.

## DEFINITIONS

The definitions and explanations below are for the terms as used throughout the entire patent application including both the specification and claims.

All temperatures are in degrees Celsius.

TLC refers to thin-layer chromatography.

Brine refers to an aqueous saturated sodium chloride solution.

When solvent pairs are used, the ratio of solvents used are volume/volume (v/v).

TABLE A

| C | Y | a | $R_1$ | $R_2$ | $R_3$ | X | m.p.(°C) | P | H |
|---|---|---|---|---|---|---|---|---|---|
| 1 | S | 2 | H | H | H | Ph | 203.3 | 1 | - |
| 2 | $NCH_3$ | 2 | H | H | H | 4-$CF_3$Ph | 197.4 | 5 | - |
| 3 | S | 2 | H | H | H | 4-$CH_3$Ph | 219.1 | 2 | - |
| 4 | $NCH_3$ | 2 | H | H | H | Ph | 176.8 | 2 | - |
| 5 | S | 2 | H | H | H | 4-pyridinyl | 235.9d | 2 | - |
| 6 | S | 2 | H | H | H | 3-pyridinyl | 300.5d | 2 | - |
| 7 | S | 2 | H | H | H | 2-ClPh | 177.0 | 2 | - |
| 8 | S | 2 | H | H | H | 4-ClPh | 203.8 | 2 | - |
| 9 | NPh | 3 | 2-$CH_3$ | 5-$CH_3$ | H | $CH_3CH_2CH_2$ | 140.3 | 5 | - |
| 10 | NPh | 3 | 2-$CH_3$ | 5-$CH_3$ | H | 4-pyridinyl | 140.4 | 5 | + |
| 11 | NPh | 3 | 2-$CH_3$ | 5-$CH_3$ | H | $CH_3CH_2O$ | 176.2 | 5 | - |
| 12 | S | 2 | H | H | H | $NCCH_2$ | 158.0 | 3 | - |
| 13 | $NCH_3$ | 2 | H | H | H | $NCCH_2$ | 199.2 | 2 | - |
| 14 | S | 2 | H | H | $CH_3$ | 3-$NO_2$Ph | 223.1 | 4 | - |
| 15 | O | 2 | H | H | H | 3-$NO_2$Ph | 197.7 | 4 | - |
| 16 | $NCH_3$ | 2 | H | H | H | 3-$NO_2$Ph | 204.8 | 4 | + |
| 17 | O | 2 | H | H | H | $NCCH_2$ | indef. | 3 | - |
| 18 | S | 2 | H | H | H | $CH_3CH_2O$ | 111.0 | 5 | - |
| 19 | O | 2 | H | H | H | $CH_3CH_2O$ | 135.5 | 5 | - |
| 20 | $NCH_3$ | 2 | H | H | H | $CH_3CH_2O$ | 94.2 | 5 | - |
| 21 | S | 2 | H | H | H | $PhCH_2$ | 167.1 | 5 | - |
| 22 | $NCH_3$ | 2 | H | H | H | $PhCH_2$ | 131.4 | 5 | - |
| 23 | S | 2 | H | H | H | c-$C_3H_5$ | 154.6 | 5 | - |
| 24 | S | 2 | H | H | $CH_3$ | $NCCH_2$ | 163.4d | 5 | - |
| 25 | S | 2 | H | H | H | $CH_3$ | 152.1 | 5 | - |
| 26 | O | 2 | H | H | H | $CH_3$ | 135.9 | 5 | - |
| 27 | S | 2 | H | H | H | N-morpholino-$CH_2(CH_3)CH$ | 146.7 | 5 | - |
| 28 | S | 2 | H | H | H | H | 132.0 | 5 | - |
| 29 | $NCH_3$ | 2 | H | H | H | H | 99.8d | 5 | - |
| 30 | NH | 2 | H | H | $CH_3$ | $CH_3CH_2O$ | 128.7 | 8 | - |
| 31 | S | 2 | H | H | H | $CH_3CH_2CH_2$ | 120.3 | 5 | - |
| 32 | S | 2 | H | H | $CH_3$ | $CH_3CH_2CH_2$ | 128.4 | 5 | - |
| 33 | S | 2 | H | H | $CH_3$ | $PhCH_2O$ | 146.0 | 5 | - |
| 34 | O | 2 | H | H | $CH_3$ | $PhCH_2O$ | 134.2 | 5 | - |
| 35 | O | 2 | 5-$CH_3$ | H | $CH_3$ | $CH_3CH_2O$ | 106.0 | 5 | - |

TABLE A

| C | Y | a | $R_1$ | $R_2$ | $R_3$ | X | m.p.(°C) | P | H |
|---|---|---|---|---|---|---|---|---|---|
| 36 | O | 2 | 5-$CH_2$ | H | $CH_3$ | $PhCH_2O$ | 110.3 | 6 | - |
| 37 | O | 2 | 5-$CH_3$ | H | $CH_3$ | $Ph_2CHO$ | 175.0 | 6 | - |
| 38 | S | 2 | H | H | $CH_3$ | 1-menthylO | 168.0 | 7 | - |
| 39 | S | 2 | H | H | $CH_3$ | 4-$CH_3OPhCH_2O$ | 174.1 | 7 | - |
| 40 | S | 2 | H | H | $CH_3$ | Ph | 189.0 | 7 | - |
| 41 | S | 2 | H | H | $CH_3$ | 2-ClPh | 129.0 | 7 | - |
| 42 | S | 2 | H | H | $CH_3$ | 3-ClPh | 192.4 | 7 | - |
| 43 | S | 2 | H | H | $CH_3$ | 4-ClPh | 210.2 | 7 | - |
| 44 | S | 2 | H | H | $CH_3$ | 2-$CH_3Ph$ | 191.0 | 7 | - |
| 45 | S | 2 | H | H | $CH_3$ | 3-$CH_3Ph$ | 158.1 | 7 | - |
| 46 | S | 2 | H | H | $CH_3$ | 4-$CH_3Ph$ | 185.3 | 7 | - |
| 47 | S | 2 | H | H | $CH_3$ | 4-$CF_3Ph$ | 221.8 | 7 | - |
| 48 | S | 2 | H | H | $CH_3$ | 4-$CH_3OPh$ | 169.6 | 7 | - |
| 49 | S | 2 | H | H | $CH_3$ | 4-$(CH_3)_2NPh$ | 175.3 | 8 | - |
| 50 | S | 2 | H | H | $CH_3$ | 4-t-$C_4H_9Ph$ | 162.8 | 7 | - |
| 51 | S | 2 | H | H | $CH_3$ | 3,4-$(CH_3O)_2PhCH_2$ | 174.5 | 7 | - |
| 52 | S | 2 | H | H | $CH_3$ | $PhCH_2$ | 143.0 | 7 | - |
| 53 | S | 2 | H | H | $CH_3$ | 1-naphthyl$CH_2$ | 170.5 | 7 | - |
| 54 | S | 2 | H | H | $CH_3$ | $CH_3CH_2OCH_2CH_2$ | 84.5 | 7 | - |
| 55 | S | 2 | H | H | $CH_3$ | 2-thienyl | 206.1 | 7 | - |
| 56 | S | 2 | H | H | $CH_3$ | 4-pyridinyl | 225.5 | 8 | - |
| 57 | S | 2 | 3-$CH_3$ | H | H | H | 125.2 | 9 | - |
| 58 | S | 2 | 3-$CH_3$ | H | H | $CH_3$ | 112.7 | 9 | - |
| 59 | S | 2 | 3-$CH_3$ | H | H | $CH_3CH_2$ | 97.4 | 5 | - |
| 60 | S | 2 | 3-$CH_3$ | H | H | i-$C_3H_7$ | 140.7 | 5 | - |
| 61 | S | 2 | 3-$CH_3$ | H | H | $CH_3CH_2CH_2$ | 89.9 | 5 | - |
| 62 | S | 2 | 3-$CH_3$ | H | H | c-$C_4H_7$ | 109.9 | 5 | - |
| 63 | S | 2 | 3-$CH_3$ | H | H | c-$C_6H_{11}CH_2CH_2$ | 111.6 | 5 | - |
| 64 | S | 2 | 3-$CH_3$ | H | H | $CH_3O$ | 117.2d | 5 | - |
| 65 | S | 2 | 3-$CH_3$ | H | H | $CH_3CH_2O$ | 85.2 | 5 | - |
| 66 | S | 2 | 3-$CH_3$ | H | H | t-$C_4H_9O$ | 158.6 | 5 | - |
| 67 | S | 2 | 3-$CH_3$ | H | H | 1-menthylO | 137.2 | 5 | - |
| 68 | S | 2 | 3-$CH_3$ | H | H | 4-$CH_3OPhCH_2O$ | 102.1 | 5 | - |
| 69 | S | 2 | 3-$CH_3$ | H | H | Ph | 162.7 | 5 | - |
| 70 | S | 2 | 3-$CH_3$ | H | H | 2-ClPh | 180.7 | 7 | - |
| 71 | S | 2 | 3-$CH_3$ | H | H | 3-ClPh | 211.3 | 10 | - |

TABLE A

| C | Y | a | $R_1$ | $R_2$ | $R_3$ | X | m.p.(°C) | P | H |
|---|---|---|---|---|---|---|---|---|---|
| 72 | S | 2 | 3-$CH_3$ | H | H | 4-ClPh | 192.7 | 10 | - |
| 73 | S | 2 | 3-$CH_3$ | H | H | 2-$CH_3$Ph | 156.8 | 10 | - |
| 74 | S | 2 | 3-$CH_3$ | H | H | 3-$CH_3$Ph | 199.8 | 10 | - |
| 75 | S | 2 | 3-$CH_3$ | H | H | 4-$CH_3$Ph | 205.5 | 10 | - |
| 76 | S | 2 | 3-$CH_3$ | H | H | 4-$CH_3$OPh | 184.1 | 9 | - |
| 77 | S | 2 | 3-$CH_3$ | H | H | 4-($CH_3$)NPh | 231.5 | 11 | - |
| 78 | S | 2 | 3-$CH_3$ | H | H | 4-t-$C_4H_9$Ph | 217.2 | 9 | - |
| 79 | S | 2 | 3-$CH_3$ | H | H | 2-PhOPh | 146.7 | 9 | - |
| 80 | S | 2 | 3-$CH_3$ | H | H | 2-$CH_3$OPh | 156.3 | 9 | - |
| 81 | S | 2 | 3-$CH_3$ | H | H | $PhCH_2$ | 127.2 | 9 | - |
| 82 | S | 2 | 3-$CH_3$ | H | H | 3,4-$(CH_3O)_2PhCH_2$ | 157.2 | 9 | - |
| 83 | S | 2 | 3-$CH_3$ | H | H | 1-naphthyl$CH_2$ | 168.6 | 10 | - |
| 84 | S | 2 | 3-$CH_3$ | H | H | 2-thienyl | 169.4 | 5 | - |
| 85 | S | 2 | 3-$CH_3$ | H | H | 3-pyridinyl | 178.3 | 5 | - |
| 86 | S | 2 | 5-$CH_3$ | H | H | H | 146.9 | 5 | - |
| 87 | S | 2 | 5-$CH_3$ | H | H | $CH_3$ | 155.3 | 5 | - |
| 88 | S | 2 | 5-$CH_3$ | H | H | $CH_3CH_2$ | 138.6 | 5 | - |
| 89 | S | 2 | 5-$CH_3$ | H | H | i-$C_3H_7$ | 173.9 | 5 | - |
| 90 | S | 2 | 5-$CH_3$ | H | H | $CH_3CH_2CH_2$ | 126.2 | 5 | - |
| 91 | S | 2 | 5-$CH_3$ | H | H | c-$C_4H_7$ | 134.0 | 5 | - |
| 92 | S | 2 | 5-$CH_3$ | H | H | c-$C_6H_{11}CH_2CH_2$ | 128.8 | 5 | - |
| 93 | S | 2 | 5-$CH_3$ | H | H | $CH_3O$ | 147.1 | 5 | - |
| 94 | S | 2 | 5-$CH_3$ | H | H | $CH_3CH_2O$ | 118.8 | 5 | - |
| 95 | S | 2 | 5-$CH_3$ | H | H | t-$C_4H_9O$ | 158.9 | 5 | - |
| 96 | S | 2 | 5-$CH_3$ | H | H | 1-menthylO | 156.6 | 5 | - |
| 97 | S | 2 | 5-$CH_3$ | H | H | 4-$CH_3OPhCH_2O$ | 133.5 | 5 | - |
| 98 | S | 2 | 5-$CH_3$ | H | H | Ph | 172.4 | 5 | - |
| 99 | S | 2 | 5-$CH_3$ | H | H | 2-ClPh | 211.9 | 5 | - |
| 100 | S | 2 | 5-$CH_3$ | H | H | 3-ClPh | 172.4 | 5 | - |
| 101 | S | 2 | 5-$CH_3$ | H | H | 4-ClPh | 228.1 | 5 | - |
| 102 | S | 2 | 5-$CH_3$ | H | H | 2-$CH_3$Ph | 179.5 | 5 | - |
| 103 | S | 2 | 5-$CH_3$ | H | H | 3-$CH_3$Ph | 170.4 | 5 | - |
| 104 | S | 2 | 5-$CH_3$ | H | H | 4-$CH_3$Ph | 232.5 | 5 | - |
| 105 | S | 2 | 5-$CH_3$ | H | H | 2-$CH_3CH_2$OPh | 158.7 | 5 | - |
| 106 | S | 2 | 5-$CH_3$ | H | H | 4-$CH_3CH_2$OPh | 201.0 | 5 | - |
| 107 | S | 2 | 5-$CH_3$ | H | H | 2-furanyl | 181.5 | 5 | - |

TABLE A

| C | Y | a | $R_1$ | $R_2$ | $R_3$ | X | m.p.(°C) | P | H |
|---|---|---|---|---|---|---|---|---|---|
| 108 | S | 2 | 5-$CH_3$ | H | H | 3-pyridinyl | 219.6 | 5 | - |
| 109 | S | 2 | 5-$CH_3$ | H | H | 2-PhOPh | 158.8 | 5 | - |
| 110 | S | 2 | 5-$CH_3$ | H | H | chrysanthemyl | 117.3d | 5 | - |
| 111 | S | 2 | 5-$CH_3$ | H | H | $PhCH_2$ | 161.5 | 5 | - |
| 112 | S | 2 | 5-$CH_3$ | H | H | 4-$NO_2$Ph | 224.8 | 12 | - |
| 113 | S | 2 | 5-$CH_3$ | H | H | 2,5-$(CH_3O)_2$Ph | 154.4 | 7 | - |
| 114 | S | 3 | H | H | $CH_3$ | H | 141.5 | 7 | - |
| 115 | S | 3 | H | H | $CH_3$ | $CH_3$ | 148.0 | 7 | - |
| 116 | S | 3 | H | H | $CH_3$ | $CH_3CH_2$ | 129.8 | 7 | - |
| 117 | S | 3 | H | H | $CH_3$ | i-$C_3H_7$ | 161.2 | 7 | - |
| 118 | S | 3 | H | H | $CH_3$ | $CH_3CH_2CH_2$ | 129.2 | 7 | - |
| 119 | S | 3 | H | H | $CH_3$ | $CH_3O$ | 152.0 | 7 | - |
| 120 | S | 3 | H | H | $CH_3$ | $CH_3CH_2O$ | 139.1 | 7 | - |
| 121 | S | 3 | H | H | $CH_3$ | t-$C_4H_9O$ | 167.7 | 7 | - |
| 122 | S | 3 | H | H | $CH_3$ | PhO | 164.1 | 7 | - |
| 123 | S | 3 | H | H | $CH_3$ | 1-menthylO | 171.2 | 7 | - |
| 124 | S | 3 | H | H | $CH_3$ | Ph | 173.4 | 7 | - |
| 125 | S | 3 | H | H | $CH_3$ | 2-ClPh | 125.9 | 7 | - |
| 126 | S | 3 | H | H | $CH_3$ | 4-ClPh | 196.7 | 7 | - |
| 127 | S | 3 | H | H | $CH_3$ | 2-$CH_3CH_2O$Ph | 143.5 | 7 | - |
| 128 | S | 3 | H | H | $CH_3$ | 4-$CH_3CH_2O$Ph | 189.4 | 7 | - |
| 129 | S | 2 | H | H | $CH_2CH_3$ | H | 101.5 | 7 | - |
| 130 | S | 2 | H | H | $CH_2CH_3$ | $CH_3$ | 106.7d | 7 | - |
| 131 | S | 2 | H | H | $CH_2CH_3$ | $CH_3CH_2$ | 130.4 | 7 | - |
| 132 | S | 2 | H | H | $CH_2CH_3$ | i-$C_3H_7$ | 171.4 | 7 | - |
| 133 | S | 2 | H | H | $CH_2CH_3$ | $CH_3CH_2CH_2$ | 122.9 | 7 | - |
| 134 | S | 2 | H | H | $CH_2CH_3$ | c-$C_6H_{11}$ | 214.4 | 7 | - |
| 135 | S | 2 | H | H | $CH_2CH_3$ | c-$C_6H_{11}CH_2$ | 139.4 | 7 | - |
| 136 | S | 2 | H | H | $CH_2CH_3$ | $CH_3CH_2OCH_2CH_2$ | 113.0 | 7 | - |
| 137 | S | 2 | H | H | $CH_2CH_3$ | $CH_3O$ | 154.4 | 7 | - |
| 138 | S | 2 | H | H | $CH_2CH_3$ | $CH_3CH_2O$ | 144.4 | 7 | - |
| 139 | S | 2 | H | H | $CH_2CH_3$ | t-$C_4H_9O$ | 171.4 | 7 | - |
| 140 | S | 2 | H | H | $CH_2CH_3$ | 1-menthylO | 151.7 | 7 | - |
| 141 | S | 2 | H | H | $CH_2CH_3$ | 4-$CH_3O$Ph$CH_2O$ | 140.0 | 7 | - |
| 142 | S | 2 | H | H | $CH_2CH_3$ | 2-furanyl | 142.1 | 7 | - |
| 143 | S | 2 | H | H | $CH_2CH_3$ | 2-thienyl | 186.4 | 7 | - |

TABLE A

| C | Y | a | $R_1$ | $R_2$ | $R_3$ | X | m.p.(°C) | P | H |
|---|---|---|---|---|---|---|---|---|---|
| 144 | S | 2 | H | H | $CH_2CH_3$ | Ph | 161.2 | 7 | - |
| 145 | S | 2 | H | H | $CH_2CH_3$ | 2-ClPh | 151.9 | 7 | - |
| 146 | S | 2 | H | H | $CH_2CH_3$ | 4-ClPh | 154.6 | 7 | - |
| 147 | S | 2 | H | H | $CH_2CH_3$ | 2-$CH_3CH_2$OPh | 127.4 | 7 | - |
| 148 | S | 2 | H | H | $CH_2CH_3$ | 4-$CH_3CH_2$OPh | 135.1 | 7 | - |
| 149 | S | 2 | H | H | $CH_2CH_3$ | 2-$NO_2$Ph | 194.4 | 7 | - |
| 150 | S | 2 | H | H | $CH_2CH_3$ | 4-$CH_3$Ph | 165.0 | 7 | - |
| 151 | S | 2 | H | H | $CH_2CH_3$ | 3,4-($CH_3$O)Ph$CH_2$ | 152.3 | 7 | - |
| 152 | S | 2 | H | H | $CH_2CH_3$ | 4-t-$C_4H_9$Ph | 143.6 | 7 | - |
| 153 | S | 2 | 5-Cl | H | $CH_3$ | H | 171.4d | 7 | - |
| 154 | S | 2 | 5-Cl | H | $CH_3$ | $CH_3$ | 183.0 | 7 | - |
| 155 | S | 2 | 5-Cl | H | $CH_3$ | $CH_3CH_2$ | 167.5 | 7 | - |
| 156 | S | 2 | 5-Cl | H | $CH_3$ | i-$C_3H_7$ | 204.6 | 7 | - |
| 157 | S | 2 | 5-Cl | H | $CH_3$ | $CH_3CH_2CH_2$ | 163.0 | 7 | - |
| 158 | S | 2 | 5-Cl | H | $CH_3$ | c-$C_6H_{11}$ | 201.1 | 7 | - |
| 159 | S | 2 | 5-Cl | H | $CH_3$ | c-$C_6H_{11}CH_2$ | 164.0 | 7 | - |
| 160 | S | 2 | 5-Cl | H | $CH_3$ | $CH_3CH_2OCH_2CH_2$ | 134.0 | 7 | - |
| 161 | S | 2 | 5-Cl | H | $CH_3$ | $CH_3O$ | 175.2 | 7 | - |
| 162 | S | 2 | 5-Cl | H | $CH_3$ | $CH_3CH_2O$ | 165.8 | 7 | - |
| 163 | S | 2 | 5-Cl | H | $CH_3$ | t-$C_4H_9$O | 188.3 | 7 | - |
| 164 | S | 2 | 5-Cl | H | $CH_3$ | PhO | 166.9 | 7 | - |
| 165 | S | 2 | 5-Cl | H | $CH_3$ | 1-menthylO | 196.6 | 7 | - |
| 166 | S | 2 | 5-Cl | H | $CH_3$ | 2-furanyl | 200.7 | 7 | - |
| 167 | S | 2 | 5-Cl | H | $CH_3$ | 2-thienyl | 192.4 | 7 | - |
| 168 | S | 2 | 5-Cl | H | $CH_3$ | 3-pyridinyl | 203.2 | 8 | - |
| 169 | S | 2 | 5-Cl | H | $CH_3$ | Ph | 213.8 | 7 | - |
| 170 | S | 2 | 5-Cl | H | $CH_3$ | 2-ClPh | 229.9d | 13 | - |
| 171 | S | 2 | 5-Cl | H | $CH_3$ | 4-ClPh | 225.2 | 13 | - |
| 172 | S | 2 | 5-Cl | H | $CH_3$ | 2-$CH_3CH_2$OPh | 193.3 | 13 | - |
| 173 | S | 2 | 5-Cl | H | $CH_3$ | 4-$CH_3CH_2$OPh | 206.2 | 13 | - |
| 174 | S | 2 | 5-Cl | H | $CH_3$ | 4-$CH_3$Ph | 207.6 | 13 | - |
| 175 | S | 2 | 5-Cl | H | $CH_3$ | 4-t-$C_4H_9$Ph | 211.8 | 13 | - |
| 176 | S | 2 | 5-Cl | H | $CH_3$ | chrysanthemyl | 186.4 | 7 | - |
| 177 | NH | 2 | H | H | $CH_3$ | 3-pyridinyl | 219.6 | 8 | - |
| 178 | NH | 2 | H | H | $CH_3$ | $CH_3O$ | 119.0 | 8 | - |
| 179 | NH | 2 | H | H | $CH_3$ | 4-ClPh | 197.5 | 8 | + |

TABLE A

| C | Y | a | $R_1$ | $R_2$ | $R_3$ | X | m.p.(°C) | P | H |
|---|---|---|---|---|---|---|---|---|---|
| 180 | NH | 2 | H | H | $CH_3$ | c-$C_6H_{11}$ | 135.7 | 8 | - |
| 181 | NH | 2 | H | H | $CH_3$ | $CH_3$ | 172.7 | 8 | - |
| 182 | NH | 2 | H | H | $CH_3$ | $PhCH_2$ | 169.6 | 8 | - |
| 183 | NH | 2 | H | H | $CH_3$ | 4-$CH_3CH_2OPh$ | 188.2 | 8 | + |
| 184 | NH | 2 | H | H | $CH_3$ | t-$C_4H_9O$ | 161.0 | 8 | - |
| 185 | NH | 2 | H | H | $CH_3$ | 2-ClPh | 126.0 | 8 | - |
| 186 | NPh | 3 | 2-$CH_3$ | 5-$CH_3$ | H | $CH_3$ | 200.1 | 17 | - |
| 187 | NPh | 3 | 2-$CH_3$ | 5-$CH_3$ | H | 4-$CF_3Ph$ | 227.0 | 17 | - |
| 188 | O | 2 | H | H | H | 2-thienyl | 168.4 | 17 | - |
| 189 | O | 2 | H | H | H | 3,4,5-$(CH_3O)_3Ph$ | 175.9 | 17 | - |
| 190 | NPh | 3 | 2-$CH_3$ | 5-$CH_3$ | H | c-$C_6H_{11}$ | 194.9 | 17 | - |
| 191 | NPh | 3 | 2-$CH_3$ | 5-$CH_3$ | H | $CH_3CH_2$ | 184.3 | 17 | - |
| 192 | NH | 2 | H | H | $CH_3$ | $PhCH_2O$ | 172.5 | 18 | - |
| 193 | NH | 2 | H | H | $CH_3$ | c-$C_4H_7$ | 169.0 | 18 | - |
| 194 | NH | 2 | H | H | $CH_3$ | c-$C_6H_{11}CH_2CH_2$ | 109.4 | 18 | - |
| 195 | O | 2 | H | H | H | chrysanthemyl | 133.0 | 19 | - |
| 196 | NH | 2 | H | H | $CH_3$ | $CH_3CH_2CH_2$ | 115.0 | 20 | - |
| 197 | | | | | | | | | |
| 198 | | | | | | | | | |
| 199 | | | | | | | | | |
| 200 | S | 2 | 3-$CH_3$ | H | $CH_3$ | H | 101.6 | 7 | - |
| 201 | S | 2 | 3-$CH_3$ | H | $CH_3$ | $CH_3$ | 149.5 | 7 | - |
| 202 | S | 2 | 3-$CH_3$ | H | $CH_3$ | $CH_3CH_2$ | 108.6 | 7 | - |
| 203 | S | 2 | 3-$CH_3$ | H | $CH_3$ | i-$C_3H_7$ | 111.4 | 7 | - |
| 204 | S | 2 | 3-$CH_3$ | H | $CH_3$ | $CH_3CH_2CH_2$ | 94.2 | 7 | - |
| 205 | S | 2 | 3-$CH_3$ | H | $CH_3$ | c-$C_3H_5$ | 132.8 | 7 | - |
| 206 | S | 2 | 3-$CH_3$ | H | $CH_3$ | c-$C_6H_{11}$ | 131.2 | 7 | - |
| 207 | S | 2 | 3-$CH_3$ | H | $CH_3$ | c-$C_6H_{11}CH_2$ | 104.7 | 7 | - |
| 208 | S | 2 | 3-$CH_3$ | H | $CH_3$ | $CH_3O$ | 71.5 | 7 | - |
| 209 | S | 2 | 3-$CH_3$ | H | $CH_3$ | $CH_3CH_2O$ | 79.9 | 7 | - |
| 210 | S | 2 | 3-$CH_3$ | H | $CH_3$ | t-$C_4H_9O$ | 101.9 | 7 | - |
| 211 | S | 2 | 3-$CH_3$ | H | $CH_3$ | Ph | 102.8 | 7 | - |
| 212 | S | 2 | 3-$CH_3$ | H | $CH_3$ | 4-$CH_3CH_2OPh$ | 128.7 | 7 | - |
| 213 | S | 2 | 5-$CH_3$ | H | $CH_3$ | H | 164.1 | 7 | - |
| 214 | S | 2 | 5-$CH_3$ | H | $CH_3$ | $CH_3$ | 157.7 | 7 | - |
| 215 | S | 2 | 5-$CH_3$ | H | $CH_3$ | $CH_3CH_2$ | 146.4 | 7 | - |

TABLE A

| C | Y | a | $R_1$ | $R_2$ | $R_3$ | X | m.p.(°C) | P | H |
|---|---|---|---|---|---|---|---|---|---|
| 216 | S | 2 | 5-$CH_3$ | H | $CH_3$ | i-$C_3H_7$ | 177.5 | 7 | - |
| 217 | S | 2 | 5-$CH_3$ | H | $CH_3$ | $CH_3CH_2CH_2$ | 134.9 | 7 | - |
| 218 | S | 2 | 5-$CH_3$ | H | $CH_3$ | c-$C_3H_5$ | 181.0 | 7 | - |
| 219 | S | 2 | 5-$CH_3$ | H | $CH_3$ | c-$C_6H_{11}$ | 190.7 | 7 | - |
| 220 | S | 2 | 5-$CH_3$ | H | $CH_3$ | c-$C_6H_{11}CH_2$ | 167.1 | 7 | - |
| 221 | S | 2 | 5-$CH_3$ | H | $CH_3$ | $CH_3CH_2OCH_2CH_2$ | 119.3 | 7 | - |
| 222 | S | 2 | 5-$CH_3$ | H | $CH_3$ | $PhCH_2$ | 180.0 | 7 | - |
| 223 | S | 2 | 5-$CH_3$ | H | $CH_3$ | $CH_3O$ | 156.3 | 7 | - |
| 224 | S | 2 | 5-$CH_3$ | H | $CH_3$ | $CH_3CH_2O$ | 133.5 | 7 | - |
| 225 | S | 2 | 5-$CH_3$ | H | $CH_3$ | t-$C_4H_9O$ | 163.7 | 7 | - |
| 226 | S | 2 | 5-$CH_3$ | H | $CH_3$ | 1-menthylO | 163.2 | 7 | - |
| 227 | S | 2 | 5-$CH_3$ | H | $CH_3$ | $PhCH_2O$ | 147.0 | 7 | - |
| 228 | S | 2 | 5-$CH_3$ | H | $CH_3$ | 2-furanyl | 183.3 | 7 | - |
| 229 | S | 2 | 5-$CH_3$ | H | $CH_3$ | 2-thienyl | 199.4 | 7 | - |
| 230 | S | 2 | 5-$CH_3$ | H | $CH_3$ | 4-pyridinyl | 210.4 | 7 | - |
| 231 | S | 2 | 5-$CH_3$ | H | $CH_3$ | Ph | 179.5 | 7 | - |
| 232 | S | 2 | 5-$CH_3$ | H | $CH_3$ | 4-$CH_3$Ph | 185.9 | 7 | - |
| 233 | S | 2 | 5-$CH_3$ | H | $CH_3$ | 4-ClPh | 215.7 | 7 | - |
| 234 | S | 2 | 5-$CH_3$ | H | $CH_3$ | 4-$CH_3CH_2O$Ph | 173.2 | 7 | - |
| 235 | S | 2 | 5-$CH_3$ | H | $CH_3$ | 4-$(CH_3)_2$NPh | 237.2 | 8 | - |
| 236 | S | 2 | 5-$CH_3$ | H | $CH_3$ | 2,5-$(Cl)_2$Ph | 212.0 | 7 | - |
| 237 | S | 2 | 5-$CH_3$ | H | $CH_3$ | 2-PhOPh | 177.1 | 7 | - |
| 238 | S | 3 | 2-$CH_3$ | 5-$CH_3$ | $CH_3$ | H | 110.0d | 7 | - |
| 239 | S | 3 | 2-$CH_3$ | 5-$CH_3$ | $CH_3$ | $CH_3$ | 144.9 | 7 | - |
| 240 | S | 3 | 2-$CH_3$ | 5-$CH_3$ | $CH_3$ | $CH_3CH_2$ | 134.4 | 7 | - |
| 241 | S | 3 | 2-$CH_3$ | 5-$CH_3$ | $CH_3$ | i-$C_3H_7$ | 124.0 | 7 | - |
| 242 | S | 3 | 2-$CH_3$ | 5-$CH_3$ | $CH_3$ | $CH_3CH_2CH_2$ | 92.7d | 7 | - |
| 243 | S | 3 | 2-$CH_3$ | 5-$CH_3$ | $CH_3$ | c-$C_3H_5$ | 131.6d | 7 | - |
| 244 | S | 3 | 2-$CH_3$ | 5-$CH_3$ | $CH_3$ | chrysanthemyl | 123.2 | 7 | - |
| 245 | S | 3 | 2-$CH_3$ | 5-$CH_3$ | $CH_3$ | $CH_3CH_2OCH_2CH_2$ | 94.3d | 7 | - |
| 246 | S | 3 | 2-$CH_3$ | 5-$CH_3$ | $CH_3$ | c-$C_6H_{11}$ | 151.5 | 7 | - |
| 247 | S | 3 | 2-$CH_3$ | 5-$CH_3$ | $CH_3$ | c-$C_6H_{11}CH_2$ | 101.2 | 7 | - |
| 248 | S | 3 | 2-$CH_3$ | 5-$CH_3$ | $CH_3$ | $CH_3O$ | 104.3 | 7 | - |
| 249 | S | 3 | 2-$CH_3$ | 5-$CH_3$ | $CH_3$ | $CH_3CH_2O$ | 90.0 | 7 | - |
| 250 | S | 3 | 2-$CH_3$ | 5-$CH_3$ | $CH_3$ | t-$C_4H_9O$ | 118.6 | 7 | - |
| 251 | S | 3 | 2-$CH_3$ | 5-$CH_3$ | $CH_3$ | $PhCH_2O$ | 93.5 | 7 | - |

TABLE A

| C | Y | a | $R_1$ | $R_2$ | $R_3$ | X | m.p.(°C) | P | H |
|---|---|---|---|---|---|---|---|---|---|
| 252 | S | 3 | 2-$CH_3$ | 5-$CH_3$ | $CH_3$ | $PhCH_2$ | 95.9 | 7 | - |
| 253 | S | 3 | 2-$CH_3$ | 5-$CH_3$ | $CH_3$ | 2-thienyl | 138.3 | 7 | - |
| 254 | S | 3 | 2-$CH_3$ | 5-$CH_3$ | $CH_3$ | 3-pyridinyl | 135.6 | 8 | - |
| 255 | S | 3 | 2-$CH_3$ | 5-$CH_3$ | $CH_3$ | 2-$CH_3CH_2OPh$ | 154.0 | 7 | - |
| 256 | S | 3 | 2-$CH_3$ | 5-$CH_3$ | $CH_3$ | 3-$CH_3CH_2OPh$ | 125.9 | 7 | - |
| 257 | S | 3 | 2-$CH_3$ | 5-$CH_3$ | $CH_3$ | 4-$CH_3CH_2OPh$ | 144.9 | 7 | - |
| 258 | S | 3 | 2-$CH_3$ | 5-$CH_3$ | $CH_3$ | 2-$CH_3Ph$ | 141.6 | 7 | - |
| 259 | S | 3 | 2-$CH_3$ | 5-$CH_3$ | $CH_3$ | 3-$CH_3Ph$ | 128.1 | 7 | - |
| 260 | S | 3 | 2-$CH_3$ | 5-$CH_3$ | $CH_3$ | 4-$CH_3Ph$ | 128.5 | 7 | - |
| 261 | | | | | | | | | |
| 262 | | | | | | | | | |
| 263 | S | 2 | H | H | H | N-morpholino-$CH_2CH_2$ | 160.7 | 15 | - |
| 264 | O | 2 | H | H | H | N-morpholino-$CH_2CH_2$ | 126.0 | 15 | - |
| 265 | O | 2 | H | H | H | $CH_3O$ | 124.5d | 16 | - |
| 266 | S | 3 | H | H | H | H | 123.1 | 5 | - |
| 267 | S | 3 | H | H | H | $CH_3$ | 148.9 | 5 | - |
| 268 | S | 3 | H | H | H | $CH_3CH_2$ | 109.2 | 5 | - |
| 269 | S | 3 | H | H | H | $CH_3CH_2CH_2$ | 148.5 | 5 | - |
| 270 | S | 3 | H | H | H | i-$C_3H_7$ | 142.4 | 5 | - |
| 271 | S | 3 | H | H | H | Ph | 198.8 | 5 | - |
| 272 | S | 3 | H | H | H | c-$C_6H_{11}$ | 184.3 | 5 | - |
| 273 | S | 3 | H | H | H | c-$C_6H_{11}CH_2CH_2$ | 110.6 | 5 | - |
| 274 | S | 3 | H | H | H | c-$C_4H_7$ | 137.7 | 5 | - |
| 275 | S | 3 | H | H | H | 4-ClPh | 211.5 | 5 | - |
| 276 | S | 3 | H | H | H | 4-$CH_3OPh$ | 187.0 | 5 | - |
| 277 | S | 3 | H | H | H | $CH_3CH_2O$ | 131.9 | 5 | - |
| 278 | S | 3 | H | H | H | t-$C_4H_9O$ | 164.0 | 5 | - |
| 279 | S | 3 | H | H | H | PhO | 166.3 | 5 | - |
| 280 | S | 3 | H | H | H | 4-$(CH_3)_2NPh$ | 261.8 | 5 | - |
| 281 | S | 3 | H | H | H | 3-pyridinyl | 222.3 | 5 | - |
| 282 | S | 3 | H | H | H | 2-thienyl | 211.1 | 5 | - |
| 283 | S | 2 | H | H | $CH_3$ | H | 140.4 | 7 | - |
| 284 | S | 2 | H | H | $CH_3$ | $CH_3$ | 162.7 | 7 | - |
| 285 | S | 2 | H | H | $CH_3$ | $CH_3CH_2$ | 123.5 | 7 | - |

TABLE A

| C | Y | a | $R_1$ | $R_2$ | $R_3$ | X | m.p.(°C) | P | H |
|---|---|---|---|---|---|---|---|---|---|
| 286 | S | 2 | H | H | $CH_3$ | i-$C_3H_7$ | 150.9 | 7 | - |
| 287 | S | 2 | H | H | $CH_3$ | c-$C_6H_{11}$ | 173.2 | 7 | - |
| 288 | S | 2 | H | H | $CH_3$ | c-$C_6H_{11}CH_2CH_2$ | 95.2 | 7 | - |
| 289 | S | 2 | H | H | $CH_3$ | c-$C_4H_7$ | 143.7 | 7 | - |
| 290 | S | 2 | H | H | $CH_3$ | PhO | 147.4 | 7 | - |
| 291 | O | 2 | 5-$CH_3$ | H | H | H | 143.3 | 5 | - |
| 292 | O | 2 | 5-$CH_3$ | H | H | $CH_3$ | 129.9 | 5 | - |
| 293 | O | 2 | 5-$CH_3$ | H | H | $CH_3CH_2$ | 141.7 | 5 | - |
| 294 | O | 2 | 5-$CH_3$ | H | H | $CH_3CH_2CH_2$ | 99.6 | 5 | - |
| 295 | O | 2 | 5-$CH_3$ | H | H | i-$C_3H_7$ | 161.1 | 5 | - |
| 296 | O | 2 | 5-$CH_3$ | H | H | c-$C_6H_{11}$ | 184.2 | 5 | - |
| 297 | O | 2 | 5-$CH_3$ | H | H | c-$C_6H_{11}CH_2$ | 125.9 | 5 | - |
| 298 | O | 2 | 5-$CH_3$ | H | H | c-$C_3H_5$ | 177.0 | 5 | - |
| 299 | O | 2 | 5-$CH_3$ | H | H | $CH_3CH_2OCH_2CH_2$ | 89.2 | 5 | - |
| 300 | O | 2 | 5-$CH_3$ | H | H | $CH_3O$ | 165.5 | 5 | - |
| 301 | O | 2 | 5-$CH_3$ | H | H | $CH_3CH_2O$ | 141.0 | 5 | - |
| 302 | O | 2 | 5-$CH_3$ | H | H | t-$C_4H_9O$ | 149.3 | 5 | - |
| 303 | O | 2 | 5-$CH_3$ | H | H | PhO | 203.1 | 5 | - |
| 304 | O | 2 | 5-$CH_3$ | H | H | l-menthylO | 129.5 | 5 | - |
| 305 | O | 2 | 5-$CH_3$ | H | H | $PhCH_2O$ | 108.1 | 5 | - |
| 306 | O | 2 | 5-$CH_3$ | H | H | Ph | 163.7 | 5 | - |
| 307 | O | 2 | 5-$CH_3$ | H | H | 4-ClPh | 196.1 | 5 | - |
| 308 | O | 2 | 5-$CH_3$ | H | H | 3-ClPh | 177.8 | 5 | - |
| 309 | O | 2 | 5-$CH_3$ | H | H | 2-ClPh | 203.0 | 5 | - |
| 310 | O | 2 | 5-$CH_3$ | H | H | 2,5-$(Cl)_2$Ph | 218.9 | 5 | - |
| 311 | O | 2 | 5-$CH_3$ | H | H | 3,4-$(CH_3O)_2PhCH_2$ | 167.4 | 5 | - |
| 312 | O | 2 | 5-$CH_3$ | H | H | 4-$CH_3$Ph | 187.5 | 17 | - |
| 313 | O | 2 | 5-$CH_3$ | H | H | 3-$CH_3$Ph | 168.3 | 17 | - |
| 314 | O | 2 | 5-$CH_3$ | H | H | 2-$CH_3$Ph | 205.1 | 17 | - |
| 315 | O | 2 | 5-$CH_3$ | H | H | 4-$CH_3CH_2O$Ph | 214.7 | 17 | - |
| 316 | O | 2 | 5-$CH_3$ | H | H | 2-$CH_3CH_2O$Ph | 106.0 | 5 | - |
| 317 | O | 2 | 5-$CH_3$ | H | H | 2-furanyl | 165.2 | 17 | - |
| 318 | O | 2 | 5-$CH_3$ | H | H | 2-thienyl | 171.0 | 17 | - |
| 319 | O | 2 | 5-$CH_3$ | H | H | 4-t-$C_4H_9$Ph | 243.3 | 17 | - |
| 320 | O | 2 | 5-$CH_3$ | H | H | 2-PhOPh | 142.9 | 8 | - |
| 321 | O | 2 | 5-$CH_3$ | H | H | 2-$NO_2$Ph | 194.0 | 17 | - |

TABLE A

| C | Y | a | $R_1$ | $R_2$ | $R_3$ | X | m.p.(°C) | P | H |
|---|---|---|---|---|---|---|---|---|---|
| 322 | 0 | 2 | H | H | $CH_3$ | c-$C_6H_{11}CH_2$ | 132.1 | 18 | - |
| 323 | 0 | 2 | H | H | $CH_3$ | H | 143.6 | 17 | - |
| 324 | 0 | 2 | H | H | $CH_3$ | $CH_3$ | 150.0 | 17 | - |
| 325 | 0 | 2 | H | H | $CH_3$ | $CH_3CH_2$ | 136.4 | 5 | - |
| 326 | 0 | 2 | H | H | $CH_3$ | $CH_3CH_2CH_2$ | 140.1 | 5 | - |
| 327 | 0 | 2 | H | H | $CH_3$ | i-$C_3H_7$ | 140.1 | 5 | - |
| 328 | 0 | 2 | H | H | $CH_3$ | c-$C_6H_{11}$ | 171.5 | 5 | - |
| 329 | 0 | 2 | H | H | $CH_3$ | c-$C_3H_5$ | 204.2 | 17 | - |
| 330 | 0 | 2 | H | H | $CH_3$ | $CH_3CH_2OCH_2CH_2$ | 91.7 | 17 | - |
| 331 | 0 | 2 | H | H | $CH_3$ | $CH_3O$ | 139.7 | 5 | - |
| 332 | 0 | 2 | H | H | $CH_3$ | $CH_3CH_2O$ | 149.8 | 5 | - |
| 333 | 0 | 2 | H | H | $CH_3$ | t-$C_4H_9O$ | 155.5 | 5 | - |
| 334 | 0 | 2 | H | H | $CH_3$ | 1-menthylO | 171.8 | 17 | - |
| 335 | 0 | 2 | H | H | $CH_3$ | Ph | 147.4 | 7 | - |
| 336 | 0 | 2 | H | H | $CH_3$ | 3-ClPh | 120.7 | 7 | - |
| 337 | 0 | 2 | H | H | $CH_3$ | 2-ClPh | 122.1 | 7 | - |
| 338 | 0 | 2 | H | H | $CH_3$ | 2,5-$(Cl)_2$Ph | 174.7 | 7 | - |
| 339 | 0 | 2 | H | H | $CH_3$ | 3,4-$(CH_3O)_2PhCH_2$ | 159.4 | 7 | - |
| 340 | 0 | 2 | H | H | $CH_3$ | 4-$CH_3$Ph | 153.0 | 18 | - |
| 341 | 0 | 2 | H | H | $CH_3$ | 3-$CH_3$Ph | 176.6 | 18 | - |
| 342 | 0 | 2 | H | H | $CH_3$ | 2-$CH_3$Ph | 160.2 | 18 | - |
| 343 | 0 | 2 | H | H | $CH_3$ | 4-$CH_3CH_2O$Ph | 157.2 | 18 | - |
| 344 | 0 | 2 | H | H | $CH_3$ | 2-$CH_3CH_2O$Ph | 183.3 | 17 | - |
| 345 | 0 | 2 | H | H | $CH_3$ | 2-furanyl | 172.3 | 17 | - |
| 346 | 0 | 2 | H | H | $CH_3$ | 2-thienyl | 197.8 | 17 | - |
| 347 | 0 | 2 | H | H | $CH_3$ | 4-t-$C_4H_9$Ph | 179.3 | 17 | - |
| 348 | 0 | 2 | H | H | $CH_3$ | 2-PhOPh | 175.4d | 5 | - |
| 349 | 0 | 2 | H | H | $CH_3$ | 2-$NO_2$Ph | 183.6 | 5 | - |
| 350 | 0 | 2 | H | H | H | $CH_3CH_2$ | 120.4 | 5 | - |
| 351 | 0 | 2 | H | H | H | $CH_3CH_2CH_2$ | 116.3 | 5 | - |
| 352 | 0 | 2 | H | H | H | i-$C_3H_7$ | 159.7 | 5 | - |
| 353 | 0 | 2 | H | H | H | c-$C_3H_5$ | 175.4 | 5 | - |
| 354 | 0 | 2 | H | H | H | c-$C_6H_{11}$ | 187.9 | 5 | - |
| 355 | 0 | 2 | H | H | H | c-$C_6H_{11}CH_2$ | 145.0 | 5 | - |
| 356 | 0 | 2 | H | H | H | c-$C_6H_{11}CH_2CH_2$ | 106.7 | 5 | - |
| 357 | 0 | 2 | H | H | H | $CH_3CH_2OCH_2CH_2$ | 89.8 | 5 | - |

TABLE A

| C | Y | a | $R_1$ | $R_2$ | $R_3$ | X | m.p.(°C) | P | H |
|---|---|---|---|---|---|---|---|---|---|
| 358 | O | 2 | H | H | H | $PhCH_2$ | 158.5 | 5 | - |
| 359 | O | 2 | H | H | H | 1-naphthyl$CH_2$ | 192.0 | 5 | - |
| 360 | O | 2 | H | H | H | t-$C_4H_9O$ | 168.5 | 5 | - |
| 361 | O | 2 | H | H | H | PhO | 149.7 | 5 | - |
| 362 | O | 2 | H | H | H | 1-menthylO | 156.2 | 5 | - |
| 363 | O | 2 | H | H | H | $PhCH_2O$ | 131.9 | 5 | - |
| 364 | O | 2 | H | H | H | 4-$CH_3OPhCH_2O$ | 163.3 | 5 | - |
| 365 | O | 2 | H | H | $CH_3$ | PhO | 158-160 | 5 | - |
| 366 | O | 2 | H | H | $CH_3$ | 4-ClPh | 189.0 | 18 | - |
| 367 | O | 2 | H | H | H | Ph | 184.0 | 5 | - |
| 368 | O | 2 | H | H | H | 2-ClPh | 164.8 | 5 | - |
| 369 | O | 2 | H | H | H | 3-ClPh | 186.9 | 17 | - |
| 370 | O | 2 | H | H | H | 4-ClPh | 216.0 | 17 | - |
| 371 | O | 2 | H | H | H | 2,5-$(Cl)_2$Ph | 202.9 | 17 | - |
| 372 | O | 2 | H | H | H | 2-$CH_3$OPh | 160.5 | 17 | - |
| 373 | O | 2 | H | H | H | 2-$CH_3CH_2$OPh | 138.0 | 5 | - |
| 374 | O | 2 | H | H | H | 4-$CH_3CH_2$OPh | 229.9 | 17 | - |
| 375 | O | 2 | H | H | H | 2-PhOPh | 181.8 | 17 | - |
| 376 | O | 2 | H | H | H | 2,5-$(CH_3O)_2$Ph | 164.7 | 17 | - |
| 377 | O | 2 | H | H | H | 3,5-$(CH_3O)_2$Ph | 196.0 | 17 | - |
| 378 | O | 2 | H | H | H | 4-t-$C_4H_9$Ph | 229.3 | 17 | - |
| 379 | O | 2 | H | H | H | 4-$CF_3$Ph | 233.8 | 17 | - |
| 380 | O | 2 | H | H | H | 4-$(CH_3)_2$NPh | 236.5 | 17 | - |
| 381 | O | 2 | H | H | H | 3,4-$(CH_3O)_2$Ph | 196.2 | 17 | - |
| 382 | O | 2 | H | H | H | 2-$CH_3$Ph | 172.5 | 5 | - |
| 383 | O | 2 | H | H | H | 3-$CH_3$Ph | 151.0 | 5 | - |
| 384 | O | 2 | H | H | H | 4-$CH_3$Ph | 206.2 | 17 | - |
| 385 | O | 2 | H | H | H | 2-furanyl | 204.3 | 5 | - |
| 386 | O | 2 | H | H | H | 4-$CH_3$OPh | 211.9 | 17 | - |
| 387 | O | 2 | H | H | H | 3-$CH_3$OPh | 181.5 | 17 | - |
| 388 | O | 2 | H | H | H | 3-pyridinyl | 192.0 | 17 | - |
| 389 | O | 2 | H | H | H | 4-pyridinyl | 222.4 | 5 | - |
| 390 | N-$CH_3$ | 2 | H | H | H | 4-pyridinyl | 215.7 | 5 | - |
| 391 | N-$CH_3$ | 2 | H | H | H | c-$C_6H_{11}$ | 157.1 | 5 | - |
| 392 | N-$CH_3$ | 2 | H | H | H | c-$C_6H_{11}CH_2$ | 149.5 | 5 | - |
| 393 | N-$CH_3$ | 2 | H | H | H | $CH_3O$ | 130.4 | 5 | - |

TABLE A

| C | Y | a | $R_1$ | $R_2$ | $R_3$ | X | m.p.(°C) | P | H |
|---|---|---|---|---|---|---|---|---|---|
| 394 | NH | 2 | H | H | $CH_3$ | Ph | 170.0 | 18 | - |
| 395 | NH | 2 | H | H | H | $CH_3CH_2CH_2$ | 192.3d | 5 | - |
| 396 | NH | 2 | H | H | H | Ph | 197.2d | 5 | + |
| 397 | NH | 2 | H | H | H | 4-ClPh | 238.8d | 5 | + |
| 398 | NH | 2 | H | H | H | $CH_3CH_2O$ | 151.2d | 5 | - |
| 399 | NH | 2 | H | H | H | 4-$CH_3CH_2O$Ph | 207.2d | 5 | + |
| 400 | NH | 2 | H | H | H | c-$C_4H_7$ | 199.5d | 5 | - |
| 401 | NH | 2 | H | H | H | $CH_3CH_2$ | 176.9d | 5 | - |
| 402 | NH | 2 | H | H | H | i-$C_3H_7$ | 166.0 | 5 | + |
| 403 | NH | 2 | H | H | H | t-$C_4H_9O$ | 181.5d | 5 | - |
| 404 | NH | 2 | H | H | H | c-$C_6H_{11}CH_2CH_2$ | 162.9 | 5 | - |
| 405 | NH | 2 | H | H | H | $CH_3$ | 193.9 | 5 | - |
| 406 | NH | 2 | H | H | H | $CH_3CH_2CH_2$ | 194.9 | 5 | - |
| 407 | NH | 2 | H | H | H | c-$C_3H_5$ | 207.7d | 5 | + |
| 408 | NH | 2 | H | H | H | 4-$CF_3$Ph | 239.6d | 5 | - |
| 409 | NH | 2 | H | H | H | 3-ClPh | 197.3d | 5 | - |
| 410 | NH | 2 | H | H | H | 2-ClPh | 102.7 | 5 | - |
| 411 | NH | 2 | H | H | H | 1-menthylO | 163.5 | 5 | - |
| 412 | NH | 2 | H | H | H | 2-thienyl | 255.1d | 12 | - |
| 413 | NH | 2 | H | H | H | 4-$CH_3$Ph | 230.4d | 5 | - |
| 414 | NH | 2 | H | H | H | 3-$CH_3$Ph | 199.2 | 5 | - |
| 415 | NH | 2 | H | H | H | 2-$CH_3$Ph | 94.3 | 5 | - |
| 416 | NH | 2 | H | H | H | 4-t-$C_4H_9$Ph | 133.0 | 5 | - |
| 417 | NH | 2 | H | H | H | 2-furanyl | 253.4d | 5 | - |
| 418 | NH | 2 | H | H | H | 2-$CH_3CH_2O$Ph | 162.8 | 5 | - |
| 419 | NH | 2 | H | H | H | PhOPh | 156.8 | 5 | - |
| 420 | NH | 2 | H | H | H | 2-$CH_3O$Ph | 130.9 | 5 | + |
| 421 | NH | 2 | H | H | H | 1-naphthyl$CH_2$ | 213.6 | 5 | - |
| 422 | NH | 2 | H | H | H | 3,4-$(CH_3O)_2$Ph$CH_2$ | 164.3 | 5 | - |
| 423 | NH | 2 | H | H | H | 4-$CH_3O$Ph$CH_2O$ | 134.7 | 5 | - |
| 424 | NH | 2 | H | H | H | 3-pyridinyl | 240.2d | 5 | - |
| 425 | NH | 2 | H | H | H | Ph$CH_2$ | 228.3d | 5 | - |
| 426 | NH | 2 | H | H | H | Ph$CH_2O$ | 125.4 | 5 | - |
| 427 | NH | 2 | H | H | H | c-$C_6H_{11}$ | 210.2d | - | |
| 428 | NH | 2 | H | H | H | c-$C_6H_{11}CH_2$ | 203.2 | 5 | - |
| 429 | NH | 2 | H | H | H | 4-pyridinyl | 220.6d | 5 | + |

TABLE A

| C | Y | a | $R_1$ | $R_2$ | $R_3$ | X | m.p.(°C) | P | H |
|---|---|---|---|---|---|---|---|---|---|
| 430 | $N-CH_3$ | 2 | H | H | H | $CH_3$ | 155.0 | 5 | - |
| 431 | $N-CH_3$ | 2 | H | H | H | $CH_3CH_2$ | 143.1 | 5 | - |
| 432 | $N-CH_3$ | 2 | H | H | H | $CH_3CH_2CH_2$ | 101.7 | 5 | - |
| 433 | $N-CH_3$ | 2 | H | H | H | $i-C_3H_7$ | 124.5 | 5 | - |
| 434 | $N-CH_3$ | 2 | H | H | H | $c-C_6H_{11}CH_2CH_2$ | 116.6 | 5 | - |
| 435 | $N-CH_3$ | 2 | H | H | H | 4-ClPh | 202.7 | 5 | - |
| 436 | $N-CH_3$ | 2 | H | H | H | 3-ClPh | 182.2 | 5 | - |
| 437 | $N-CH_3$ | 2 | H | H | H | 2-ClPh | 173.5 | 5 | - |
| 438 | $N-CH_3$ | 2 | H | H | H | $4-CH_3CH_2OPh$ | 195.1 | 5 | - |
| 439 | $N-CH_3$ | 2 | H | H | H | 3-pyridinyl | 80.1 | 5 | - |
| 440 | $N-CH_3$ | 2 | H | H | H | $4-CH_3Ph$ | 207.4 | 5 | - |
| 441 | $N-CH_3$ | 2 | H | H | H | $4-t-C_4H_9Ph$ | 220.7 | 5 | - |
| 442 | $N-CH_3$ | 2 | H | H | H | $t-C_4H_9O$ | 167.4d | 5 | + |
| 443 | $N-CH_3$ | 2 | H | H | H | $4-CH_3OPh$ | 214.7 | 5 | - |
| 444 | N-Ph | 3 | $2-CH_3$ | $5-CH_3$ | H | Ph | 216.9 | 5 | - |
| 445 | N-Ph | 3 | $2-CH_3$ | $5-CH_3$ | H | $CH_3CH_2O$ | 175.3 | 5 | - |
| 446 | N-Ph | 3 | $2-CH_3$ | $5-CH_3$ | H | $CH_3CH_2CH_2$ | 140.6 | 5 | - |
| 447 | S | 2 | H | H | 2-thienyl-CHCH | $CH_3O$ | 155.5 | 7 | - |
| 448 | S | 2 | $4-CH_3$ | H | $CH_3$ | $4-CH_3CH_2OPh$ | 168.1 | 7 | - |
| 449 | S | 2 | $4-CH_3$ | H | $CH_3$ | 4-ClPh | 183.6 | 7 | - |
| 450 | S | 2 | $4-CH_3$ | H | $CH_3$ | Ph | 170.6 | 7 | - |
| 451 | S | 2 | $4-CH_3$ | H | $CH_3$ | $c-C_6H_{11}CH_2$ | 139.2 | 7 | - |
| 452 | S | 2 | $4-CH_3$ | H | $CH_3$ | $PhCH_2O$ | 150.9 | 7 | - |
| 453 | S | 2 | $4-CH_3$ | H | $CH_3$ | $CH_3CH_2O$ | 132.0 | 7 | - |
| 454 | S | 2 | $4-CH_3$ | H | $CH_3$ | $CH_3O$ | 156.9 | 7 | - |
| 455 | S | 2 | $4-CH_3$ | H | $CH_3$ | $i-C_3H_7$ | 178.6 | 7 | - |
| 456 | S | 2 | $4-CH_3$ | H | $CH_3$ | $CH_3CH_2CH_2$ | 127.8 | 7 | - |
| 457 | S | 2 | $4-CH_3$ | H | $CH_3$ | $CH_3CH_2$ | 138.7 | 7 | - |
| 458 | S | 2 | H | H | $c-C_3H_5$ | $4-CH_3CH_2OPh$ | 141.0 | 7 | - |
| 459 | S | 2 | H | H | $4-CH_3OPh$ | $PhCH_2O$ | 132.0 | 7 | - |
| 460 | S | 2 | H | H | $4-CH_3OPh$ | $CH_3CH_2O$ | 120.6 | 7 | - |
| 461 | S | 2 | H | H | $4-CH_3OPh$ | $CH_3O$ | 125.0 | 7 | - |
| 462 | S | 2 | H | H | Ph | $2-CH_3OPh$ | 178.3 | 7 | - |
| 463 | S | 2 | H | H | Ph | $2-NO_2Ph$ | 194.3 | 7 | - |
| 464 | S | 2 | H | H | Ph | 2-PhOPh | 170.2 | 7 | - |

TABLE A

| C | Y | a | $R_1$ | $R_2$ | $R_3$ | X | m.p.(°C) | P | H |
|---|---|---|---|---|---|---|---|---|---|
| 465 | S | 2 | H | H | Ph | 2,5-$(Cl)_2$Ph | 146.0 | 7 | - |
| 466 | S | 2 | H | H | Ph | 2-furanyl | 150.7 | 7 | - |
| 467 | S | 2 | H | H | Ph | 3,4-$(CH_3O)_2$Ph | 157.6 | 7 | - |
| 468 | S | 2 | H | H | Ph | 4-$CF_3$Ph | 143.2 | 7 | - |
| 469 | S | 2 | H | H | Ph | 3-ClPh | 140.5 | 7 | - |
| 470 | S | 2 | H | H | Ph | 2-ClPh | 98.2 | 7 | - |
| 471 | S | 2 | H | H | Ph | 4-$CH_3$Ph | 140.8 | 7 | - |
| 472 | S | 2 | H | H | Ph | 3-$CH_3$Ph | 72.9 | 7 | - |
| 473 | S | 2 | H | H | Ph | 2-$CH_3$Ph | 93.2 | 7 | - |
| 474 | S | 2 | H | H | Ph | 4-$CH_3CH_2O$Ph | 118.1 | 7 | - |
| 475 | S | 2 | H | H | Ph | 2-$CH_3CH_2O$Ph | 173.0 | 7 | - |
| 476 | S | 2 | H | H | Ph | $PhCH_2O$ | 107.0 | 7 | - |
| 477 | S | 2 | H | H | Ph | $CH_3CH_2O$ | 115.2 | 7 | - |
| 478 | S | 2 | H | H | Ph | $CH_3O$ | 121.9 | 7 | - |
| 479 | S | 2 | H | H | Ph | c-$C_6H_{11}CH_2$ | 100.7 | 7 | - |
| 480 | S | 2 | H | H | Ph | c-$C_6H_{11}$ | 118.2 | 7 | - |
| 481 | S | 2 | H | H | Ph | c-$C_3H_5$ | 126.0 | 7 | - |
| 482 | S | 2 | H | H | Ph | $CH_3CH_2CH_2$ | 71.2 | 7 | - |
| 483 | S | 3 | 2-$CH_3$ | 5-$CH_3$ | $CH_3$ | Ph | 163.3 | 7 | - |
| 487 | $NCH_3$ | 2 | H | H | H | c-$C_4H_7$ | 115.2 | 5 | - |
| 488 | $NCH_3$ | 2 | H | H | H | c-$C_3H_5$ | 153.7 | 5 | - |
| 489 | $NCH_3$ | 2 | H | H | H | 3-$CH_3$Ph | 186.2 | 17 | - |
| 490 | $NCH_3$ | 2 | H | H | H | 2-$CH_3$Ph | 158.5 | 17 | - |
| 491 | $NCH_3$ | 2 | H | H | H | 1-naphthyl$CH_2$ | 166.3 | 17 | - |
| 492 | $NCH_3$ | 2 | H | H | H | 3,4-$(CH_3O)_2PhCH_2$ | 167.4 | 17 | - |
| 493 | $NCH_3$ | 2 | H | H | H | 4-$CH_3OPhCH_2O$ | 126.8 | 5 | - |
| 494 | $NCH_3$ | 2 | H | H | H | $PhCH_2O$ | 109.6 | 5 | - |
| 495 | $NCH_3$ | 2 | H | H | H | 2-thienyl | 174.8 | 17 | - |
| 496 | $NCH_3$ | 2 | H | H | H | 2-furanyl | 147.5 | 17 | - |
| 497 | $NCH_3$ | 2 | H | H | H | 3-$CH_3O$Ph | 133.0 | 17 | - |
| 498 | $NCH_3$ | 2 | H | H | H | 3,5-$(CH_3O)_2$Ph | 156.3 | 17 | - |
| 499 | $NCH_3$ | 2 | H | H | H | 1-naphthyl | 196.3 | 17 | - |
| 500 | $NCH_3$ | 2 | H | H | H | 2-$CH_3CH_2O$Ph | 139.2 | 5 | - |
| 501 | $NCH_3$ | 2 | H | H | H | 2,4-$(Cl)_2$Ph | 139.9 | 17 | - |
| 502 | $NCH_3$ | 2 | H | H | H | 2-pyridinyl | 138.5 | 5 | - |
| 503 | NH | 2 | H | H | H | 4-$CH_3O$Ph | 169.7 | 5 | - |

TABLE A

| C | Y | a | $R_1$ | $R_2$ | $R_3$ | X | m.p.(°C) | P | H |
|---|---|---|---|---|---|---|---|---|---|
| 504 | NH | 2 | H | H | H | H | 166.9 | 5 | - |
| 505 | NH | 2 | H | H | H | 3-$CH_3$OPh | 103.2 | 5 | - |
| 506 | $NCH_3$ | 2 | H | H | H | PhO | 133.6 | 5 | - |
| 507 | $NCH_3$ | 2 | H | H | H | 1-menthylO | 118.1 | 5 | - |
| 508 | NPh | 3 | 2-$CH_3$ | 5-$CH_3$ | H | $CH_3$O | 173.2 | 5 | - |
| 509 | NPh | 3 | 2-$CH_3$ | 5-$CH_3$ | H | 4-ClPh | 218.2 | 17 | - |
| 510 | NPh | 3 | 2-$CH_3$ | 5-$CH_3$ | H | 4-$CH_3CH_2$OPh | 239.6 | 17 | - |
| 511 | $NCH_3$ | 2 | H | H | $CH_3$ | 4-$CH_3$Ph | 139.6 | 13 | - |
| 512 | $NCH_3$ | 2 | H | H | $CH_3$ | 3-$CH_3$Ph | 141.1 | 13 | - |
| 513 | $NCH_3$ | 2 | H | H | $CH_3$ | 1-naphthyl$CH_2$ | 220.2 | 13 | - |
| 514 | $NCH_3$ | 2 | H | H | $CH_3$ | 2-$CH_3CH_2$OPh | 143.6 | 13 | - |
| 515 | $NCH_3$ | 2 | H | H | $CH_3$ | 4-$CH_3CH_2$OPh | 142.4 | 13 | - |
| 516 | $NCH_3$ | 2 | H | H | $CH_3$ | $CH_3CH_2$O | 90.1 | 13 | - |
| 517 | $NCH_3$ | 2 | H | H | $CH_3$ | c-$C_6H_{11}$ | 141.7 | 13 | - |
| 518 | $NCH_3$ | 2 | H | H | $CH_3$ | c-$C_3H_5$ | 155.0 | 13 | - |
| 519 | $NCH_3$ | 2 | H | H | $CH_3$ | c-$C_4H_7$ | 123.6 | 13 | - |
| 520 | $NCH_3$ | 2 | H | H | $CH_3$ | 1-menthylO | 100.4 | 13 | - |
| 521 | $NCH_3$ | 2 | H | H | $CH_3$ | 2,5-$(Cl)_2$Ph | 224.9 | 13 | - |
| 522 | NPh | 3 | 2-$CH_3$ | 5-$CH_3$ | H | t-$C_4H_9$O | 173.7 | 17 | - |
| 523 | NPh | 3 | 2-$CH_3$ | 5-$CH_3$ | H | 3-pyridinyl | 171.7 | 17 | - |
| 524 | NPh | 3 | 2-$CH_3$ | 5-$CH_3$ | H | 2-ClPh | 163.8 | 17 | + |
| 525 | NPh | 3 | 2-$CH_3$ | 5-$CH_3$ | H | 3-ClPh | 194.7 | 17 | - |
| 526 | NPh | 3 | 2-$CH_3$ | 5-$CH_3$ | H | c-$C_6H_{11}CH_2CH_2$ | 179.8 | 17 | - |
| 527 | NPh | 3 | 2-$CH_3$ | 5-$CH_3$ | H | c-$C_6H_{11}CH_2$ | 164.3 | 17 | - |
| 528 | NPh | 3 | 2-$CH_3$ | 5-$CH_3$ | H | i-$C_3H_7$ | 175.5 | 17 | + |
| 529 | NPh | 3 | 2-$CH_3$ | 5-$CH_3$ | H | 4-$CH_3$OPh | 217.3 | 17 | - |
| 530 | NPh | 3 | 2-$CH_3$ | 5-$CH_3$ | H | 4-t-$C_4H_9$Ph | >300 | 17 | - |
| 531 | NPh | 3 | 2-$CH_3$ | 5-$CH_3$ | H | Ph$CH_2$ | 171.7 | 17 | - |
| 532 | NPh | 3 | 2-$CH_3$ | 5-$CH_3$ | H | 4-$CH_3$Ph | 216.3 | 17 | - |
| 533 | NPh | 3 | 2-$CH_3$ | 5-$CH_3$ | H | c-$C_4H_7$ | 170.6 | 17 | - |
| 534 | NPh | 3 | 2-$CH_3$ | 5-$CH_3$ | H | 3-$CH_3$Ph | 243.5 | 17 | - |
| 535 | NPh | 3 | 2-$CH_3$ | 5-$CH_3$ | H | 2-$CH_3$Ph | 204.6 | 17 | - |
| 536 | NPh | 3 | 2-$CH_3$ | 5-$CH_3$ | H | 2-$CH_3CH_2$OPh | 172.9 | 17 | - |
| 537 | NPh | 3 | 2-$CH_3$ | 5-$CH_3$ | H | 1-menthylO | 151.1 | 5 | - |
| 538 | NPh | 3 | 2-$CH_3$ | 5-$CH_3$ | H | 2-PhOPh | 200.5 | 17 | - |
| 539 | NPh | 3 | 2-$CH_3$ | 5-$CH_3$ | H | 2-$CH_3$OPh | 194.5 | 17 | - |

TABLE A

| C | Y | a | $R_1$ | $R_2$ | $R_3$ | X | m.p.(°C) | P | H |
|---|---|---|---|---|---|---|---|---|---|
| 540 | NPh | 3 | 2-$CH_3$ | 5-$CH_3$ | H | 1-naphthyl$CH_2$ | 213.1 | 17 | - |
| 541 | NPh | 3 | 2-$CH_3$ | 5-$CH_3$ | H | 3,4-$(CH_3O)_2PhCH_2$ | 172.7 | 17 | + |
| 542 | NPh | 3 | 2-$CH_3$ | 5-$CH_3$ | H | 4-$CH_3OPhCH_2O$ | 153.0 | 17 | - |
| 543 | NPh | 3 | 2-$CH_3$ | 5-$CH_3$ | H | 3-$CH_3OPh$ | 217.8 | 17 | - |
| 544 | NPh | 3 | 2-$CH_3$ | 5-$CH_3$ | H | 3,5-$(CH_3O)_2Ph$ | 263.0 | 17 | - |
| 545 | NPh | 3 | 2-$CH_3$ | 5-$CH_3$ | H | 2,5-$(CH_3O)_2Ph$ | 228.7 | 17 | - |
| 546 | NPh | 3 | 2-$CH_3$ | 5-$CH_3$ | H | 2-thienyl | 201.1 | 17 | - |
| 547 | NPh | 3 | 2-$CH_3$ | 5-$CH_3$ | H | 2-furanyl | >300 | 17 | + |
| 548 | $NCH_3$ | 2 | H | H | H | 2,5-$(Cl)_2Ph$ | 166.9 | 17 | - |
| 549 | NH | 2 | H | H | H | $CH_3O$ | 138.5 | 5 | - |
| 550 | NH | 2 | H | H | H | 3,5-$(CH_3O)_2Ph$ | 220.6 | 5 | - |
| 551 | NH | 2 | H | H | H | 2,5-$(CH_3O)_2Ph$ | 241.3 | 17 | - |
| 552 | $NCH_3$ | 2 | H | H | H | 2-PhOPh | 111.3 | 17 | + |
| 553 | $NCH_3$ | 2 | H | H | H | 2,5-$(CH_3O)_2Ph$ | 118.0 | 5 | - |
| 554 | NH | 2 | H | H | H | 2,5-$(Cl)_2Ph$ | 185.4 | 5 | + |
| 555 | NH | 2 | H | H | H | 3,4-$(CH_3O)_2Ph$ | 215.9 | 17 | + |
| 556 | $NCH_3$ | 2 | H | H | H | 3,4,5-$(CH_3O)_3Ph$ | 191.7 | 17 | + |
| 557 | $NCH_3$ | 2 | H | H | $CH_3$ | 3,4-$(CH_3O)_2PhCH_2$ | 137.3 | 13 | - |
| 558 | $NCH_3$ | 2 | H | H | H | 2-$CH_3OPh$ | 65.9 | 5 | + |
| 559 | $NCH_3$ | 2 | H | H | $CH_3$ | 2-ClPh | 173.8 | 13 | - |
| 560 | $NCH_3$ | 2 | H | H | $CH_3$ | 2-$CH_3Ph$ | 129.3 | 13 | - |
| 561 | $NCH_3$ | 2 | H | H | $CH_3$ | $PhCH_2O$ | 103.0 | 13 | - |
| 562 | NH | 2 | H | H | H | 4-$(CH_3)_2NPh$ | 263.1 | 17 | - |
| 563 | NH | 2 | H | H | H | 3,4,5-$(CH_3O)_3Ph$ | 232.1 | 17 | + |
| 564 | $NCH_3$ | 2 | H | H | H | 4-$(CH_3)_2NPh$ | 225.5 | 17 | - |
| 565 | $NCH_3$ | 2 | H | H | H | 3-$CH_3CH_2O$,4-HOPh | 268.1 | 17 | - |
| 566 | NH | 2 | H | H | H | $C_2H_5OCH_2CH_2$ | 107.1 | 5 | - |
| 567 | O | 3 | 2-$CH_3$ | 5-$CH_3$ | $CH_3$ | $CH_3CH_2O$ | 96.7 | 13 | - |
| 568 | O | 3 | 2-$CH_3$ | 5-$CH_3$ | $CH_3$ | n-$C_3H_7$ | 104.3 | 13 | - |
| 569 | O | 3 | 2-$CH_3$ | 5-$CH_3$ | $CH_3$ | Ph | 155.1 | 13 | - |
| 570 | O | 3 | 2-$CH_3$ | 5-$CH_3$ | $CH_3$ | 4-pyridinyl | 152.8 | 18 | - |
| 571 | O | 3 | 2-$CH_3$ | 5-$CH_3$ | $CH_3$ | c-$C_6H_{11}$ | 144.4 | 13 | - |
| 572 | O | 3 | 2-$CH_3$ | 5-$CH_3$ | $CH_3$ | c-$C_6H_{11}CH_2CH_2$ | 107.8 | 13 | - |
| 573 | O | 3 | 2-$CH_3$ | 5-$CH_3$ | $CH_3$ | $PhCH_2O$ | 106.5 | 13 | - |
| 574 | O | 3 | 2-$CH_3$ | 5-$CH_3$ | $CH_3$ | 2-ClPh | 160.7 | 13 | - |
| 575 | O | 3 | 2-$CH_3$ | 5-$CH_3$ | $CH_3$ | $C_2H_5$ | 116.3 | 13 | - |

TABLE A

| C | Y | a | $R_1$ | $R_2$ | $R_3$ | X | m.p.(°C) | P | H |
|---|---|---|---|---|---|---|---|---|---|
| 576 | O | 3 | 2-$CH_3$ | 5-$CH_3$ | $CH_3$ | 4-$CH_3$Ph | 158.0 | 13 | - |
| 577 | O | 3 | 2-$CH_3$ | 5-$CH_3$ | $CH_3$ | 4-$CH_3CH_2$OPh | 138.0 | 13 | - |
| 578 | O | 3 | 2-$CH_3$ | 5-$CH_3$ | $CH_3$ | 3,5-$(CH_3O)_2$Ph | 155.0 | 13 | - |
| 579 | O | 3 | 2-$CH_3$ | 5-$CH_3$ | $CH_3$ | 4-t-$C_4H_9$Ph | 110-112 | 13 | - |
| 580 | O | 3 | 2-$CH_3$ | 5-$CH_3$ | $CH_3$ | 4-$CF_3$Ph | 144.0 | 13 | - |
| 581 | O | 3 | 2-$CH_3$ | 5-$CH_3$ | $CH_3$ | 4-$CH_3CH_2$OPh | 157.0 | 5 | - |
| 582 | O | 3 | 2-$CH_3$ | 5-$CH_3$ | $CH_3$ | 2-furanyl | 125.0 | 13 | - |
| 583 | O | 3 | 2-$CH_3$ | 5-$CH_3$ | $CH_3$ | 2-thienyl | 155.0 | 13 | - |
| 584 | O | 2 | 5-$C_2H_5$ | H | H | $C_2H_5O$ | 112.3 | 13 | - |
| 585 | O | 2 | 5-$C_2H_5$ | H | H | Ph | 188.7 | 13 | - |
| 586 | O | 2 | 5-$C_2H_5$ | H | H | 4-pyridinyl | 195.5 | 17 | - |
| 587 | O | 2 | 5-$C_2H_5$ | H | H | c-$C_6H_{11}$ | 164.9 | 13 | - |
| 588 | O | 2 | H | H | n-$C_5H_{11}$ | Ph | 164.3 | 13 | - |
| 589 | O | 2 | H | H | n-$C_5H_{11}$ | c-$C_6H_{11}$ | 180.1 | 13 | - |
| 590 | O | 2 | 5-$C_2H_5$ | H | H | n-$C_3H_7$ | 108.2 | 13 | - |
| 591 | O | 2 | H | H | n-$C_5H_{11}$ | n-$C_3H_7$ | 102.3 | 13 | - |
| 592 | S | 2 | H | H | Ph | 2-thienyl | 154.8 | 13 | - |
| 593 | S | 2 | H | H | 4-$CH_3$OPh | 4-$C_2H_5$OPh | 167.7 | 13 | - |
| 594 | S | 2 | H | H | c-$C_3H_5$ | $CH_3O$ | 119.9 | 13 | - |
| 595 | S | 2 | H | H | $CH_3$ | 2,4,5-$(Cl)_3$PhO$CH_2$ | 221.4 | 14 | - |
| 596 | S | 2 | H | H | H | 2,4,5-$(Cl)_3$PhO$CH_2$ | 242.4 | 14 | - |
| 597 | S | 2 | H | H | H | 4-$(CH_3)_2$NPh | 206.0 | 17 | - |
| 598 | S | 2 | H | H | H | 4-t-$C_4H_9$Ph | 228.3 | 17 | - |
| 599 | S | 2 | H | H | H | PhO | 167.2 | 17 | - |
| 600 | S | 2 | H | H | H | $CH_3O$ | 154.1 | 5 | - |
| 601 | S | 2 | H | H | H | 2-$CH_3$OPh | 173.0 | 5 | - |
| 602 | S | 2 | H | H | H | 4-$CH_3$OPh | 213.3 | 5 | - |
| 603 | S | 2 | H | H | H | 2-$CH_3CH_2$OPh | 144.8 | 5 | - |
| 604 | S | 2 | H | H | H | c-$C_6H_{11}CH_2CH_2$ | 131.5 | 5 | - |
| 605 | S | 2 | H | H | H | c-$C_6H_{11}$ | 209.9 | 5 | - |
| 606 | S | 2 | H | H | H | i-$C_3H_7$ | 156.3 | 5 | - |

TABLE I

H. Contortus

| Compound No. | % Clearance P.O. | I.P. |
|---|---|---|
| 1 | 100 | 100 |
| 2 | N.T. | N.T. |
| 3 | N.T. | N.T. |
| 4 | 0 | N.T. |
| 5 | N.T. | N.T. |
| 6 | 100 | 98.3 |
| 7 | 72.7 | N.T. |
| 8 | 0 | N.T. |
| 9 | N.T. | N.T. |
| 10 | N.T. | N.T. |
| 11 | 95.9 | N.T. |
| 12 | N.T. | N.T. |
| 13 | N.T. | N.T. |
| 14 | N.T. | N.T. |
| 15 | N.T. | N.T. |
| 16 | N.T. | N.T. |
| 17 | N.T. | N.T. |
| 18 | 90.9 | N.T. |
| 19 | Toxic | N.T. |
| 20 | N.T. | N.T. |
| 21 | N.T. | N.T. |
| 22 | 98.8 | 99.9-99.8 |
| 23 | 27.3 | N.T. |
| 24 | N.T. | N.T. |
| 25 | 100 | Toxic |
| 26 | N.T. | N.T. |
| 27 | N.T. | N.T. |
| 28 | 100 | 100 |
| 29 | N.T. | N.T. |
| 30 | 99.2 | N.T. |
| 31 | 99.1 | N.T. |

TABLE I (cont'd)

H. Contortus

% Clearance

| Compound No. | P.O. | I.P. |
|---|---|---|
| 32 | 100 | 100 |
| 33 | N.T. | 37.7 |
| 34 | 67.5 | N.T. |
| 35 | 100 | 81.1-95.7 |
| 36 | N.T. | N.T. |
| 37 | N.T. | N.T. |
| 38 | N.T. | N.T. |
| 39 | 2.8 | 47.8 |
| 40 | * | N.T. |
| 41 | 59.3 | N.T. |
| 42 | 0 | N.T. |
| 43 | 72.9 | N.T. |
| 44 | 0 | N.T. |
| 45 | 97.4 | N.T. |
| 46 | 72.1 | 22.7 |
| 47 | 12.1 | 14.0 |
| 48 | 97.7 | N.T. |
| 49 | 92.6 | N.T. |
| 50 | 0 | N.T. |
| 51 | 45.5 | 43.6 |
| 52 | 64.5 | N.T. |
| 53 | 75.2 | N.T. |
| 54 | 98.1 | N.T. |
| 55 | 97.1 | N.T. |
| 56 | 93.7[a] | N.T. |
| 57 | 94.9[b] | N.T. |
| 58 | 100 | N.T. |
| 59 | 100 | N.T. |
| 60 | 99.7 | N.T. |
| 61 | 99.6 | N.T. |
| 62 | 100 | N.T. |

TABLE I (cont'd)

H. Contortus

| Compound No. | % Clearance P.O. | % Clearance I.P. |
|---|---|---|
| 63 | N.T. | N.T. |
| 64 | 99.8 | 100 |
| 65 | * | N.T. |
| 66 | 97.0 | 99.4 |
| 67 | 0 | 68.3 |
| 68 | 99.5 | N.T. |
| 69 | 99.2 | N.T. |
| 70 | 98.7 | N.T. |
| 71 | 85.7 | N.T. |
| 72 | 98.9 | N.T. |
| 73 | 96.6 | 6.4 |
| 74 | 99.4 | N.T. |
| 75 | 97.9 | N.T. |
| 76 | * | N.T. |
| 77 | 17.6 | N.T. |
| 78 | 19.7 | N.T. |
| 79 | N.T. | N.T. |
| 80 | 72.9 | N.T. |
| 81 | 99.4 | N.T. |
| 82 | 28.9 | N.T. |
| 83 | 60.4 | N.T. |
| 84 | 99.7 | N.T. |
| 85 | 99.7 | N.T. |
| 86 | 100 | N.T. |
| 87 | 99.9 | N.T. |
| 88 | 96.4 | N.T. |
| 89 | 100 | N.T. |
| 90 | N.T. | N.T. |
| 91 | 92.7[b] | N.T. |
| 92 | * | N.T. |
| 93 | N.T. | N.T. |
| 94 | 99.7 | N.T. |

TABLE I (cont'd)

H. Contortus

| Compound No. | % Clearance P.O. | % Clearance I.P. |
|---|---|---|
| 95 | 81.8 | N.T. |
| 96 | N.T. | N.T. |
| 97 | 98.6 | N.T. |
| 98 | 74.5 | N.T. |
| 99 | 0 | N.T. |
| 100 | 99.2 | N.T. |
| 101 | 0 | N.T. |
| 102 | N.T. | N.T. |
| 103 | N.T. | N.T. |
| 104 | 68.9 | N.T. |
| 105 | N.T. | N.T. |
| 106 | N.T. | N.T. |
| 107 | N.T. | N.T. |
| 108 | 99.5 | N.T. |
| 109 | 15.2 | N.T. |
| 110 | N.T. | N.T. |
| 111 | N.T. | N.T. |
| 112 | N.T. | N.T. |
| 113 | N.T. | N.T. |
| 114 | 99.4 | N.T. |
| 115 | 99.4 | N.T. |
| 116 | 96.4 | N.T. |
| 117 | N.T. | N.T. |
| 118 | 100 | N.T. |
| 119 | 100 | N.T. |
| 120 | 99.5 | N.T. |
| 121 | N.T. | N.T. |
| 122 | 76.8 | N.T. |
| 123 | N.T. | N.T. |
| 124 | 100 | N.T. |
| 125 | N.T. | N.T. |
| 126 | 99.5 | N.T. |

TABLE I (cont'd)

H. Contortus

| Compound No. | % Clearance P.O. | I.P. |
|---|---|---|
| 127 | N.T. | N.T. |
| 128 | N.T. | N.T. |
| 129 | * | N.T. |
| 130 | 98.9 | N.T. |
| 131 | 85.4 | N.T. |
| 132 | N.T. | N.T. |
| 133 | 100 | N.T. |
| 134 | N.T. | N.T. |
| 135 | N.T. | N.T. |
| 136 | N.T. | N.T. |
| 137 | 100 | N.T. |
| 138 | 100 | N.T. |
| 139 | N.T. | N.T. |
| 140 | N.T. | N.T. |
| 141 | N.T. | N.T. |
| 142 | N.T. | N.T. |
| 143 | N.T. | N.T. |
| 144 | 99.6 | N.T. |
| 145 | N.T. | N.T. |
| 146 | 94.9 | N.T. |
| 147 | N.T. | N.T. |
| 148 | 100 | N.T. |
| 149 | 0 | N.T. |
| 150 | 84.7 | N.T. |
| 151 | N.T. | N.T. |
| 152 | N.T. | N.T. |
| 153 | * | N.T. |
| 154 | 96.4 | N.T. |
| 155 | 0 | N.T. |
| 156 | * | N.T. |
| 157 | N.T. | N.T. |
| 158 | 0 | N.T. |

TABLE I (cont'd)

H. Contortus

| Compound No. | % Clearance P.O. | I.P. |
|---|---|---|
| 159 | N.T. | N.T. |
| 160 | * | N.T. |
| 161 | 9.6 | N.T. |
| 162 | 0 | N.T. |
| 163 | 0 | N.T. |
| 164 | 0 | N.T. |
| 165 | N.T. | N.T. |
| 166 | * | N.T. |
| 167 | * | N.T. |
| 168 | 63.5 | N.T. |
| 169 | * | N.T. |
| 170 | N.T. | N.T. |
| 171 | 67.2 | N.T. |
| 172 | N.T. | N.T. |
| 173 | * | N.T. |
| 174 | N.T. | N.T. |
| 175 | N.T. | N.T. |
| 176 | 0 | N.T. |
| 177 | N.T. | N.T. |
| 178 | 99.4 | N.T. |
| 179 | N.T. | N.T. |
| 180 | N.T. | N.T. |
| 181 | 75.1 | N.T. |
| 182 | N.T. | N.T. |
| 183 | N.T. | N.T. |
| 184 | N.T. | N.T. |
| 185 | N.T. | N.T. |
| 186 | 99.9 | N.T. |
| 187 | N.T. | N.T. |
| 188 | N.T. | N.T. |
| 189 | N.T. | N.T. |
| 190 | N.T. | N.T. |

TABLE I (cont'd)

H. Contortus

| Compound No. | % Clearance P.O. | I.P. |
|---|---|---|
| 191 | N.T. | N.T. |
| 192 | 99.5 | N.T. |
| 193 | N.T. | N.T. |
| 194 | N.T. | N.T. |
| 195 | N.T. | N.T. |
| 196 | N.T. | N.T. |
| 197 | | |
| 198 | | |
| 199 | | |
| 200 | * | N.T. |
| 201 | 99.4 | N.T. |
| 202 | 83.2 | N.T. |
| 203 | 99.7 | N.T. |
| 204 | * | N.T. |
| 205 | N.T. | N.T. |
| 206 | N.T. | N.T. |
| 207 | N.T. | N.T. |
| 208 | 98.9 | N.T. |
| 209 | 93.2 | N.T. |
| 210 | * | N.T. |
| 211 | 89.1 | N.T. |
| 212 | N.T. | N.T. |
| 213 | 0 | N.T. |
| 214 | 82.5 | N.T. |
| 215 | N.T. | N.T. |
| 216 | N.T. | N.T. |
| 217 | N.T. | N.T. |
| 218 | N.T. | N.T. |
| 219 | N.T. | N.T. |
| 220 | N.T. | N.T. |
| 221 | N.T. | N.T. |
| 222 | N.T. | N.T. |

TABLE I (cont'd)

H. Contortus

| Compound No. | % Clearance P.O. | I.P. |
|---|---|---|
| 223 | 0 | N.T. |
| 224 | 96.4 | N.T. |
| 225 | N.T. | N.T. |
| 226 | N.T. | N.T. |
| 227 | 0 | N.T. |
| 228 | N.T. | N.T. |
| 229 | N.T. | N.T. |
| 230 | N.T. | N.T. |
| 231 | * | N.T. |
| 232 | N.T. | N.T. |
| 233 | 41.2 | N.T. |
| 234 | N.T. | N.T. |
| 235 | N.T. | N.T. |
| 236 | N.T. | N.T. |
| 237 | N.T. | N.T. |
| 238 | * | N.T. |
| 239 | N.T. | N.T. |
| 240 | 100 | N.T. |
| 241 | 100 | N.T. |
| 242 | N.T. | N.T. |
| 243 | N.T. | N.T. |
| 244 | N.T. | N.T. |
| 245 | N.T. | N.T. |
| 246 | N.T. | N.T. |
| 247 | * | N.T. |
| 248 | Toxic | N.T. |
| 249 | 74.5 | N.T. |
| 250 | N.T. | N.T. |
| 251 | Toxic | N.T. |
| 252 | N.T. | N.T. |
| 253 | N.T. | N.T. |
| 254 | N.T. | N.T. |

TABLE I (cont'd)

H. Contortus

| Compound No. | % Clearance P.O. | I.P. |
|---|---|---|
| 255 | N.T. | N.T. |
| 256 | N.T. | N.T. |
| 257 | N.T. | N.T. |
| 258 | N.T. | N.T. |
| 259 | N.T. | N.T. |
| 260 | N.T. | N.T. |
| 261 | | |
| 262 | | |
| 263 | N.T. | N.T. |
| 264 | 95.9 | 99.9-100 |
| 265 | | |
| 266 | 99.8 | N.T. |
| 267 | Toxic | 99.9 |
| 268 | Toxic | 100 |
| 269 | N.T. | N.T. |
| 270 | 99.7 | N.T. |
| 271 | 99.6 | 96.2 |
| 272 | 99.9 | 98.7 |
| 273 | 78.1 | N.T. |
| 274 | 99.9 | N.T. |
| 275 | 30.3 | N.T. |
| 276 | 96.0 | 77.4 |
| 277 | Toxic | N.T. |
| 278 | * | N.T. |
| 279 | 98.9 | N.T. |
| 280 | 71.0 | 4.1 |
| 281 | N.T. | N.T. |
| 282 | N.T. | N.T. |
| 283 | 100 | N.T. |
| 284 | Toxic | Toxic |
| 285 | 99.4 | N.T. |

TABLE I (cont'd)

H. Contortus

| Compound No. | % Clearance P.O. | I.P. |
|---|---|---|
| 286 | * | N.T. |
| 287 | 78.1 | N.T. |
| 288 | 99.8 | N.T. |
| 289 | 99.9 | 99.2 |
| 290 | 99.7 | N.T. |
| 291 | 100 | N.T. |
| 292 | 94.6 | N.T. |
| 293 | 100 | N.T. |
| 294 | 99.6 | N.T. |
| 295 | N.T. | N.T. |
| 296 | 100 | N.T. |
| 297 | N.T. | N.T. |
| 298 | N.T. | N.T. |
| 299 | Toxic | N.T. |
| 300 | 97.7 | N.T. |
| 301 | 96.4 | N.T. |
| 302 | N.T. | N.T. |
| 303 | 96.4[c] | N.T. |
| 304 | N.T. | N.T. |
| 305 | 92.8 | N.T. |
| 306 | N.T. | N.T. |
| 307 | 79.9 | N.T. |
| 308 | N.T. | N.T. |
| 309 | 76.7 | N.T. |
| 310 | N.T. | N.T. |
| 311 | 94.6 | N.T. |
| 312 | 100 | N.T. |
| 313 | 99.5 | N.T. |
| 314 | N.T. | N.T. |
| 315 | 91.0 | N.T. |
| 316 | N.T. | N.T. |
| 317 | 94.6 | N.T. |

TABLE I (cont'd)

H. Contortus

| | % Clearance | |
|---|---|---|
| Compound No. | P.O. | I.P. |
| 318 | 100 | N.T. |
| 319 | 64.2 | N.T. |
| 320 | 96.7 | N.T. |
| 321 | 44.4 | N.T. |
| 322 | N.T. | N.T. |
| 323 | 100 | N.T. |
| 324 | 98.3 | N.T. |
| 325 | 99.1 | N.T. |
| 326 | N.T. | N.T. |
| 327 | 94.6 | N.T. |
| 328 | 100 | N.T. |
| 329 | 100 | N.T. |
| 330 | N.T. | N.T. |
| 331 | 98.9 | N.T. |
| 332 | N.T. | N.T. |
| 333 | N.T. | N.T. |
| 334 | 30.0 | N.T. |
| 335 | N.T. | N.T. |
| 336 | N.T. | N.T. |
| 337 | N.T. | N.T. |
| 338 | N.T. | N.T. |
| 339 | N.T. | N.T. |
| 340 | N.T. | N.T. |
| 341 | N.T. | N.T. |
| 342 | N.T. | N.T. |
| 343 | 100 | N.T. |
| 344 | 0 | N.T. |
| 345 | 100 | N.T. |
| 346 | 99.8 | N.T. |
| 347 | 31.9 | N.T. |
| 348 | N.T. | N.T. |
| 349 | 0 | N.T. |

TABLE I (cont'd)

H. Contortus

| Compound No. | % Clearance P.O. | I.P. |
|---|---|---|
| 350 | N.T. | N.T. |
| 351 | N.T. | N.T. |
| 352 | N.T. | N.T. |
| 353 | N.T. | N.T. |
| 354 | N.T. | N.T. |
| 355 | N.T. | N.T. |
| 356 | N.T. | N.T. |
| 357 | N.T. | N.T. |
| 358 | N.T. | N.T. |
| 359 | N.T. | N.T. |
| 360 | N.T. | N.T. |
| 361 | 100 | N.T. |
| 362 | N.T. | N.T. |
| 363 | 99.9 | N.T. |
| 364 | N.T. | N.T. |
| 365 | 100 | N.T. |
| 366 | 100 | N.T. |
| 367 | 99.6 | N.T. |
| 368 | N.T. | N.T. |
| 369 | N.T. | N.T. |
| 370 | 99.4 | N.T. |
| 371 | N.T. | N.T. |
| 372 | N.T. | N.T. |
| 373 | N.T. | N.T. |
| 374 | N.T. | N.T. |
| 375 | N.T. | N.T. |
| 376 | N.T. | N.T. |
| 377 | N.T. | N.T. |
| 378 | N.T. | N.T. |
| 379 | 20.9 | N.T. |
| 380 | N.T. | N.T. |
| 381 | N.T. | N.T. |

TABLE I (cont'd)

H. Contortus

% Clearance

| Compound No. | P.O. | I.P. |
|---|---|---|
| 382 | N.T. | N.T. |
| 383 | N.T. | N.T. |
| 384 | 100 | N.T. |
| 385 | N.T. | N.T. |
| 386 | 99.9 | N.T. |
| 387 | N.T. | N.T. |
| 388 | N.T. | N.T. |
| 389 | N.T. | N.T. |
| 390 | 100 | N.T. |
| 391 | 99.9 | N.T. |
| 392 | 95.8 | N.T. |
| 393 | N.T. | N.T. |
| 394 | 99.5 | N.T. |
| 395 | 100 | N.T. |
| 396 | N.T. | N.T. |
| 397 | 85.7 | N.T. |
| 398 | Toxic | N.T. |
| 399 | 53.4 | N.T. |
| 400 | 100 | N.T. |
| 401 | N.T. | N.T. |
| 402 | 100 | N.T. |
| 403 | N.T. | N.T. |
| 404 | 95.0 | N.T. |
| 405 | 62.4 | N.T. |
| 406 | 100 | N.T. |
| 407 | 71.8 | N.T. |
| 408 | 71.3 | N.T. |
| 409 | N.T. | N.T. |
| 410 | 80.3 | N.T. |
| 411 | 85.0 | N.T. |
| 412 | 22.9 | N.T. |
| 413 | 83.9 | N.T. |

TABLE I (cont'd)

H. Contortus

| | % Clearance | |
|---|---|---|
| Compound No. | P.O. | I.P. |
| 414 | N.T. | N.T. |
| 415 | 0 | N.T. |
| 416 | N.T. | N.T. |
| 417 | N.T. | N.T. |
| 418 | 74.9 | N.T. |
| 419 | N.T. | N.T. |
| 420 | 49.8 | N.T. |
| 421 | N.T. | N.T. |
| 422 | 89.2 | N.T. |
| 423 | 97.8 | N.T. |
| 424 | 96.4 | N.T. |
| 425 | N.T. | N.T. |
| 426 | 98.2 | N.T. |
| 427 | N.T. | N.T. |
| 428 | N.T. | N.T. |
| 429 | 100 | N.T. |
| 430 | 100 | N.T. |
| 431 | Toxic | N.T. |
| 432 | 100 | N.T. |
| 433 | Toxic | N.T. |
| 434 | 91.6 | N.T. |
| 435 | 91.0 | N.T. |
| 436 | 92.0 | N.T. |
| 437 | 99.9 | N.T. |
| 438 | 93.3 | N.T. |
| 439 | 99.8 | N.T. |
| 440 | 98.9 | N.T. |
| 441 | 83.9 | N.T. |
| 442 | N.T. | N.T. |
| 443 | 97.9 | N.T. |
| 444 | 60.0 | N.T. |
| 445 | 89.6 | N.T. |

TABLE I (cont'd)

H. Contortus

% Clearance

| Compound No. | P.O. | I.P. |
|---|---|---|
| 446 | 98.7 | N.T. |
| 447 | N.T. | N.T. |
| 448 | N.T. | N.T. |
| 449 | N.T. | N.T. |
| 450 | 99.6 | N.T. |
| 451 | N.T. | N.T. |
| 452 | 0 | N.T. |
| 453 | 0 | N.T. |
| 454 | N.T. | N.T. |
| 455 | N.T. | N.T. |
| 456 | N.T. | N.T. |
| 457 | 100 | N.T. |
| 458 | N.T. | N.T. |
| 459 | 40.4 | N.T. |
| 460 | 0 | N.T. |
| 461 | N.T. | N.T. |
| 462 | N.T. | N.T. |
| 463 | N.T. | N.T. |
| 464 | N.T. | N.T. |
| 465 | N.T. | N.T. |
| 466 | N.T. | N.T. |
| 467 | N.T. | N.T. |
| 468 | N.T. | N.T. |
| 469 | N.T. | N.T. |
| 470 | N.T. | N.T. |
| 471 | N.T. | N.T. |
| 472 | N.T. | N.T. |
| 473 | N.T. | N.T. |
| 474 | N.T. | N.T. |
| 475 | N.T. | N.T. |
| 476 | 38.5 | N.T. |
| 477 | 0 | N.T. |

TABLE I (cont'd)

H. Contortus

| | % Clearance | |
|---|---|---|
| Compound No. | P.O. | I.P. |
| 478 | N.T. | N.T. |
| 479 | N.T. | N.T. |
| 480 | N.T. | N.T. |
| 481 | N.T. | N.T. |
| 482 | N.T. | N.T. |
| 483 | 99.2 | N.T. |
| 487 | 99.9 | N.T. |
| 488 | N.T. | N.T. |
| 489 | N.T. | N.T. |
| 490 | N.T. | N.T. |
| 491 | N.T. | N.T. |
| 492 | N.T. | N.T. |
| 493 | N.T. | N.T. |
| 494 | 100 | N.T. |
| 495 | N.T. | N.T. |
| 496 | N.T. | N.T. |
| 497 | N.T. | N.T. |
| 498 | N.T. | N.T. |
| 499 | N.T. | N.T. |
| 500 | N.T. | N.T. |
| 501 | N.T. | N.T. |
| 502 | N.T. | N.T. |
| 503 | N.T. | N.T. |
| 504 | 87.8[d] | N.T. |
| 505 | N.T. | N.T. |
| 506 | 100 | N.T. |
| 507 | N.T. | N.T. |
| 508 | N.T. | N.T. |
| 509 | N.T. | N.T. |
| 510 | N.T. | N.T. |
| 511 | N.T. | N.T. |
| 512 | N.T. | N.T. |
| 513 | N.T. | N.T. |
| 514 | N.T. | N.T. |

TABLE I (cont'd)

H. Contortus

| Compound No. | % Clearance P.O. | I.P. |
|---|---|---|
| 515 | N.T. | N.T. |
| 516 | N.T. | N.T. |
| 517 | N.T. | N.T. |
| 518 | N.T. | N.T. |
| 519 | N.T. | N.T. |
| 520 | N.T. | N.T. |
| 521 | N.T. | N.T. |
| 522 | N.T. | N.T. |
| 523 | N.T. | N.T. |
| 524 | N.T. | N.T. |
| 525 | N.T. | N.T. |
| 526 | N.T. | N.T. |
| 527 | N.T. | N.T. |
| 528 | N.T. | N.T. |
| 529 | N.T. | N.T. |
| 530 | N.T. | N.T. |
| 531 | N.T. | N.T. |
| 532 | N.T. | N.T. |
| 533 | N.T. | N.T. |
| 534 | N.T. | N.T. |
| 535 | N.T. | N.T. |
| 536 | N.T. | N.T. |
| 537 | N.T. | N.T. |
| 538 | N.T. | N.T. |
| 539 | N.T. | N.T. |
| 540 | N.T. | N.T. |
| 541 | N.T. | N.T. |
| 542 | N.T. | N.T. |
| 543 | N.T. | N.T. |
| 544 | N.T. | N.T. |
| 545 | N.T. | N.T. |
| 546 | N.T. | N.T. |
| 547 | N.T. | N.T. |
| 548 | N.T. | N.T. |

TABLE I (cont'd)

H. Contortus

| Compound No. | % Clearance P.O. | I.P. |
|---|---|---|
| 549 | N.T. | N.T. |
| 550 | N.T. | N.T. |
| 551 | N.T. | N.T. |
| 552 | N.T. | N.T. |
| 553 | N.T. | N.T. |
| 554 | N.T. | N.T. |
| 555 | N.T. | N.T. |
| 556 | N.T. | N.T. |
| 557 | N.T. | N.T. |
| 558 | N.T. | N.T. |
| 559 | N.T. | N.T. |
| 560 | N.T. | N.T. |
| 561 | N.T. | N.T. |
| 562 | N.T. | N.T. |
| 563 | N.T. | N.T. |
| 564 | N.T. | N.T. |
| 565 | N.T. | N.T. |
| 566 | N.T. | N.T. |
| 567 | N.T. | N.T. |
| 568 | 99.9 | N.T. |
| 569 | 97.9 | N.T. |
| 570 | N.T. | N.T. |
| 571 | N.T. | N.T. |
| 572 | N.T. | N.T. |
| 573 | N.T. | N.T. |
| 574 | N.T. | N.T. |
| 575 | 99.5 | N.T. |
| 576 | N.T. | N.T. |
| 577 | N.T. | N.T. |
| 578 | N.T. | N.T. |
| 579 | N.T. | N.T. |
| 580 | N.T. | N.T. |
| 581 | N.T. | N.T. |
| 582 | N.T. | N.T. |

TABLE I (cont'd)

H. Contortus

| Compound No. | % Clearance P.O. | I.P. |
|---|---|---|
| 583 | N.T. | N.T. |
| 584 | N.T. | N.T. |
| 585 | 99.7 | N.T. |
| 586 | N.T. | N.T. |
| 587 | N.T. | N.T. |
| 588 | N.T. | N.T. |
| 589 | N.T. | N.T. |
| 590 | 100 | N.T. |
| 591 | 99.2 | N.T. |
| 592 | N.T. | N.T. |
| 593 | N.T. | N.T. |
| 594 | N.T. | N.T. |
| 595 | 0 | N.T. |
| 596 | N.T. | N.T. |
| 597 | N.T. | N.T. |
| 598 | N.T. | N.T. |
| 599 | 56.2 | N.T. |
| 600 | 93.7 | 97.5 |
| 601 | N.T. | N.T. |
| 602 | N.T. | N.T. |
| 603 | N.T. | N.T. |
| 604 | 89.1 | N.T. |
| 605 | 99.2 | 97.5 |
| 606 | N.T. | N.T. |

N.T. - not tested

___ - ___, means the compound was tested two or more times and the extreme values reported (for example, 23.7 - 26.6)

I.P. - Intraperitoneal administration

P.O. - oral administration

[a] 45 mg/kg dose

[b] 85 mg/kg dose

[c] 80 mg/kg dose

[d] 31 mg/kg dose

* Tested, Data inconclusive (due to failure of untreated controls)

Toxic = Sheep died within 24 hr following treatment and were not examined for worms.

TABLE IIA

Percentage Clearance (Adult Worms) of Compound #35 at 100 mg/kg

| Parasite | Percentage Clearance | Route |
|---|---|---|
| Haemonchus | 93.6 | Oral |
| | 62.8 | IP |
| Ostertagia | 46.5 | Oral |
| | 38.2 | IP |
| Trichostrongylus axei | 27.2 | Oral |
| | 0 | IP |
| Trichostrongylus colubriformis | 33.5 | Oral |
| | 0 | IP |
| Nematodirus | 81.8 | Oral |
| | 55.2 | IP |
| Cooperia | 35.4 | Oral |
| | 21.6 | IP |
| Strongyloides | 78.6 | Oral |
| | 0 | IP |
| Trichuris | 0 | Oral |
| | 22.8 | IP |

*Oral Data for Compound 35 is based on 4 lambs.

TABLE IIB

Percentage Clearance (Adult Worms) of Compound #22 and 264 (100 mg/kg)

| Parasite | Percentage Clearance (Compound 22) | Percentage Clearance (Compound 264) | Route |
|---|---|---|---|
| Haemonchus | 100 | 100 | Oral |
| | 80.0 | 100 | IP |
| Ostertagia | 0 | 2.5 | Oral |
| | 0 | 70.5 | IP |
| Nematodirus | 68.1 | 76.3 | Oral |
| | 68.1 | 97.0 | IP |
| Cooperia | 30.9 | 45.5 | Oral |
| | 0 | 50.9 | IP |
| Moniezia | 45.0 | 0 | Oral |
| | 65.0 | 60.0 | IP |
| Trichuris | 66.7 | 100 | Oral |
| | 33.3 | 66.7 | IP |

TABLE IIC

Percent Clearance (Adult Worms) of Compound =6 at 100 mg/kg (IP)

| Parasite | Percentage Clearance |
|---|---|
| Haemonchus | 92.8 |
| Ostertagia | 53.5 |
| Trichostrongylus axei | 27.0 |
| Trichostrongylus colubriformis | 0 |
| Cooperia | 0 |
| Nematodirus | 72.2 |
| Strongyloides | 35.8 |
| Trichuris | 9.6 |
| Oesphagostomum | 0 |

TABLE IID

Percentage Clearance (Adult Worms) of Compound #19 at 100 mg/kg (Orally)

| Parasite | Percentage Clearance |
|---|---|
| Haemonchus contortus | 98.3 |
| Ostertagia circumcincta | 95.0 |
| T. axei | 88.0 |
| T. colubriformis | 0 |
| Cooperia curticei | 1.0 |
| N. folicollis | 0 |
| Strongyloides | 0 |

TABLE IIE

Percentage Clearance (Adult Worms) of Compound #17 at 100 mg/kg (IP)

| Parasite | Percentage Clearance |
|---|---|
| Haemonchus contortus | 84.3 |
| Ostertagia circumcincta | 69.3 |
| T. axei | 75.6 |
| T. colubriformis | 0 |
| Cooperia curticei | 14.7 |
| N. folicollis | 54.4 |
| Strongyloides | 9.6 |
| Trichuris ovis | 0 |

TABLE IIF

Percentage Clearance (Adult Worms) of Compound =22 at 100 mg/kg (Orally)

| Parasite | Percentage Clearance |
|---|---|
| Haemonchus | 95.8 |
| Ostertagia | 86.8 |
| T. axei | 0 |
| T. colubriformis | 93.5 |
| Cooperia | 0 |
| Nematodirus | 89.0 |
| Bunostomum | 0 |
| Strongyloides | 9.5 |
| Trichuris | 0 |
| Oesophagostomum | 64.4 |

TABLE IIG

Percentage Clearance (Adult Worms) of Compound =13 at 75 mg/kg (Orally)

| Parasite | Percentage Clearance |
|---|---|
| Haemonchus contortus | 91.7 |
| Ostertagia circumcincta | 0 |
| Trichostrongylus | 62.1 |
| Nematodirus | 65.4 |
| Oesophagostomum | 0 |
| Trichuris | 0 |
| Moniezia | 0 |

FORMULAE

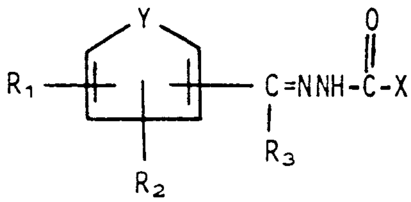

I

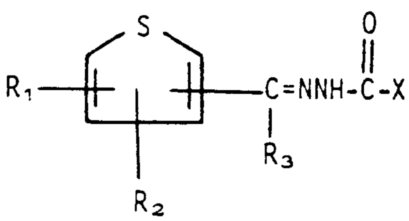

IA

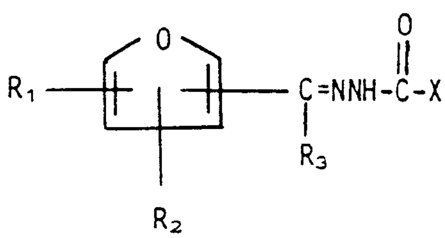

IB

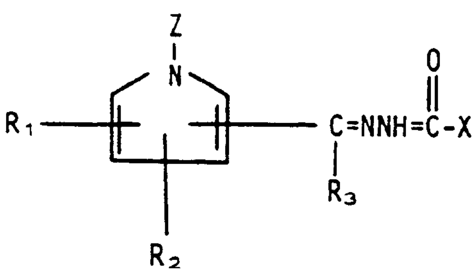

IC

CHART A

Scheme A:

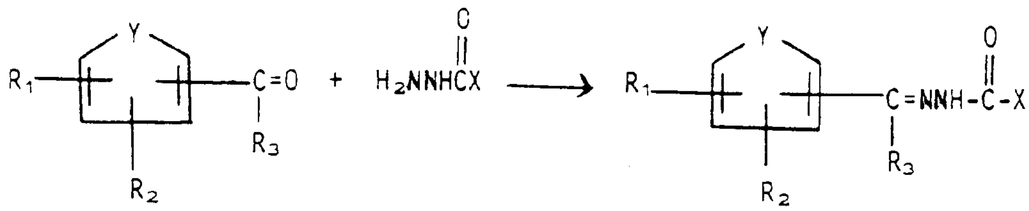

II III I

Scheme B:

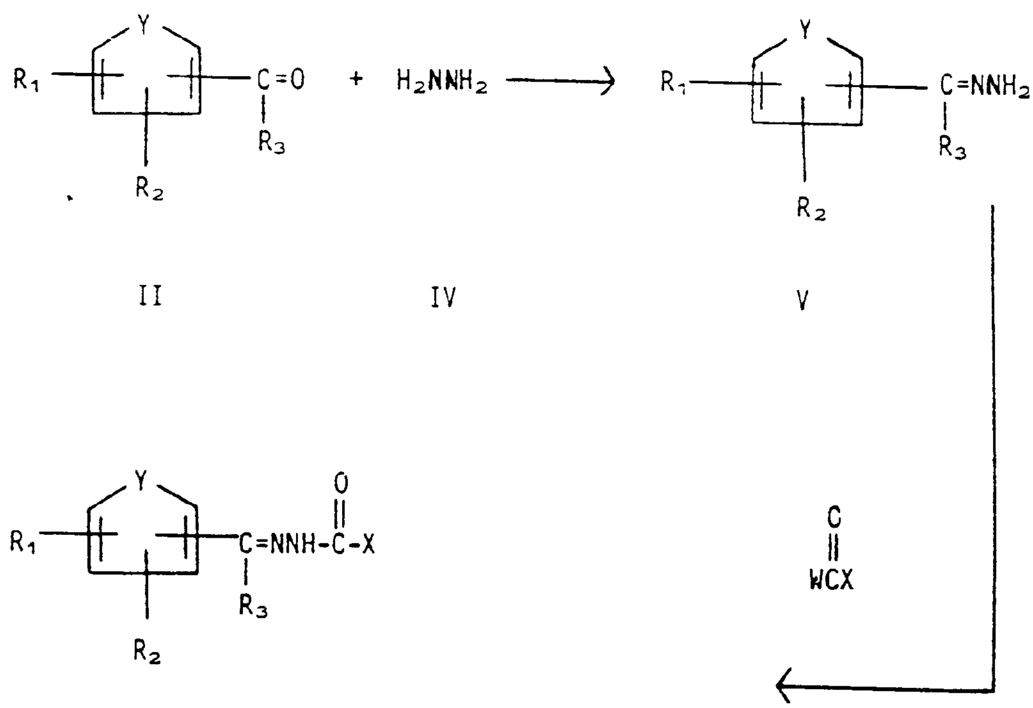

II IV V

I VI

W is a halogen atom or other active group, for example, an anhydride.

## Claims

1. Use of an acylhydrazone, or hydrate or pharmaceutically-acceptable salt thereof, for the manufacture of a medicament for use in killing parasitic worms in humans and valuable warm-blooded domestic animals, in which the acylhydrazone has the formula

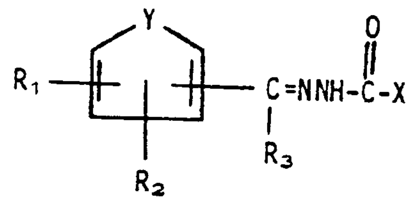

I

wherein X is

(a) hydrogen; with the proviso that when X is hydrogen and the compound is a 2-thienyl acylhydrazone, and $R_1$ is 5-methyl and $R_2$ is hydrogen, $R_3$ is other than methyl;
(b) $C_1$-$C_{10}$ alkyl; with the proviso that when X is ethyl and the compound is a 2-thienyl acylhydrazone, and $R_1$ is 5-Cl and $R_2$ is hydrogen, $R_3$ is other than methyl;
(c) $C_2$-$C_6$ alkenyl, preferably $C_2$-$C_4$ alkenyl;
(d) $C_2$-$C_6$ alkynyl;
(e) cyclo($C_3$-$C_{10}$)alkyl optionally substituted with one, 2 or 3 $C_1$-$C_4$ alkyl, $C_2$-$C_4$ alkenyl, $C_1$-$C_4$ alkoxy, trifluoromethyl, or halo; with the proviso that when X is cyclohexyl or chrysanthemyl and the compound is a 2-thienyl acylhydrazone, and $R_1$ is 5-Cl and $R_2$ is hydrogen, $R_3$ Is other than methyl;
(f) pyrrolidinyl;
(g) piperidinyl;
(h) 1-methylpyrrolidinyl;
(i) 1-methylpiperidinyl;
(j) $C_2$-$C_6$ alkoxyalkyl;
(k) cyclo($C_3$-$C_{10}$)alkyl($C_1$-$C_4$)alkyl;
(l) phenyl($C_1$-$C_4$)alkyl optionally substituted with one, 2 or 3 $C_1$-$C_4$ alkyl, $C_1$-$C_4$ alkoxy, halo, or trifluoromethyl;
(m) cyano($C_1$-$C_3$)alkyl;
(n) naphthyl($C_1$-$C_3$)alkyl optionally substituted with one or two $C_1$-$C_4$ alkyl, $C_1$-$C_4$ alkoxy, halo, or trifluoromethyl;
(o) $C_1$-$C_6$ alkoxy, with the proviso that when X is methoxy and the compound is a 2-thienyl acylhydrazone, and $R_1$ is 5-methyl and $R_2$ is hydrogen, $R_3$ is other than methyl; with the proviso that when X is ethoxy or t-butoxy and the compound is a 2-thienyl acylhydrazone, and $R_1$ is 5-Cl and $R_2$ is hydrogen, $R_3$ is other than methyl; with the proviso that when X is ethoxy and the compound is a 2-thienyl acylhydrazone, and $R_1$ is 4-methyl and $R_2$ is hydrogen, $R_3$ is other than methyl; with the proviso that when X is ethoxy and the compound is a 2-thienyl acylhydrazone, and $R_1$ and $R_2$ are hydrogen, $R_3$ is other than phenyl or 4-ethoxyphenyl;
(p) diphenylmethoxy;
(q) cyclo($C_3$-$C_6$)alkyloxy optionally substituted with one or two $C_1$-$C_3$ alkyl;
(r) phenoxy optionally substituted with one, 2 or 3 $C_1$-$C_4$ alkyl, $C_1$-$C_4$ alkoxy, halo, or trifluoromethyl; with the proviso that when X is phenoxy and the compound is a 2-thienyl acylhydrazone, and $R_1$ is 5-Cl and $R_2$ is hydrogen, $R_3$ is other than methyl;
(s) benzyloxy optionally substituted with one, 2 or 3 $C_1$-$C_4$ alkyl, $C_1$-$C_4$ alkoxy, halo, or trifluoromethyl; with the proviso that when X is benzyloxy and the compound is a 2-thienyl acylhydrazone, and $R_1$ is 4-methyl or 5-methyl and $R_2$ is hydrogen, $R_3$ is other than methyl;
(t) heteroaromatic optionally substituted with one, 2 or 3 $C_1$-$C_4$ alkyl, $C_1$-$C_3$ alkoxy, halo, $C_1$-$C_3$ alkylthio, or trifluoromethyl;
(u) phenyl optionally substituted with one, 2 or 3 $C_1$-$C_4$ alkyl, $C_1$-$C_3$ alkoxy, halo, trifluoromethyl, $C_2$-$C_6$ dialkylamino, $C_1$-$C_3$ alkylthio, nitro, or phenoxy optionally substituted with one, 2 or 3 $C_1$-$C_4$ alkyl, $C_1$-$C_3$ alkoxy, halo, or trifluoromethyl; with the proviso that when X is 4-chlorophenyl and the compound is a 2-thienyl acylhydrazone, and $R_1$ and $R_2$ are hydrogen, $R_3$ is other than hydrogen; with the proviso that when X is 3-chlorophenyl, 2-methylphenyl or 4-t-butylphenyl and the compound is a 2-thienyl acylhydrazone, and $R_1$

and $R_2$ are hydrogen, $R_3$ is other than methyl; with the further proviso that when X is 2-nitrophenyl or 2-ethoxyphenyl and the compound is a 2-furanyl acylhydrazone, and $R_1$ and $R_2$ are hydrogen, $R_3$ is other than methyl; with the further proviso that when X is phenyl and the compound is a 1-methyl-1H-pyrrol-2-yl acylhydrazone, $R_1$ and $R_2$ are hydrogen, $R_3$ is other than hydrogen; with the proviso that when X is 2-chlorophenyl or 4-chlorophenyl and the compound is a 2-thienyl acylhydrazone, and $R_2$ and $R_3$ are hydrogen, $R_1$ is other than 5-methyl; with the further proviso that when X is 2-nitrophenyl and the compound is a 2-thienyl acylhydrazone, and $R_1$ and $R_2$ are hydrogen, $R_3$ is other than ethyl; with the further proviso that when X is 2-methylphenyl and the compound is a 1H-pyrrol-2-yl acylhydrazone, $R_1$ and $R_2$ are hydrogen, $R_3$ is other than hydrogen;

(v) phenyl optionally substituted with the divalent $C_1$-$C_2$ alkylenedioxy;

(w) naphthyl optionally substituted with one or 2 $C_1$-$C_4$ alkyl, $C_1$-$C_3$ alkoxy, halo, trifluoromethyl, $C_2$-$C_6$ dialkylamino, $C_1$-$C_3$ alkylthio, nitro;

(x) bridged polycyclic hydrocarbon substituents of six to 10 nuclear carbons, optionally substituted with one, 2 or 3 ($C_1$-$C_3$) alkyl groups;

(y) perhalo($C_1$-$C_7$)alkyl;

(z) N-morpholinyl($C_1$-$C_4$)alkyl; (aa) N-piperidinyl($C_1$-$C_4$)alkyl; (bb) N-pyrrolidinyl($C_1$-$C_4$)alkyl;

wherein Y is an oxygen atom; a sulfur atom; or a N-Z group;

wherein Z is hydrogen; $C_1$-$C_4$ alkyl; phenyl optionally substituted with one, 2 or 3 $C_1$-$C_4$ alkyl, $C_1$-$C_3$ alkoxy, halo, trifluoromethyl, $C_2$-$C_6$ dialkylamino, $C_1$-$C_3$ alkylthio, nitro, or phenoxy optionally substituted with one, 2 or 3 $C_1$-$C_4$ alkyl, $C_1$-$C_3$ alkoxy, halo, or trifluoromethyl; or phenyl($C_1$-$C_4$)alkyl optionally substituted with one, 2 or 3 $C_1$-$C_4$ alkyl, $C_1$-$C_4$ alkoxy, halo, or trifluoromethyl;

wherein $R_1$ and $R_2$, being the same or different, are hydrogen; hydroxy; $C_1$-$C_4$ alkyl, preferably $C_1$-$C_3$ alkyl; $C_1$-$C_3$ alkoxy; $C_1$-$C_3$ alkylthio; halo or trifluoromethyl; with the proviso that $R_1$ and $R_2$ are not both halo; and

wherein $R_3$ is hydrogen; $C_1$-$C_6$ alkyl; cyclo($C_3$-$C_6$)alkyl optionally substituted with one, 2 or 3 $C_1$-$C_3$ alkyl, preferably cyclo(C3-$C_5$)alkyl optionally substituted; phenyl optionally substituted with one, 2 or 3 $C_1$-$C_4$ alkyl, halo, trifluoromethyl, or $C_1$-$C_3$ alkoxy; phenyl($C_1$-$C_3$)alkyl optionally substituted with one, 2 or 3 $C_1$-$C_4$ alkyl, halo, trifluoromethyl, or $C_1$-$C_3$ alkoxy; 1,3-dioxacyclohexan-5-yl; thienylvinyl; furanylvinyl; or phenylvinyl.

2. Use according to Claim 1 wherein the compound, hydrate or pharmaceutically acceptable salt thereof is a thienyl or furanyl acylhydrazone.

3. Use according to Claim 1 or claim 2 wherein X is hydrogen; $C_1$-$C_4$ alkyl; cyclohexylethyl; phenyl optionally substituted with one, 2 or 3 $C_1$-$C_4$ alkyl, $C_1$-$C_2$ alkoxy, trifluoromethyl and chloro; $C_1$-$C_4$ alkoxy; phenoxy optionally substituted with one, 2 or 3 $C_1$-$C_2$ alkyl, $C_1$-$C_2$ alkoxy, trifluoromethyl and chloro; cyclo($C_3$-$C_6$)alkyl; pyridinyl; thienyl; furanyl; benzyloxy optionally substituted with one or 2 $C_1$-$C_2$ alkoxy; di($C_1$-$C_2$)alkoxyphenylmethyl; N-morpholinylethyl; or 1-menthyl0.

4. Use according to any preceding Claim wherein $R_1$ and $R_2$ are selected from H, $CH_3$ and Br.

5. Use according to any preceding claim, wherein $R_3$ is H.

6. A compound, or hydrate or pharmaceutically-acceptable salt thereof, of formula I as given in claim 1, wherein $R_1$, $R_2$, $R_3$ and Y are as defined in claim 1, and X is cyclo($C_3$-$C_{10}$)alkyl optionally substituted by one, 2 or 3 substituents selected from $C_{1-4}$ alkyl, $C_2$-$C_4$ alkenyl, $C_1$-$C_4$ alkoxy, trifluoromethyl and halo; $C_2$-$C_6$ alkoxyalkyl; cyclo ($C_3$-$C_{10}$) alkyl ($C_1$-$C_4$) alkyl; cyclo ($C_3$-$C_6$) alkyloxy optionally substituted by one or two $C_1$-$C_3$ alkyl; benzyloxy optionally substituted by one, 2 or 3 substituents selected from $C_1$-$C_4$ alkyl, $C_1$-$C_4$ alkyl, $C_1$-$C_4$ alkoxy, halo and trifluoromethyl; or phenyl substituted by one, 2 or 3 substituents selected from $C_1$-$C_3$ alkoxy, phenoxy and trifluoromethyl;

with the proviso that $R_3$ is not methyl when

(i) X is cyclohexyl, the compound is a 2-thienyl acylhydrazone, $R_1$ is 5-Cl and $R_2$ is hydrogen,

(ii) or X is benzyloxy, the compound is a 2-thienyl acylhydrazone, $R_1$ is 4-methyl or 5-methyl and $R_2$ is hydrogen,

(iii) X is 2-ethoxyphenyl, the compound is a 2-furanyl acylhydrazone, and $R_1$ and $R_2$ are hydrogen.

7. A compound according to claim 6, wherein X is cyclohexylethyl, trifluoromethyl or phenyl optionally substituted by one, 2 or 3 methoxy or ethoxy groups.

8. A compound according to claim 6 or claim 7, wherein Y is NH.

9. A compound according to any of claims 6 to 8, wherein $R_1$ and $R_2$ are independently selected from H, $CH_3$ and Br.

10. A compound, hydrate or salt according to any of claims 6 to 9, wherein $R_3$ is H.

11. Use according to claim 1, wherein the compound is the (2-thienylmethylene)hydrazide of benzoic, nicotinic, acetic, formic, butyric, cyclohexanepropanoic or cyclohexanecarboxylic acid, or is methyl or ethyl 2-(thienyl-methylene)carbazate.

12. A compound according to claim 6, which is the (2-thienylmethylene)hydrazide of formic, butyric, cyclohexanepropanoic or cyclohexanecarboxylic acid, or methyl 2-(thienylmethylene)carbazate.

13. Use according to claim 1, wherein the compound is the [1-(2-thienyl)ethylidene]hydrazide of butyric, 3-methylbenzoic, 4-methoxybenzoic, 4-dimethylaminobenzoic, 3-ethoxypropanoic, 2-thiophenecarboxylic, nicotinic, formic, propanoic, cyclohexanepropanoic or cyclobutanecarboxylic acid, or is phenyl [1-(2-thienyl)ethylidene]-carbazate.

14. A compound according to claim 6, which is any compound defined in claim 13 except nicotinic acid [1-(2-thienyl)-ethylidene]hydrazide.

15. A compound according to claim 6, which is the [(3-methyl-2-thienyl)methylene]hydrazide of formic,acetic, propanoic, 2-methylpropanoic, butyric, cyclobutane-carboxylic, benzoic, 2-chlorobenzoic, 3-chlorobenzoic, 4-chlorobenzoic, 2-methylbenzoic, 3-methylbenzoic, 4-methylbenzoic, benzeneacetic, 2-thiophenecarboxylic or nicotinic acid, or is methyl, 1,1-dimethylethyl or 4-methoxyphenylmethyl [(3-methyl-2-thienyl)methylene]-carbazate.

16. A compound according to claim 6, which is the [(5-methyl-2-thienyl)methylene]hydrazide of formic,acetic, propanoic, 2-methylpropanoic, cyclobutanecarboxylic, 3-chlorobenzoic or nicotinic acid, or is ethyl, 1,1-dimethylethyl or 4-methoxyphenylmethyl [(5-methyl-2-thienyl)methylene]carbazate.

17. Use according to claim 1, wherein the compound is the [1-(3-thienyl)ethylidene]hydrazide of formic, acetic, propanoic, butanoic, benzoic or 4-chlorobenzoic acid, or is methyl or ethyl [1-(3-thienyl)ethylidene]carbazate.

18. A compound according to claim 6, which is any compound defined in claim 17 except acetic acid [1-(3-thienyl)-ethylidene]hydrazide.

19. A compound according to claim 6, which is the [1-(2-thienyl)propylidene/hydrazide of acetic, propanoic, butanoic, benzoic, 4-chlorobenzoic, 4-ethoxybenzoic or 4-methylbenzoic acid, or is methyl or ethyl [1-(2-thienyl)propylidene]carbazate.

20. A compound according to claim 6, which is acetic acid [1-(5-chloro-2-thienyl)ethylidene]hydrazide.

21. A compound according to claim 6, which is the [1-(3-methyl-2-thienyl)ethylidene]hydrazide of acetic,propanoic, 2-methylpropanoic or benzoic acid, or is methyl or ethyl [1-(3-methyl-2-thienyl)ethylidene]carbazate.

22. A compound according to claim 6, which is ethyl [1-(5-methyl-2-thienyl)ethylidene]carbazate.

23. A compound according to claim 6, which is the [1-(2,5-dimethyl-3-thienyl)ethylidene]hydrazide of propanoic, 2-methylpropanoic or benzoic acid.

24. Use according to claim 1, wherein the compound is the (3-thienylmethylene)hydrazide of formic, acetic, propanoic, 2-methylpropanoic, benzoic, cyclohexanecarboxylic, cyclobutanecarboxylic or 4-methoxybenzoic acid, or is phenyl (3-thienylmethylene)carbazate.

25. A compound according to claim 6, which is any compound defined in claim 24 except benzoic acid (3-thienylmethylene)hydrazide.

26. A compound according to claim 6, which is the [1-(4-methyl-2-thienyl-2-thienyl)ethylidene]hydrazide of benzoic or propanoic acid.

27. A compound according to claim 6, which is benzoic acid [1-(1H-pyrrol-2-yl)ethylidene]hydrazide or ethyl, methyl or phenylmethyl [1-(1H-pyrrol-2-yl)ethylidene]carbazate.

28. A compound according to claim 6, which is the [(2,5-dimethyl-1-phenyl-1H-pyrrol-3-yl)methylene]hydrazide of butyric or acetic acid, or ethyl [(2,5-dimethyl-1-phenyl-1H-pyrrol-3-yl)methylene]carbazate.

29. A compound according to claim 6, which is the [(1-methyl-1H-pyrrol-2-yl)methylene]hydrazide of benzeneacetic, isonicotinic, cyclohexanecarboxylic, cyclohexaneacetic, acetic, butyric, cyclohexanepropanoic, 4-chlorobenzoic, 3-chlorobenzoic, 2-chlorobenzoic, 4-ethoxybenzoic, nicotinic, 4-methylbenzoic, 4-(1,1-dimethylethyl)benzoic, 4-methoxybenzoic or cyclobutanecarboxylic acid, or is phenylmethyl or phenyl [(1-methyl-1H-pyrrol-2-yl)-methylene]carbazate.

30. Use according to claim 1, wherein the compound is (1H-pyrrol-2-ylmethylene)hydrazide of butyric, 4-chlorobenzoic, cyclobutanecarboxylic, 2-methylpropanoic, 3-cyclohexylpropanoic, butanoic, 2-chlorobenzoic,4-methylbenzoic, 3,4-dimethoxyphenylacetic, nicotinic, isonicotinic or formic acid, or is 1-menthyl, 4-methoxyphenylmethyl or phenylmethyl (1H-pyrrol-2-ylmethylene)carbazate.

31. A compound according to claim 6, which is any compound as defined in claim 30 except the (1H-pyrrol-2-ylmethylene)hydrazide of 4-chlorobenzoic, 2-chlorobenzoic, nicotinic or formic acid.

32. A compound according to claim 6, which is the [(5-methyl-2-furanyl)methylene]hydrazide of formic, acetic, propanoic, butyric, cyclohexanecarboxylic,4-chlorobenzoic, 3,4-dimethoxyphenylacetic, 4-methylbenzoic, 3-methylbenzoic, 4-ethoxybenzoic, 2-furoic, 2-thiophenecarboxylic or 2-phenoxybenzoic acid, or is methyl, ethyl, phenyl or benzyl [(5-methyl-2-furanyl)methylene]carbazate.

33. Use according to claim 1, wherein the compound is the [1-(2-furanyl)ethylidene]hydrazide of formic, acetic, propanoic, 2-methylpropanoic, cyclohexanecarboxylic, cyclopropanecarboxylic, 4-ethoxybenzoic, 2-furoic, 2-thiophenecarboxylic or 4-chlorobenzoic acid, or is ethyl or phenyl [1-(2-furanyl)ethylidene]carbazate.

34. A compound according to claim 6, which is any compound defined in claim 33 except the [1-(2-furanyl)ethylidene]-hydrazide of acetic or 2-furoic acid, or ethyl [1-(2-furanyl)ethylidene]carbazate.

35. Use according to claim 1, wherein the compound is the (2-furanylmethylene)hydrazide of 3-(N-morpholinyl)-propanoic, benzoic, 4-chlorobenzoic, 4-methylbenzoic or 4-methoxybenzoic acid, or is phenyl or phenylmethyl (2-furanylmethylene)carbazate.

36. A compound according to claim 6, which is any compound defined in claim 35 except the [1-(2-furanyl)methylene]-hydrazide of benzoic or 4-chlorobenzoic acid.

37. A compound according to claim 6, is the [1-(2,5-dimethyl-3-furanyl)ethylidene]hydrazide of butanoic, benzoic or propanoic acid.

38. A compound according to claim 6, which is the [(5-ethyl-2-furanyl)methylene]hydrazide of benzoic or butanoic acid.

39. A compound according to claim 6, which is butanoic acid [1-(2-furanyl)hexylidene]hydrazide.

40. A compound according to claim 6, which is ethyl [1-(5-methyl-2-furanyl)ethylidene]carbazate.

41. Use according to claim 1, wherein the compound is as defined in any of claims 15, 16, 19 to 23, 26 to 29, 32, 33 and 37 to 40.

## Patentansprüche

1. Verwendung eines Acylhydrazons, oder Hydrats oder pharmazeutisch annehmbaren Salzen davon, zur Herstellung eines Medikamentes zur Verwendung zum Töten von parasitischen Würmern in Menschen und wertvollen warmblütigen Haustieren, in welcher das Acylhydrazon die Formel

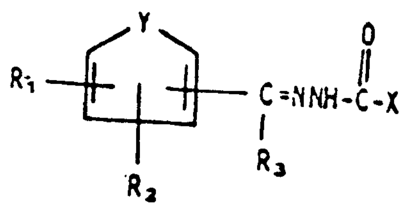

. I

hat, worin X

(a) Wasserstoff ist, unter der Bedingung, dass, wenn X Wasserstoff ist und die Verbindung ein 2-Thienylacylhydrazon ist, und $R_1$ 5-Methyl und $R_2$ Wasserstoff ist, $R_3$ anders als Methyl ist;

(b) $C_1$-$C_{10}$Alkyl; unter der Bedingung, dass, wenn X Äthyl ist und die Verbindung ein 2-Thienylacylhydrazon ist, und $R_1$ 5-Cl und $R_2$ Wasserstoff ist, $R_3$ anders als Methyl ist;

(c) $C_2$-$C_0$Alkenyl, vorzugsweise $C_2$-$C_4$Alkenyl;

(d) $C_2$-$C_0$Alkynyl;

(e) Cyclo($C_3$-$C_{10}$)alkyl wahlweise substituiert durch ein, 2 oder 3 $C_1$-$C_4$Alkyl, $C_2$-$C_4$Alkenyl, $C_1$-$C_4$Alkoxy, Trifluoromethyl, oder Halo; unter der Bedingung, dass, wenn X Cyclohexyl oder Chrysanthemyl ist und die Verbindung ein 2-Thienylacylhydrazon ist, und $R_1$ 5-Cl und $R_2$ Wasserstoff ist, $R_3$ anders als Methyl ist;

(f) Pyrrolidinyl;

(g) Piperidinyl;

(h) 1-Methylpyrrolidinyl;

(i) 1-Methylpiperidinyl;

(j) $C_2$-$C_8$Alkoxyalkyl;

(k) Cyclo($C_3$-$C_{10}$)alkyl($C_1$-$C_4$)alkyl;

(l) Phenyl($C_1$-$C_4$)alkyl wahlweise substituiert durch ein, 2 oder 3 $C_1$-$C_4$Alkyl, $C_1$-$C_4$Alkoxy, Halo, oder Trifluoromethyl;

(m) Cyano($C_1$-$C_3$)alkyl;

(n) Naphthyl($C_1$-$C_3$)alkyl, wahlweise substituiert durch ein oder zwei $C_1$-$C_4$Alkyl, $C_1$-$C_4$Alkoxy, Halo, oder Trifluoromethyl;

(o) $C_1$-$C_0$Alkoxy, unter der Bedingung, dass, wenn X Methoxy ist und die Verbindung ein 2-Thienylacylhydrazon ist, und $R_1$ 5-Methyl und $R_2$ Wasserstoff ist, $R_3$ anders als Methyl ist; unter der Bedingung, dass, wenn X Äthoxy oder t-Butoxy ist und die Verbindung ein 2-Thienylacylhydrazon ist, und $R_1$ 5-Cl und $R_2$

Wasserstoff ist, $R_3$ anders als Methyl ist; unter der Bedingung, dass, wenn X Äthoxy und die Verbindung ein 2-Thienylacylhydrazon ist, und $R_1$ 4-Methyl und $R_2$ Wasserstoff ist, $R_3$ anders als Methyl ist; unter der Bedingung, dass, wenn X Äthoxy und die Verbindung ein 2-Thienylacylhydrazon ist, und $R_1$ und $R_2$ Wasserstoff sind, $R_3$ anders als Phenyl oder 4-Äthoxyphenyl ist;

(p) Diphenylrethoxy;

(q) Cyclo($C_3$-$C_5$)alkoxy, wahlweise substituiert durch ein oder zwei $C_1$-$C_3$Alkyl;

(r) Phenoxy, wahlweise substituiert durch ein, 2 oder 3 $C_1$-$C_4$Alkyl, $C_1$-$C_4$Alkoxy, Halo oder Trifluoromethyl; unter der Bedingung, dass, wenn X Phenoxy ist und die Verbindung ein 2-Thienylacylhydrazon ist, und $R_1$ 5-Cl ist und $R_2$ Wasserstoff ist, $R_3$ anders als Methyl ist;

(s) Benzyloxy wahlweise substituiert durch ein, 2 oder 3 $C_1$-$C_4$Alkyl, $C_1$-$C_4$Alkoxy, Halo, oder Trifluoromethyl; unter der Bedingung, dass, wenn X Benzyloxy ist und die Verbindung ein 2-Thienylacylhydrazon, und $R_1$ 4-Methyl oder 5-Methyl und $R_2$ Wasserstoff ist, $R_3$ anders als Methyl ist;

(t) Heteroaroratisch wahlweise substituiert durch ein, 2 oder 3 $C_1$-$C_4$Alkyl, $C_1$-$C_3$Alkoxy, Halo, $C_1$-$C_3$Alkylthio, oder Trifluororethyl;

(u) Phenyl, wahlweise subsituiert durch ein, 2 oder 3 $C_1$-$C_4$Alkyl, $C_1$-$C_3$Alkoxy, Halo, Trifluoromethyl, $C_2$-$C_6$Dialkylamino, $C_1$-$C_3$Alkylthio, Nitro, oder Phenoxy wahlweise subsituiert durch ein, 2 oder 3 $C_1$-$C_4$Alkyl, $C_1$-$C_3$Alkoxy, Halo, oder Trifluoromethyl; unter der Bedingung, dass, wenn X 4-Chlorophenyl ist und die Verbindung ein 2-Thienylacylhydrazon ist, und $R_1$ und $R_2$ Wasserstoff sind, $R_3$ anders als Wasserstoff ist; unter der Bedingung, dass, wenn X 3-Chlorophenyl, 2-Methylphenyl oder 4-t-Butylphenyl ist und die Verbindung ein 2-Thienylacylhydrazon ist, und $R_1$ und $R_2$ Wasserstoff sind, $R_3$ anders als Methyl ist; unter der weiteren Bedingung, dass, wenn X 2-Nitrophenyl oder 2-Äthoxyphenyl ist und die Verbindung ein 2-Furanylacylhydrazon ist, und $R_1$ und $R_2$ Wasserstoff sind, $R_3$ anders als Methyl ist; unter der weiteren Bedingung, dass, wenn X Phenyl ist und die Verbindung ein 1-Methyl-1H-pyrrol-2-ylacylhydrazon ist, $R_1$ und $R_2$ Wasserstoff sind, $R_3$ anders als Wasserstoff ist; unter der Bedingung, dass, wenn X 2-Chlorphenyl oder 4-Chlorophenyl ist und die Verbindung ein 2-Thienylacylhydrazon ist, und $R_2$ und $R_3$ Wasserstoff sind, $R_1$ etwas anderes als 5-Methyl ist, unter der weiteren Bedingung, dass, wenn X 2-Nitrophenyl ist und die Verbindung ein 2-Thienylacylhydrazon ist, und $R_1$ und $R_2$ Wasserstoff sind, $R_3$ anders als Äthyl ist; unter der weiteren Bedingung, dass, wenn X 2-Methylphenyl ist und die Verbindung ein 1H-Pyrrol-2-ylacylhydrazon ist, $R_1$ und $R_2$ Wasserstoff sind, $R_3$ anders als Wasserstoff ist;

(v) Phenyl wahlweise substituiert durch zweiwertiges $C_1$-$C_2$Alkylendioxy;

(w) Naphthyl wahlweise substituiert durch ein oder 2 $C_1$-$C_4$Alkyl, $C_1$-$C_3$Alkoxy, Halo, Trifluoromethyl, $C_2$-$C_6$Dialkylamino, $C_1$-$C_3$Alkylthio, Nitro;

(x) überbrückte polyzyklische Kohlenwasserstoffsubstituenten von sechs bis 10 Kernkohlenstoffen, wahlweise substituiert durch ein, 2 oder 3 ($C_1$-$C_3$)Alkylgruppen;

(y) Perhalo($C_1$-$C_7$)alkyl;

(z) N-Morpholinyl($C_1$-$C_4$)alkyl; (aa) N-Piperidinyl($C_1$-$C_4$)alkyl; (bb) N-Pyrrolidinyl($C_1$-$C_4$)alkyl;

worin Y ein Sauerstoffatom ist; ein Schwefelatom; oder eine N-Z Gruppe;

worin Z Wasserstoff ist; $C_1$-$C_4$Alkyl, Phenyl wahlweise substituiert durch ein, 2 oder 3 $C_1$-$C_4$Alkyl, $C_1$-$C_3$ Alkoxy, Halo, oder Trifluoromethyl, $C_2$-$C_6$Dia1ky1amino, $C_1$-$C_3$Alkylthio, Nitro, oder Phenoxy wahlweise substituiert durch ein, 2 oder 3 $C_1$-$C_4$Alkyl, $C_1$-$C_3$Alkoxy, Halo oder Trifluoromethyl; oder Phenyl($C_1$-$C_4$)alkyl wahlweise substituiert durch ein, 2 oder 3 $C_1$-$C_4$Alkyl, $C_1$-$C_4$Alkoxy, Halo, oder Trifluoromethyl;

worin $R_1$ und $R_2$, entweder gleich oder verschieden, Wasserstoff sind; Hydroxy; $C_1$-$C_4$Alkyl, vorzugsweise $C_1$-$C_3$Alkyl; $C_1$-$C_3$Alkoxy; $C_1$-$C_3$Alkylthio; Halo oder Trifluoromethyl; unter der Bedingung, dass $R_1$ und $R_2$ nicht beide Halo sind; und

worin $R_3$ Wasserstoff ist; $C_1$-$C_3$Alkyl; Cyclo($C_3$-$C_6$)alkyl wahlweise substituiert durch ein, 2 oder 3 $C_1$-$C_4$ Alkyl, vorzugsweise Cyclo($C_3$-$C_5$)alkyl wahlweise substituiert; Phenyl wahlweise substituiert durch ein, 2 oder 3 $C_1$-$C_4$Alkyl, Halo, Trifluoromethyl, oder $C_1$-$C_3$Alkoxy; Phenyl($C_1$-$C_3$)alkyl wahlweise substituiert durch ein, 2 oder 3 $C_1$-$C_4$Alkyl, Halo, Trifluoromethyl, oder $C_1$-$C_3$Alkoxy; 1,3-Dioxacyclohexan-5-yl; Thienylvinyl; Furanylvinyl; oder Phenylvinyl.

2. Verwendung nach Anspruch 1, worin die Verbindung, das Hydrat, oder das pharmazeutisch annehmbare Salz davon ein Thienyl oder Furanylacylhydrazon ist.

3. Verwendung nach Anspruch 1 oder Anspruch 2, worin X Wasserstoff ist; $C_1$-$C_4$Alkyl; Cyclohexyläthyl; Phenyl wahlweise substituiert durch ein, 2 oder 3 $C_1$-$C_2$Alkyl, $C_1$-$C_2$Alkoxy, Trifluoromethyl und Chloro; $C_1$-$C_4$ Alkoxy; Phenoxy wahlweise substituiert durch ein, 2 oder 3 $C_1$-$C_2$Alkyl, $C_1$-$C_2$Alkoxy, Trifluoromethyl und Chloro; Cyclo($C_3$-$C_6$)alkyl; Pyridinyl; Thienyl; Furanyl; Benzyloxy wahlweise substituiert durch ein oder 2 $C_1$-$C_2$Alkoxy; Di($C_1$-$C_2$)alkoxyphenylmethyl; N-Morpholinylethyl; oder 1-Menthyl0.

4. Verwendung nach einem der vorhergehenden Ansprüche, worin $R_1$ und $R_2$ von H, $CH_3$ und Br ausgewählt werden.

5. Verwendung nach einem der vorhergehenden Ansprüche, worin $R_3$ H ist.

6. Verbindung, oder Hydrat oder pharmazeutisch annehmbares Salz davon, der wie in Anspruch 1 gegebenen Formel I, worin $R_1$, $R_2$, $R_3$ und Y wie in Anspruch 1 definiert sind, und X Cyclo($C_3$-$C_{10}$)alkyl ist, wahlweise substituiert durch einen, 2 oder 3 Substituenten, die von $C_1$-$C_4$Alkyl, $C_2$-$C_4$Alkenyl, $C_1$-$C_4$Alkoxy, Trifluoromethyl und Halo ausgewählt werden; $C_2$-$C_6$Alkoxyalkyl; Cyclo($C_3$-$C_{10}$)alkyl($C_1$-$C_4$)alkyl; Cyclo($C_3$-$C_6$)alkyloxy, wahlweise substituiert durch ein oder 2 $C_1$-$C_3$Alkyl; Benzyloxy wahlweise substituiert durch einen, 2 oder 3 Substituenten, die von $C_1$-$C_4$Alkyl, $C_1$-$C_4$Alkyl, $C_1$-$C_4$Alkoxy, Halo und Trifluoromethyl ausgewählt sind; oder Phenyl substituiert durch einen, 2 oder 3 Substituenten, die von $C_1$-$C_3$Alkoxy, Phenoxy und Trifluoromethyl ausgewählt sind;

unter der Bedingung, dass $R_3$ nicht Methyl ist, wenn

(i) X Cyclohexyl ist, die Verbindung ein 2-Thienylacylhydrazon ist, $R_1$ 5-Cl und $R_2$ Wasserstoff ist,

(ii) oder X Benzyloxy ist, die Verbindung ein 2-Thienylacylhydrazon, $R_1$ 4-Methyl oder 5-Methyl und $R_2$ Wasserstoff ist,

(iii) X 2-Äthoxyphenyl ist, die Verbindung ein 2-Furanylacylhydrazon ist, und $R_1$ und $R_2$ Wasserstof sind.

7. Verbindung nach Anspruch 6, worin X Cyclohexyläthyl, Trifluoromethyl oder Phenyl ist, wahlweise substituiert durch eine, 2 oder 3 Methoxy- oder Äthoxygruppe(n).

8. Verbindung nach Anspruch 6 oder Anspruch 7, worin Y NH ist.

9. Verbindung nach einem der Ansprüche 6 bis 8, worin $R_1$ und $R_2$ unabhängig von H, $CH_3$ und Br ausgewählt werden.

10. Verbindung, Hydrat oder Salz nach einem der Ansprüche 6 bis 9, worin $R_3$ H ist.

11. Verwendung nach Anspruch 1, worin die Verbindung das (2-Thienylmethylen)hydrazid von Benzoe-, Nikotin-, Essig-, Ameisen-, Butter-, Cyclohexanpropion- oder Cyclohexancarbonsäure ist, oder Methyl- oder Äthyl-2-(Thienylmethylen)carbazat ist.

12. Verbindung nach Anspruch 6, die das (2-Thienylmethylen)hydrazid von Ameisen-, Butter-, Cyclohexanpropion- oder Cyclohexancarbonsäure, oder Methyl-2-(Thienylmethylen)carbazat ist.

13. Verwendung nach Anspruch 1, worin die Verbindung das [1-(2-Thienyl)äthyliden]hydrazid von Butter-, 3-Methylbenzoe-, 4-Methoxybenzoe-, 4-Dimethylaminobenzoe-, 3-Äthoxypropion-, 2-Thiophencarbon-, Nikotin-, Ameisen-, Propion-, Cyclohexanpropion- oder Cyclobutancarbonsäure, oder Phenyl[1-(2-thienyl)äthyliden]-carbazat ist.

14. Verbindung nach Anspruch 6, die irgendeine in Anspruch 13 definierte Verbindung ausser Nikotinsäure[1-(2-thienyl)äthylidenhydrazid ist.

15. Verbindung nach Anspruch 6, die das [(3-Methyl-2-thienyl)methylen]hydrazid von Ameisen-, Essig-, Propion-, 2-Methylpropion-, Butter-, Cyclobutancarbon-, Benzoe-, 2-Chlorobenzoe-, 3-Chlorobenzoe-, 4-Chlorobenzoe-, 2-Methylbenzoe-, 3-Methylbenzoe-, 4-Methylbenzoe-, Benzolameisen-, 2-Thiophencarbon- oder Nikotinsäure, oder Methyl-, 1,1-Dimethyläthyl- oder 4-Methoxyphenylmethyl[(3-methyl-2-thienyl)methylen]-carbazat ist.

16. Verbindung nach Anspruch 6, die das [(5-Methyl-2-thienyl)methylen]hydrazid von Ameisen-, Essig-, Propion-, 2-Methylpropion-, Cyclobutancarbon-, 3-Chlorobenzoe- oder Nikotinsäure, oder Äthyl, 1,1-Dimethyläthyl oder 4-Methoxyphenylmethyl[(5-methyl-2-thienyl)methylen]carabazat ist.

17. Verwendung nach Anspruch 1, worin die Verbindung das [1-(3-Thienyl)äthyliden]hydrazid von Ameisen-, Essig-, Propion-, Butan-, Benzoe-, oder 4-Chlorobenzoesäure, oder Methyl- oder Äthyl[1-(3-thienyl)-äthyliden]hydrazid ist.

18. Verbindung nach Anspruch 6, die irgendeine in Anspruch 17 definierte Verbindung ausser Essigsäure[1-(3-thienyl)-äthylidenhydrazid ist.

19. Verbindung nach Anspruch 6, die das [1-(2-Thienyl)propyliden]hydrazid von Essig-, Propion-, Butan-, Benzoe-, 4-Chlorobenzoe-, 4-Äthoxybenzoe- oder 4-Methylbenzoesäure, oder Methyl- oder Äthyl[1-(2-thienyl)propyliden]carabazat ist.

20. Verbindung nach Anspruch 6, die Essigsäure[1-(5-chloro-2-thienyl)äthyliden]hydrazid ist.

21. Verbindung nach Anspruch 6, die das [1-(3-Methyl-2-thienyl)äthyliden]hydrazid von Essig-, Propion-, 2-Methylpropion- oder Benzoesäure, oder Methyl- oder Äthyl[1-(3-Methyl-2-thienyl)äthyliden]carbazat ist.

22. Verbindung nach Anspruch 6, die Äthyl[1-(5-methyl-2-thienyl)äthyliden]carbazat ist.

23. Verbindung nach Anspruch 6, die das [1-(2,5-Dimethyl-3-thienyl)äthyliden]hydrazid von Propion-, 2-Methylpropion- oder Benzoesäure ist.

24. Verwendung nach anspruch 1, worin die verbindung das (3-Thienylmethylen) hydrazid von Ameisen-, Essig-, Propion-, 2-Methylpropion-, Benzoe-, Cyclohexancarbon-, Cyclobutancarbon- oder 4-Methoxybenzoesäure, oder Phenyl(3-thienylmethylen)carbazat ist.

25. Verbindung nach anspruch 6, die irgendeine in Anspruch 24 definierte Verbindung ausser Benzoesäure(3-thienylmethylen)hydrazid ist.

26. Verbindung nach Anspruch 6, die das [1-(4-Methyl-2-thienyl-2-thienyl)äthyliden]hydrazid von Benzoe- oder Propionsäure ist.

27. Verbindung nach Anspruch 6, die Benzoesäure[1-(1H-pyrrol-2-yl)äthyliden]hydrazid oder Äthyl-, Methyl- oder Phenylmethyl[1-(1H-pyrrol-2-yl)äthyliden]carbazat ist.

28. Verbindung nach Anspruch 6, die das [(2,5-Dimethyl-1-phenyl-1H-pyrrol-3-yl)methylen]hydrazid von Butter- oder Essigsäure, oder Äthyl[(2,5-dimethyl-1-phenyl-1H-pyrrol-3-yl)methylencarbazat ist.

29. Verbindung nach Anspruch 6, die das [(1-Methyk-1H-pyrrol-2-yl)methylen]hydrazid von Benzolessig-, Isonikotin-, Cyclohexancarbon-, Cyclohexanessig-, Essig-, Butter-, Cyclohexanpropion-, 4-Chlorobenzoe-, 3-Chlorobenzoe-, 2-Chlorobenzoe-, 4-Äthoxybenzoe-, Nikotin-, 4-Methylbenzoe, 4-(1,1-Dimethyläthyl)benzoe-, 4-Methoxybenzoe- oder Cyclobutancarbonsäure, oder Phenylmethyl oder Phenyl[(1-methyl-1H-pyrrol-2-yl)-methylencarbazat ist.

30. Verwendung nach Anspruch 1, worin die verbindung (1H-Pyrrol-2-ylmethylen)hydrazid von Butter-, 4-Chlorobenzoe-, Cyclobutancarbon-, 2-Methylpropion-, 3-Cyclohexylpropion-, Butan-, 2-Chlorobenzoe-, 4-Methylbenzoe-, 3,4-Dimethoxyphenylessig-, nikotin-, Isonikotin- oder Ameisensäure, oder 1-Menthyl-, 4-Methoxyphenylmethyl- oder Phenylmethyl(1H-pyrrol-2-ylmethylen)carbazat ist.

31. Verbindung nach Anspruch 6, die irgendeine wie in Anspruch 30 defininierte Verbindung ausser (1H-Pyrrol-2-ylmethylen)hydrazid von 4-Chlorobenzoe-, 2-Chlorobenzoe-, nikotin- oder Ameisensäure ist.

32. Verbindung nach Anspruch 6, die das [(5-Methyl-2-furanyl)methylen]hydrazid von Ameisen-, Essig-, Propion-, Butter-, Cyclohexancarbon-, 4-Chlorobenzoe-, 3,4-Dimethoxyphenylessig-, 4-Methylbenzoe-, 3-Methylbenzoe-, 4-Äthoxybenzoe-, 2-Furol-, 2-Thiophencarbon- oder 2-Phenoxybenzoesäure, oder Methyl-, Äthyl-, Phenyl- oder Benzyl[(5-methyl-2-furanyl)methylen]carbazat ist.

33. Verwendung nach Anspruch 1, worin die Verbindung das [1-(2-Furanyl)äthyliden]hydrazid von Ameisen-, Essig-, Propion-, 2-Methylpropion-, Cyclohexancarbon-, Cyclopropancarbon-, 4-Äthoxybenzoe-, 2-Furol-, 2-Thiophencarbon- oder 4-Chlorobenzoesäure, oder Äthyl- oder Phenyl[1-(2-furanyl)äthyliden]carbazat ist.

34. Verbindung nach Anspruch 6, die irgendeine in Anspruch 33 definierte Verbindung ausser dem [1-(2-Furanyl)äthyliden]hydrazid von Essig- oder 2-Furolsäure, oder Äthyl[1-(2-furanyl)äthyliden]carbazat ist.

35. Verwendung nach Anspruch 1, worin die Verbindung das (2-Furanylmethylen)hydrazid von 3-(N-Morpholinyl-propion-, Benzoe-, 4-Chlorobenzoe-, 4-Methylbenzoe- oder 4-Methoxybenzoesäure, oder Phenyl- oder Phenylmethyl(2-furanylmethylen)carbazat ist.

36. Verbindung nach Anspruch 6, die irgendeine in Anspruch 35 definierte Verbindung ausser dem [1-(2-Furanyl)methylen]hydrazid von Benzoe- oder 4-Chlorobenzoesäure ist.

37. Verbindung nach Anspruch 6, die das [1-(2,5-Dimethyl-3-furanyl)methyliden]hydrazid von Butan-, Benzoe- oder Propionsäure ist.

38. Verbindung nach Anspruch 6, die das [(5-Äthyl-2-furanyl)methylenhydrazid von Benzoe- oder Butansäure ist.

39. Verbindung nach Anspruch 6, die Butansäure[1-(2-furanyl)hexyliden]hydrazid ist.

40. Verbindung nach Anspruch 6, die Äthyl[1-(5-methyl-2-furanyl)äthyliden]carbazat ist.

41. Verwendung nach Anspruch 1, worin die Verbindung wie in einem der Ansprüche 15, 16, 19 bis 23, 26 bis 29, 32, 33, und 37 bis 40 definiert ist.

## Revendications

1. Utilisation d'une acylhydrazone, ou un de ses hydrates ou sels pharmaceutiquement acceptables, pour la production d'un médicament destiné à être utilisé dans la destruction de vers parasites chez l'homme et des animaux domestiques à sang chaud intéressants, dans laquelle l'acylhydrazone répond à la formule

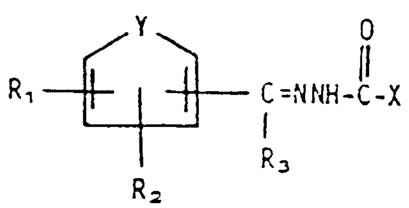

I

dans laquelle X représente

(a) l'hydrogène ; sous réserve que, lorsque X représente l'hydrogène et le composé consiste en une 2-thiénylacylhydrazone, et $R_1$ représente un groupe 5-méthyle et $R_2$ représente l'hydrogène, $R_3$ représente

un groupe différent du groupe méthyle ;

(b) un groupe alkyle en $C_1$ à $C_{10}$ ; sous réserve que, lorsque X représente un groupe éthyle et le composé consiste en une 2-thiénylacylhydrazone, et $R_1$ représente un groupe 5-Cl et $R_2$ représente l'hydrogène, $R_3$ représente un groupe différent du groupe méthyle ;

(c) un groupe alcényle en $C_2$ à $C_6$, de préférence alcényle en $C_2$ à $C_4$ ;

(d) un groupe alcynyle en $C_2$ à $C_6$ ;

(e) un groupe cyclo(alkyle en $C_3$ à $C_{10}$) substitué facultativement avec un, deux ou trois groupes alkyle en $C_1$ à $C_4$, alcényle en $C_2$ à $C_4$, alkoxy en $C_1$ à $C_4$, trifluorométhyle ou halogéno ; sous réserve que, lorsque X représente un groupe cyclohexyle ou chrysanthémyle et le composé consiste en une 2-thiénylacylhydrazone, et $R_1$ représente un groupe 5-Cl et $R_2$ représente l'hydrogène, $R_3$ représente un groupe autre que le groupe méthyle ;

(f) un groupe pyrrolidinyle ;

(g) un groupe pipéridinyle ;

(h) un groupe 1-méthylpyrrolidinyle ;

(i) un groupe 1-méthylpipéridinyle ;

(j) un groupe alkoxyalkyle en $C_2$ à $C_6$ ;

(k) un groupe cyclo(alkyle en $C_3$ à $C_{10}$)-(alkyle en $C_1$ à $C_4$) ;

(l) un groupe phényl(alkyle en $C_1$ à $C_4$) substitué facultativement avec un, deux ou trois groupes alkyle en $C_1$ à $C_4$, alkoxy en $C_1$ à $C_4$, halogéno ou trifluorométhyle ;

(m) un groupe cyano(alkyle en $C_1$ à $C_3$) ;

(n) un groupe naphtyl-(alkyle en $C_1$ à $C_3$) facultativement substitué avec un ou deux groupes alkyle en $C_1$ à $C_4$, alkoxy en $C_1$ à $C_4$, halogéno ou trifluorométhyle ;

(o) un groupe alkoxy en $C_1$ à $C_6$, sous réserve que, lorsque X représente un groupe méthoxy et le composé consiste en une 2-thiénylacylhydrazone, et $R_1$ représente un groupe 5-méthyle et $R_2$ représente l'hydrogène, $R_3$ représente un groupe autre que le groupe méthyle ; sous réserve que, lorsque X représente un groupe éthoxy ou tertio-butoxy et le composé consiste en une 2-thiénylacylhydrazone, et $R_1$ représente un groupe 5-Cl et $R_2$ représente l'hydrogène, $R_3$ représente un groupe différent du groupe méthyle ; sous réserve que, lorsque X représente un groupe éthoxy et le composé consiste en une 2-thiénylacylhydrazone, et $R_1$ représente un groupe 4-méthyle et $R_2$ représente l'hydrogène, $R_3$ représente un groupe différent du groupe méthyle ; sous réserve que, lorsque X représente un groupe éthoxy et le composé consiste en une 2-thiénylacylhydrazone, et $R_1$ et $R_2$ représentent l'hydrogène, $R_3$ représente un groupe différent du groupe phényle ou 4-éthoxyphényle ;

(p) un groupe diphénylméthoxy ;

(q) un groupe cyclo(alkyle en $C_3$ à $C_6$)oxy facultativement substitué avec un ou deux groupes alkyle en $C_1$ à $C_3$ ;

(r) un groupe phénoxy facultativement substitué avec un, deux ou trois groupes alkyle en $C_1$ à $C_4$, alkoxy en $C_1$ à $C_4$, halogéno ou trifluorométhyle ; sous réserve que, lorsque X représente un groupe phénoxy et le composé consiste en une 2-thiénylacylhydrazone, et $R_1$ représente un groupe 5-Cl et $R_2$ représente l'hydrogène, $R_3$ représente un groupe différent du groupe méthyle ;

(s) un groupe benzyloxy facultativement substitué avec un, deux ou trois groupes alkyle en $C_1$ à $C_4$, alkoxy en $C_1$ à $C_4$, halogéno ou trifluorométhyle ; sous réserve que, lorsque X représente un groupe benzyloxy et le composé consiste en une 2-thiénylacylhydrazone, et $R_1$ représente un groupe 4-méthyle ou 5-méthyle et $R_2$ représente l'hydrogène, $R_3$ représente un groupe différent du groupe méthyle ;

(t) un groupe hétéroaromatique facultativement substitué avec un, deux ou trois groupes alkyle en $C_1$ à $C_4$, alkoxy en $C_1$ à $C_3$, halogéno, alkylthio en $C_1$ à $C_3$ ou trifluorométhyle ;

(u) un groupe phényle facultativement substitué avec un, deux ou trois groupes alkyle en $C_1$ à $C_4$, alkoxy en $C_1$ à $C_3$, halogéno, trifluorométhyle, dialkylamino en $C_2$ à $C_6$, alkylthio en $C_1$ à $C_3$, nitro ou phénoxy facultativement substitué avec un, deux ou trois groupes alkyle en $C_1$ à $C_4$, alkoxy en $C_1$ à $C_3$, halogéno ou trifluorométhyle ; sous réserve que, lorsque X représente un groupe 4-chlorophényle et le composé consiste en une 2-thiénylacylhydrazone, et $R_1$ et $R_2$ représentent l'hydrogène, $R_3$ représente un groupe diffèrent de l'hydrogène ; sous réserve que, lorsque X représente un groupe 3-chlorophényle, 2-méthylphényle ou 4-tertio-butylphényle et le composé consiste en une 2-thiénylacylhydrazone, et $R_1$ et $R_2$ représentent l'hydrogène, $R_3$ représente un groupe différent du groupe méthyle ; sous réserve en outre que, lorsque X représente un groupe 2-nitrophényle ou 2-éthoxyphényle et le composé consiste en une 2-furannylacylhydrazone, et $R_1$ et $R_2$ représentent l'hydrogène, $R_3$ représente un groupe différent du groupe méthyle ; sous réserve en outre que, lorsque X représente un groupe phényle et le composé consiste en une 1-méthyl-1H-pyrrole-2-ylacylhydrazone, et $R_1$ et $R_2$ représentent l'hydrogène, $R_3$ représente un groupe autre que l'hydrogène ; sous réserve que, lorsque X représente un groupe 2-chlorophényle ou 4-chlorophényle et le composé consiste en une 2-thiénylacylhydrazone, et $R_2$ et $R_3$ représentent l'hydrogène, $R_1$ représente un groupe différent du groupe 5-méthyle ; sous réserve en outre que, lorsque X représente un groupe 2-nitrophényle et le composé consiste en une 2-thiénylacylhydrazone, et $R_1$ et $R_2$ représentent l'hydrogène, $R_3$ représente un groupe différent du groupe éthyle ; sous réserve en outre que, lorsque X représente un groupe 2-méthylphényle et le composé consiste en une 1H-pyrrole-2-ylacylhydrazone, et $R_1$ et $R_2$ représentent l'hydrogène, $R_3$ représente un groupe différent de l'hydrogène ;

(v) un groupe phényle facultativement substitué avec un groupe (alkylène en $C_1$ ou $C_2$)dioxy divalent ;

(w) un groupe naphtyle facultativement substitué avec un ou deux groupes alkyle en $C_1$ à $C_4$, alkoxy en $C_1$ à $C_3$, halogéno, trifluorométhyle, dialkylamino en $C_2$ à $C_6$, alkylthio en $C_1$ à $C_3$, nitro ;

(x) des substituants hydrocarbonés polycycliques pontés ayant 6 à 10 atomes de carbone dans le noyau, facultativement substitués avec un, deux ou trois groupes alkyle en $C_1$ à $C_3$ ;

(y) un groupe perhalogéno(alkyle en $C_1$ à $C_7$) ;

(z) un groupe N-morpholinyl(alkyle en $C_1$ à $C_4$) ; (aa) un groupe N-pipéridinyl(alkyle en $C_1$ à $C_4$) ; (bb) un groupe N-pyrrolidinyl(alkyle en $C_1$ à $C_4$) ;

Y représente un atome d'oxygène, un atome de soufre ou un groupe N-Z ;

dans lequel Z représente l'hydrogène ; un groupe alkyle en $C_1$ à $C_4$ ; phényle facultativement substitué avec un, deux ou trois groupes alkyle en $C_1$ à $C_4$, alkoxy en $C_1$ à $C_3$, halogéno, trifluorométhyle, dialkylamino en $C_2$ à $C_6$, alkylthio en $C_1$ à $C_3$, nitro ou phénoxy facultativement substitué avec un, deux ou trois groupes alkyle en $C_1$ à $C_4$, alkoxy en $C_1$ à $C_3$, halogéno ou trifluorométhyle ; ou un groupe phényl(alkyle en $C_1$ à $C_4$) facultativement substitué avec un, deux ou trois groupes alkyle en $C_1$ à $C_4$, alkoxy en $C_1$ à $C_4$, halogéno ou trifluorométhyle ;

$R_1$ et $R_2$, identiques ou différents, représentent l'hydrogène ; un groupe hydroxy ; alkyle en $C_1$ à $C_4$, de préférence alkyle en $C_1$ à $C_3$ ; alkoxy en $C_1$ à $C_3$ ; alkylthio en $C_1$ à $C_3$ ; halogéno ou trifluorométhyle ; sous réserve que $R_1$ et $R_2$ ne représentent pas l'un et l'autre un halogène ; et

$R_3$ représente l'hydrogène ; un groupe alkyle en $C_1$ à $C_6$ ; cyclo(alkyle en $C_3$ à $C_6$) ; facultativement substitué avec un, deux ou trois groupes alkyle en $C_1$ à $C_3$, de préférence cyclo(alkyle en $C_3$ à $C_5$) facultativement substitué ; phényle facultativement substitué avec un, deux ou trois groupes alkyle en $C_1$ à $C_4$, halogéno, trifluorométhyle ou alkoxy en $C_1$ à $C_3$ ; phényl(alkyle en $C_1$ à $C_3$) facultativement substitué avec un, deux ou trois groupes alkyle en $C_1$ à $C_4$, halogéno, trifluorométhyle ou alkoxy en $C_1$ à $C_3$ ; 1,3-dioxacyclohexane-5-yle ; thiénylvinyle ; furannylvinyle ; ou phénylvinyle.

2. Utilisation suivant la revendication 1, dans laquelle le composé ou un de ses hydrates ou sels pharmaceutiquement acceptables, est une thiényl- ou furannyl acylhydrazone.

3. Utilisation suivant la revendication 1 ou la revendication 2, dans laquelle X représente l'hydrogène ; un groupe alkyle en $C_1$ à $C_4$ ; cyclohexyléthyle ; phényle facultativement substitué avec un, deux ou trois groupes alkyle en $C_1$ à $C_4$, alkoxy en $C_1$ ou $C_2$, trifluorométhyle ou chloro ; alkoxy en $C_1$ à $C_4$ ; phénoxy facultativement substitué avec un, deux ou trois groupes alkyle en $C_1$ ou $C_2$, alkoxy en $C_1$ ou $C_2$, trifluorométhyle ou chloro ; cyclo(alkyle en $C_3$ à $C_6$) ; pyridinyle ; thiényle ; furannyle ; benzyloxy facultativement substitué avec un ou deux groupes alkoxy en $C_1$ ou $C_2$ ; di(alkoxy en $C_1$ ou $C_2$)phénylméthyle ; N-morpholinyléthyle ; ou 1-menthyl-O.

4. Utilisation suivant l'une quelconque des revendications précédentes, dans laquelle $R_1$ et $R_2$ sont choisis entre H, $CH_3$ et Br.

5. Utilisation suivant l'une quelconque des revendications précédentes, dans laquelle $R_3$ représente H.

6. Composé ou un de ses hydrates ou sels pharmaceutiquement acceptables, répondant à la formule I suivant la revendication 1, dans laquelle $R_1$, $R_2$, $R_3$ et Y répondent aux définitions suivant la revendication 1 et X représente un groupe cyclo(alkyle en $C_3$ à $C_{10}$) portant facultativement un, deux ou trois substituants choisis entre des groupes alkyle en $C_1$ à $C_4$, alcényle en $C_2$ à $C_4$, alkoxy en $C_1$ à $C_4$, trifluorométhyle et halogéno ; alkoxyalkyle en $C_2$ à $C_6$ ; cyclo(alkyle en $C_3$ à $C_{10}$)-(alkyle en $C_1$ à $C_4$ ; cyclo(alkyle en $C_3$ à $C_6$)oxy facultativement substitué avec un ou deux groupes alkyle en $C_1$ à $C_3$ ; benzyloxy portant facultativement un, deux ou trois substituants choisis entre des groupes alkyle en $C_1$ à $C_4$, alkoxy en $C_1$ à $C_4$, halogéno et trifluorométhyle ; ou phényle portant un, deux ou trois substituants choisis entre les groupes alkoxy en $C_1$ à $C_3$, phénoxy et trifluorométhyle ;

sous réserve que $R_3$ ne représente pas un groupe méthyle lorsque

(i) X représente un groupe cyclohexyle, le composé consiste en une 2-thiénylacylhydrazone, $R_1$ représente un groupe 5-Cl et $R_2$ représente l'hydrogène,

(ii) ou X représente un groupe benzyloxy, le composé consiste en une 2-thiénylacylhydrazone, $R_1$ représente un groupe 4-méthyle ou 5-méthyle et $R_2$ représente l'hydrogène,

(iii) ou bien X représente un groupe 2-éthoxyphényle, le composé consiste en une 2-furannylacylhydrazone et $R_1$ et $R_2$ représentent l'hydrogène.

7. Composé suivant la revendication 6, dans lequel X représente un groupe cyclohexyléthyle, trifluorométhyle ou phényle facultativement substitué avec un, deux ou trois groupes méthoxy ou éthoxy.

8. Composé suivant la revendication 6 ou la revendication 7, dans lequel Y représente un groupe NH.

9. Composé suivant l'une quelconque des revendications 6 à 8, dans lequel $R_1$ et $R_2$ sont choisis indépendamment entre H, $CH_3$ et Br.

10. Composé, hydrate ou sel suivant l'une quelconque des revendications 6 à 9, dans lequel $R_3$ représente H.

11. Utilisation suivant la revendication 1, dans laquelle le composé est le (2-thiénylméthylène)-hydrazide de l'acide benzoïque, nicotinique, acétique, formique, butyrique, cyclohexanepropanoïque ou cyclohexanecarboxylique, ou bien est le 2-(thiénylméthylène)carbazate de méthyle ou d'éthyle.

12. Composé suivant la revendication 6, qui est le (2-thiénylméthylène)hydrazide de l'acide formique, butyrique, cyclohexanepropanoïque ou cyclohexanecarboxylique, ou bien le 2-(thiénylméthylène)carbazate de méthyle.

13. Utilisation suivant la revendication 1, dans laquelle le composé est le [1-(2-thiényl)éthylidène]-hydrazide de l'acide butyrique, 3-méthylbenzoïque, 4-méthoxybenzoïque, 4-diméthylaminobenzoïque, 3-éthoxypropanoïque, 2-thiophènecarboxylique, nicotinique, formique, propanoïque, cyclohexanepropanoïque ou cyclobutanecarboxylique, ou bien est le [1-(2-thiényl)éthylidène]carbazate de phényle.

14. Composé suivant la revendication 6, qui est n'importe quel composé répondant à la définition suivant la revendication 13, à l'exception du [1-(2-thiényl)éthylidène]hydrazide de l'acide nicotinique.

15. Composé suivant la revendication 6, qui est le [(3-méthyl-2-thiényl)méthylène]hydrazide de l'acide formique, acétique, propanoïque, 2-méthylpropanoïque, butyrique, cyclobutanecarboxylique, benzoïque, 2-chlorobenzoïque, 3-chlorobenzoïque, 4-chlorobenzoïque, 2-méthylbenzoïque, 3-méthylbenzoïque, 4-méthylbenzoïque, benzèneacétique, 2-thiophènecarboxylique ou nicotinique, ou bien est le [(3-méthyl-2-thiényl)méthylène]carbazate de méthyle, de 1,1-diméthyléthyle ou de 4-méthoxyphénylméthyle.

16. Composé suivant la revendication 6, qui est le [(5-méthyl-2-thiényl)méthylène]hydrazide de l'acide formique, acétique, propanoïque, 2-méthylpropanoïque, cyclobutanecarboxylique, 3-chlorobenzoïque ou nicotinique, ou bien est le [(5-méthyl-2-thiényl)méthylène]carbazate d'éthyle, de 1,1-diméthyléthyle ou de 4-méthoxyphénylméthyle.

17. Utilisation suivant la revendication 1, dans laquelle le composé est le [1-(3-thiényl)éthylidène]-hydrazide de l'acide formique, acétique, propanoïque, butanoïque, benzoïque ou 4-chlorobenzoïque, ou bien est le [1-(3-thiényl)éthylidène]carbazate de méthyle ou d'éthyle.

18. Composé suivant la revendication 6, qui est n'importe quel composé répondant à la définition suivant la revendication 17, à l'exception du [1-(3-thiényl)éthylidène]hydrazide de l'acide acétique.

19. Composé suivant la revendication 6, qui est le 1-(2-thiényl)propylidène]hydrazide de l'acide acétique, propanoïque, butanoïque, benzoïque, 4-chlorobenzoïque, 4-éthoxybenzoïque ou 4-méthylbenzoïque, ou bien est le [1-(2-thiényl)propylidène]carbazate de méthyle ou d'éthyle.

20. Composé suivant la revendication 6, qui est le [1-(5-chloro-2-thiényl)éthylidène]hydrazide de l'acide acétique.

21. Composé suivant la revendication 6, qui est le [1-(3-méthyl-2-thiényl)éthylidène]hydrazide de l'acide acétique, propanoïque, 2-méthylpropanoïque ou benzoïque, ou bien est le [1-(3-méthyl-2-thiényl)éthylidène]carbazate de méthyle ou d'éthyle.

22. Composé suivant la revendication 6, qui est le [1-(5-méthyl-2-thiényl)éthylidène]carbazate d'éthyle.

23. Composé suivant la revendication 6, qui est le [1-(2,5-diméthyl-3-thiényl)éthylidène]hydrazide de l'acide propanoïque, 2-méthylpropanoïque ou benzoïque.

24. Utilisation suivant la revendication 1, dans laquelle le composé est le (3-thiénylméthylène)-hydrazide de l'acide formique, acétique, propanoïque, 2-méthylpropanoïque, benzoïque, cyclohexanecarboxylique, cyclobutanecarboxylique ou 4-méthoxybenzoïque, ou bien est le (3-thiénylméthylène)carbazate de phényle.

25. Composé suivant la revendication 6, qui est n'importe quel composé répondant à la définition suivant la revendication 24, à l'exception du (3-thiénylméthylène)hydrazide de l'acide benzoïque.

26. Composé suivant la revendication 6, qui est le [1-(4-méthyl-2-thiényl)éthylidène]hydrazide de l'acide benzoïque ou propanoïque.

27. Composé suivant la revendication 6, qui est le [1-(1H-pyrrole-2-yl)éthylidène]hydrazide de l'acide benzoïque ou bien le [1-(1H-pyrrole-2-yl)éthylidène]carbazate d'éthyle, de méthyle ou de phénylméthyle.

28. Composé suivant la revendication 6, qui est le [(2,5-diméthyl-1-phényl-1H-pyrrole-3-yl)méthylène]-hydrazide de l'acide butyrique ou acétique ou bien le [(2,5-diméthyl-1-phényl-1H-pyrrole-3-yl)méthylène]carbazate d'éthyle.

29. Composé suivant la revendication 6, qui est le [(1-méthyl-1H-pyrrole-2-yl)méthylène]hydrazide de l'acide benzèneacétique, isonicotinique, cyclohexanecarboxylique, cyclohexane-acétique, acétique, butyrique, cyclohexanepropanoïque, 4-chlorobenzoïque, 3-chlorobenzoïque, 2-chlorobenzoïque, 4-éthoxybenzoïque,

nicotinique, 4-méthylbenzoïque, 4-(1,1-diméthyléthyl)benzoïque, 4-méthoxybenzoïque ou cyclobutanecarboxylique, ou bien est le [(1-méthyl-1H-pyrrole-2-yl)méthylène]carbazate de phénylméthyle ou de phényle.

30. Utilisation suivant la revendication 1, dans laquelle le composé est le (1H-pyrrole-2-ylméthylène)-hydrazide de l'acide butyrique, 4-chlorobenzoïque, cyclobutanecarboxylique, 2-méthylpropanoïque, 3-cyclohexylpropanoïque, butanoïque, 2-chlorobenzoïque, 4-méthylbenzoïque, 3,4-diméthoxyphénylacétique, nicotinique, isonicotinique ou formique, ou bien est le (1H-pyrrole-2-ylméthylène)-carbazate de 1-menthyle, de 4-méthoxyphénylméthyle ou de phénylméthyle.

31. Composé suivant la revendication 6, qui est n'importe quel composé répondant à la définition suivant la revendication 30, à l'exception du (1H-pyrrole-2-ylméthylène)hydrazide de l'acide 4-chlorobenzoïque, 2-chlorobenzoïque, nicotinique ou formique.

32. Composé suivant la revendication 6, qui est le [(5-méthyl-2-furannyl)méthylène]hydrazide de l'acide formique, acétique, propanoïque, butyrique, cyclohexanecarboxylique, 4-chlorobenzoïque, 3,4-diméthoxyphénylacétique, 4-méthylbenzoïque, 3-méthylbenzoïque, 4-éthoxybenzoïque, 2-furoïque, 2-thiophènecarboxylique ou 2-phénoxybenzoïque, ou bien est le [(5-méthyl-2-furannyl)méthylène]carbazate de méthyle, d'éthyle, de phényle ou de benzyle.

33. Utilisation suivant la revendication 1, dans laquelle le composé est le [1-(2-furannyl)éthylidène]-hydrazide de l'acide formique, acétique, propanoïque, 2-méthylpropanoïque, cyclohexanecarboxylique, cyclopropanecarboxylique, 4-éthoxybenzoïque, 2-furoïque, 2-thiophènecarboxylique ou 4-chlorobenzoïque, ou bien est le [1-(2-furannyl)éthylidène]carbazate d'éthyle ou de phényle.

34. Composé suivant la revendication 6, qui est n'importe quel composé répondant à la définition suivant la revendication 33, à l'exception du [1-(2-furannyl)éthylidène]hydrazide de l'acide acétique ou 2-furoïque, ou bien du [1-(2-furannyl)éthylidène]carbazate d'éthyle.

35. Utilisation suivant la revendication 1, dans laquelle le composé est le (2-furannylméthylène)-hydrazide de l'acide 3-(N-morpholinyl)propanoïque, benzoïque, 4-chlorobenzoïque, 4-méthylbenzoïque ou 4-méthoxybenzoïque, ou bien est le (2-furannylméthylène)carbazate de phényle ou de phénylméthyle.

36. Composé suivant la revendication 6, qui est n'importe quel composé répondant à la définition suivant la revendication 35, à l'exception du [1-(2-furannyl)méthylène]hydrazide de l'acide benzoïque ou 4-chlorobenzoïque.

37. Composé suivant la revendication 6, qui est le [1-(2,5-diméthyl-3-furannyl)éthylidène]hydrazide de l'acide butanoïque, benzoïque ou propanoïque.

38. Composé suivant la revendication 6, qui est le [(5-éthyl-2-furannyl)méthylène]hydrazide de l'acide benzoïque ou butanoïque.

39. Composé suivant la revendication 6, qui est le [1-(2-furannyl)hexylidène]hydrazide de l'acide butanoïque.

40. Composé suivant la revendication 6, qui est le [1-(5-méthyl-2-furannyl)éthylidène]carbazate d'éthyle.

41. Utilisation suivant la revendication 1, dans laquelle le composé répond à la définition suivant l'une quelconque des revendications 15, 16, 19 à 23, 26 à 29, 32, 33 et 37 à 40.